(19) **Europäisches Patentamt European Patent Office Office européen des brevets**

(11) **EP 4 484 449 A1**

(12) **EUROPEAN PATENT APPLICATION**
published in accordance with Art. 153(4) EPC

(43) Date of publication:
01.01.2025   Bulletin 2025/01

(21) Application number: 23755918.2

(22) Date of filing: 21.02.2023

(51) International Patent Classification (IPC):
C07K 19/00 (2006.01)          C07K 16/22 (2006.01)
C07K 14/005 (2006.01)         C12N 15/86 (2006.01)
A61K 38/17 (2006.01)          A61P 27/02 (2006.01)

(52) Cooperative Patent Classification (CPC):
A61K 38/17; A61P 27/02; C07K 14/005;
C07K 16/22; C07K 19/00; C12N 15/86

(86) International application number:
PCT/CN2023/077365

(87) International publication number:
WO 2023/155918 (24.08.2023 Gazette 2023/34)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority:  21.02.2022   CN 202210157037
              21.02.2022   CN 202210156042

(71) Applicant: Shanghai Regenelead Therapies Co.,
Ltd.
Shanghai 201206 (CN)

(72) Inventors:
• YUAN, Xiaojing
  Shanghai 201206 (CN)
• ZHANG, Wentao
  Shanghai 201206 (CN)

• LU, Cheng
  Shanghai 201206 (CN)
• ZHANG, Wei
  Shanghai 201206 (CN)
• NING, Wei
  Shanghai 201206 (CN)
• ZHOU, Caihong
  Shanghai 201206 (CN)
• LIAO, Cheng
  Shanghai 201206 (CN)

(74) Representative: Regimbeau
20, rue de Chazelles
75847 Paris Cedex 17 (FR)

Remarks:
The complete document including Reference
Table(s) and the Sequence Listing(s) can be
downloaded from the EPO website

(54) **VEGF-BINDING MOLECULE AND PHARMACEUTICAL USE THEREOF**

(57)    The present disclosure provides a VEGF-binding molecule and a pharmaceutical use thereof. In particular, the present disclosure relates to a VEGF-binding molecule, a nucleic acid encoding the VEGF-binding molecule, a vector comprising the nucleic acid, and a pharmaceutical use.

EP 4 484 449 A1

**Description**

The present disclosure claims priority to the following patent applications: Chinese Patent Application No. CN202210157037.X, filed on Feb. 21, 2022 and entitled "VEGF-BINDING MOLECULE AND PHARMACEUTICAL USE THEREOF", and Chinese Patent Application No. CN202210156042.9, filed on Feb. 21, 2022 and entitled "NUCLEIC ACID ENCODING VEGF-BINDING MOLECULE AND PHARMACEUTICAL USE THEREOF", which are incorporated herein by reference in their entirety.

**TECHNICAL FIELD**

The present disclosure relates to the field of pharmaceutics and particularly to a VEGF-binding molecule, a nucleic acid encoding the VEGF-binding molecule, and pharmaceutical use thereof.

**BACKGROUND**

The vascuar endothelial growth factor (VEGF) family comprises 5 major members: VEGF-A, VEGF-B, VEGF-C, VEGF-D, and PGF (placental growth factor). There are 3 types of VEGF receptors: flt-1 (fms-like tyrosine kinase, VEGFR1), Flk-1/KDR (fetal liver kinase 1-murine homologue/Kinase insert Domain containing Receptor-human homologue, VEGFR2), and flt-4 (VEGFR3). VEGFR1 and VEGFR2 are mainly distributed on the surface of vascular endothelial cells, whereas VEGFR3 is mainly distributed on the lymphatic endothelium. They consist of an extracellular region containing 7 immunoglobulin-like structures, a membrane region, and a tyrosine kinase region. The activity of the tyrosine kinase is activated through the binding of the receptor to the ligand, leading to receptor phosphorylation and thus triggering many enzymatic reactions and other reactions in the cell.

VEGF plays a key role in normal embryonic vasculogenesis processes, and it also plays an important role in abnormal vasculogenesis processes in many diseases including cancer and hemangiomas and in the pathological development of new blood vessels. Solid tumors utilize blood vessels to obtain oxygen and nutrients and to remove waste products. Additionally, these tumors produce stromal factors, inducing the formation of new blood vessels to support their continued growth. Therefore, anti-angiogenic approaches represent viable cancer treatment options, e.g., by blocking or inhibiting the VEGF signaling pathway. One strategy is to use VEGF-binding proteins to isolate VEGF in the circulating serum. The VEGF-binding proteins act as decoy receptors, reducing the number of circulating VEGF ligands. VEGF-binding proteins include humanized monoclonal antibodies (e.g., Avastin®, bevacizumab and Lucentis®, ranibizumab), soluble VEGF receptors (e.g., Eylea® aflibercept), or chimeric VEGF trap molecules.

Age-related macular degeneration (AMD) is a serious ocular disease that becomes increasingly prominent as the human population ages. It is a common eye disease among people over 50 and also a leading cause of vision loss. Clinically, AMD is categorized into atrophic or dry AMD and exudative or wet AMD (wAMD) based on the presence of abnormal neovascularization. AMD has become the third major ocular disease that causes blindness, making it one of the disease areas of global research. Wet AMD is characterized by abnormal new blood vessels growing under the retina of the macula, known as choroidal neovascularization (CNV). This leads to localized edema or hemorrhage in the macular area, causing elevation of the macular area and localized detachment of the pigment epithelium. Ultimately, this results in scar formation, damaging retinal photoreceptor cells and thus causing vision loss. Therefore, inhibiting the vascular endothelial growth factor (VEGF) to block the growth of pathological new blood vessels can effectively treat fundus diseases. The recognized first-line treatment for wet AMD is intravitreal injection of protein-based VEGF inhibitors. Currently, the main protein-based VEGF inhibitor products for treating wet AMD include Roche's bevacizumab, Roche/Novartis' Fab antibody fragment Lucentis, Regeneron's VEGFR-Fc (VEGF Trap) fusion protein aflibercept, and Sichuan Kanghong Pharmaceutical's VEGFR-Fc fusion protein conbercept.

Developing VEGF-binding molecules of novel structures capable of targeting more members of the VEGF family (e.g., capable of binding to both VEGF-A and VEGF-C) is of great significance. The present disclosure provides molecules capable of simultaneously binding to VEGF-A and VEGF-C and having good efficacy, high stability, and good druggability.

**SUMMARY**

The present disclosure provides a VEGF-binding molecule and pharmaceutical use, wherein the VEGF-binding molecule has both the capacity to bind to VEGF-A and the capacity to bind to VEGF-C.

**VEGF-Binding Molecule**

The present disclosure provides a VEGF-binding molecule comprising immunoglobulin-like domain 2 of VEGFR2 (R2D2) and/or immunoglobulin-like domain 3 of VEGFR2 (R2D3); or, the VEGF-binding molecule consists of R2D2 and/or R2D3.

In some embodiments, provided is a VEGF-binding molecule comprising: a first VEGF-binding domain and a second VEGF-binding domain, wherein the first VEGF-binding domain comprises immunoglobulin-like domain 2 of VEGFR2 (R2D2) and immunoglobulin-like domain 3 of VEGFR2 (R2D3) or consists of R2D2-R2D3; the second VEGF-binding domain comprises or consists of a VEGF Trap or an anti-VEGF antibody or an antigen-binding fragment thereof.

In some embodiments, the VEGF Trap in the second VEGF-binding domain is immunoglobulin-like domain 2 of VEGFR1 (R1D2) and/or immunoglobulin-like domain 3 of VEGFR2 (R2D3).

In some embodiments, the anti-VEGF antibody in the second VEGF-binding domain is bevacizumab or ranibizumab.

In some embodiments, provided is a VEGF-binding molecule comprising:

a first VEGF-binding domain and a second VEGF-binding domain, wherein the first VEGF-binding domain comprises or consists of R2D2-R2D3;
the second VEGF-binding domain comprises or consists of R1D2-R2D3, or comprises the heavy chain variable region (VH) and the light chain variable region (VL) of an anti-VEGF antibody; illustratively, the anti-VEGF antibody is bevacizumab or ranibizumab.

By way of non-limiting illustration, R2D2-R2D3 represents a VEGF-binding domain formed by linking R2D2 and R2D3, wherein R2D2 and R2D3 may be linked directly or indirectly (e.g., by a linker).

In some embodiments, provided is a VEGF-binding molecule comprising one or more selected from the group consisting of R1D2, R2D2, and R2D3. For example, the VEGF-binding molecule comprises or consists of 1 R1D2, 1 R2D2, and 2 R2D3s. For example, the VEGF-binding molecule comprises or consists of 1 R2D2 and 1 R2D3.

In some embodiments, the VEGF-binding molecule comprises an amino acid mutation that improves stability.

In some embodiments, the VEGF-binding molecule comprises an amino acid mutation that improves affinity for a ligand.

In some embodiments, the VEGF-binding molecule comprises an amino acid mutation that improves affinity for the ligand(s) VEGF-A and/or VEGF-C.

In some embodiments, the VEGF-binding molecule comprises both an amino acid mutation that improves stability and an amino acid mutation that improves affinity for the ligand(s) VEGF-A and/or VEGF-C.

In a first aspect, in some embodiments, the R2D2 and/or R2D3 of the VEGF-binding molecule comprise(s) an amino acid mutation at any one or more positions selected from the group consisting of the following: position 162, position 221, position 222, position 274, position 276, position 277, position 280, position 284, position 288, position 289, and position 313.

The position of the amino acid mutation described above is relative to a natural sequence position of the amino acid sequence set forth in SEQ ID NO: 1.

In some embodiments, the R2D2 and/or R2D3 comprise(s) an amino acid mutation at any one or more sets of positions selected from the group consisting of the following:

- position 162;
- position 162 and position 221;
- position 162 and position 222;
- position 162 and position 274;
- position 162 and position 276;
- position 162 and position 277;
- position 162 and position 289;
- position 162 and position 313;
- position 221 and position 222;
- position 277 and position 289;
- position 162, position 221, and position 222;
- position 162, position 277, and position 289; and
- position 162, position 221, position 222, and position 274.

In some embodiments, the R2D2 and/or R2D3 comprise(s) an amino acid mutation at any one set of positions selected from the group consisting of the following:

- position 162; and
- position 162, position 221, position 222, and position 274.

In some embodiments, the R2D2 and/or R2D3 comprise(s) any one or more sets of amino acid mutations selected from the group consisting of the following:

- 162A, 162V, 162S, 162I, or 162L;
- 221S or 221N;
- 222K;
- 274F or 274I;
- 276Q;
- 277R;
- 280E;
- 284H;
- 288Y;
- 289Y; and
- 313R.

In some embodiments, the R2D2 and/or R2D3 comprise(s) any one or more sets of amino acid mutations selected from the group consisting of the following:

- C162A, C 162 V, C162S, C162I, or C162L;
- Y221S or Y221N;
- R222K;
- N274F or N274I;
- D276Q;
- L277R;
- Q280E;
- E284H;
- F288Y;
- L289Y; and
- L313R.

In some embodiments, the R2D2 and/or R2D3 comprise(s) any one or more sets of amino acid mutations selected from the group consisting of the following:

- 162V or 162I;
- 162V/274F; 162V/274I; 162I/274F; or 162I/274I;
- 221S/222K;
- 277R/289Y;
- 162V/221S/222K;
- 162V/276Q;
- 162V/289Y;
- 162V/277/289Y;
- 162V/313R; and
- 162V/221S/222K/274F.

In some embodiments, the R2D2 and/or R2D3 comprise(s) any one or more sets of amino acid mutations selected from the group consisting of the following:

- C162V or C162I;
- C162V/N274F;C162V/N274I;C162I/N274F; or C162I/N274I;
- Y221S/R222K;
- L277R/L289Y;
- C162V/Y221 S/R222K;
- C162V/D276Q;
- C162V/L289Y;
- C162V/L277R/L289Y;
- C162V/L313R; and
- C162V/Y221S/R222K/N274F.

In some embodiments, the R2D2 and/or R2D3 comprise(s) any one or more sets of amino acid mutations selected from the group consisting of the following:

- 162V; and
- 162V/221S/222K/274F.

In some embodiments, the R2D2 and/or R2D3 comprise(s) any one or more sets of amino acid mutations selected from the group consisting of the following:

- C162V; and
- C162V/Y221 S/R222K/N274F.

In some embodiments, VEGF-binding molecules comprising the aforementioned amino acid mutations have improved stability as compared to a wild type having the same molecular format.

In a second aspect, in some embodiments, the R2D2 and/or R2D3 of the VEGF-binding molecule comprise(s) an amino acid mutation at one or more positions selected from the group consisting of the following: position 135, position 197, position 199, position 213, position 215, position 219, position 257, position 275, position 277, position 278, position 283, position 289, position 313, and position 314.

The position of the amino acid mutation described above is relative to a natural sequence position of the amino acid sequence set forth in SEQ ID NO: 1.

In some embodiments, the R2D2 and/or R2D3 comprise(s) an amino acid mutation at position 283.

In some embodiments, the R2D2 and/or R2D3 comprise(s) any one or more sets of amino acid mutations selected from the group consisting of the following:

- 135Y;
- 197Y;
- 199Q or 199T;
- 213N;
- 215F or 215H;
- 219I;
- 257Q or 257T;
- 275L or 275E;
- 277R;
- 278D, 278S, or 278E;
- 283T;
- 289Y;
- 313Y; and
- 314S or 314Q.

In some embodiments, the R2D2 and/or R2D3 comprise(s) any one or more sets of amino acid mutations selected from the group consisting of the following:

- V135Y;
- M197Y;
- F199Q or F199T;
- M213N;
- I215F or I215H;
- V219I;
- D257Q or D257T;
- R275L or R275E;
- L277R;
- K278D, K278S, or K278E;
- S283T;
- L289Y;
- L313Y; and
- M314S or M314Q.

In some embodiments, the R2D2 and/or R2D3 comprise(s) 283T.

In some embodiments, the R2D2 and/or R2D3 comprise(s) the amino acid mutation S283T.

In some embodiments, VEGF-binding molecules comprising the aforementioned amino acid mutations have improved ligand-binding activity as compared to a wild type having the same molecular format.

In a third aspect, in some embodiments, the R2D2 and/or R2D3 of the VEGF-binding molecule comprise(s) an amino acid mutation at any one or more positions selected from the group consisting of the following: position 162, position 221, position 222, position 274, position 276, position 277, position 280, position 284, position 288, position 289, and position 313; and comprises an amino acid mutation at any one or more positions selected from the group consisting of the following: position 135, position 197, position 199, position 213, position 215, position 219, position 257, position 275, position 277, position 278, position 283, position 289, position 313, and position 314.

In some embodiments, the R2D2 and/or R2D3 of the VEGF-binding molecule comprise(s) an amino acid mutation at any one set of positions selected from the group consisting of the following:

- position 162 and position 275;
- position 162 and position 283;
- position 162 and position 313;
- position 162, position 274, and position 283;
- position 162, position 221, position 222, and position 283; and
- position 162, position 221, position 222, position 274, and position 283.

In some embodiments, the R2D2 and/or R2D3 comprise(s) an amino acid mutation at any one set of positions selected from the group consisting of the following:

- position 162 and position 283; and
- position 162, position 221, position 222, position 274, and position 283.

In some embodiments, the R2D2 and/or R2D3 comprise(s):

(i) any one or more sets of amino acid mutations selected from the group consisting of the following:

- 162A, 162V, 162S, 162I, or 162L;
- 221S;
- 222K;
- 274F or 274I;
- 276Q;
- 277R;
- 280E;
- 284H;
- 288Y;
- 289Y; and
- 313R; and

(ii) any one or more sets of amino acid mutations selected from the group consisting of the following:

- 135Y;
- 197Y;
- 199Q or 199T;
- 213N;
- 215F or 215H;
- 219I;
- 257Q or 257T;
- 275L or 275E;
- 277R;
- 278D, 278S, or 278E;
- 283T;
- 289Y;
- 313Y; and
- 314S or 314Q.

In some embodiments, the R2D2 and/or R2D3 comprise(s):

(i) any one or more sets of amino acid mutations selected from the group consisting of the following:

- C162A, C 162 V, C162S, C162I, or C162L;
- Y221S;
- R222K;
- N274F or N274I;
- D276Q;
- L277R;
- Q280E;
- E284H;
- F288Y;
- L289Y; and
- L313R; and

(ii) any one or more sets of amino acid mutations selected from the group consisting of the following:

- V135Y;
- M197Y;
- F199Q or F199T;
- M213N;
- I215F or I215H;
- V219I;
- D257Q or D257T;
- R275L or R275E;
- L277R;
- K278D, K278S, or K278E;
- S283T;
- L289Y;
- L313Y; and
- M314S or M314Q.

In some embodiments, the R2D2 and/or R2D3 comprise(s) any one or more sets of amino acid mutations selected from the group consisting of the following:

- 162V/275L;
- 162 V/283 T;
- 162V/313Y;
- 162V/274F/283T;
- 162V/221S/222K/283T; and
- 162V/221S/222K/274F/283T.

In some embodiments, the R2D2 and/or R2D3 comprise(s) any one or more sets of amino acid mutations selected from the group consisting of the following:

- C162V/R275L;
- C162V/S283T;
- C162V/L313Y;
- C162V/N274F/S283T;
- C162V/Y221S/R222K/S283T; and
- C162V/Y221S/R222K/N274F/S283T.

In some embodiments, the R2D2 and/or R2D3 comprise(s) any one or more sets of amino acid mutations selected from the group consisting of the following:

- 162V/283T; and
- 162V/221S/222K/274F/283T.

In some embodiments, the R2D2 and/or R2D3 comprise(s) any one or more sets of amino acid mutations selected from the group consisting of the following:

- C162V/S283T; and
- C162V/Y221S/R222K/N274F/S283T.

In the context of the present disclosure, "/" indicates that the amino acid mutations are present simultaneously in the same VEGF-binding molecule.

In the context of the present disclosure, ordinal numbers such as "first" and "second" are not to be understood as defining the number, level, or order of technical features; they are used only for distinguishing different technical features, steps, and elements.

In the embodiments described above, the mutations in the R2D2 and/or R2D3 of the VEGF-binding molecule may comprise the mutations described above or consist only of the mutations described above.

In some embodiments, the R2D2 and/or R2D3 of VEGFR2 are/is wild-type one(s) or functional variant(s) having at least 80%, at least 85%, at least 90%, at least 95%, at least 96%, at least 97%, at least 98%, or at least 99% sequence identity to the wild type one(s). In some embodiments, immunoglobulin-like domain 2 and immunoglobulin-like domain 3 of wild-type VEGFR2 have the same amino acid sequences as the R2D2 and R2D3 in the sequence set forth in SEQ ID NO: 1.

In some embodiments, immunoglobulin-like domain 2 of VEGFR2 (R2D2) and immunoglobulin-like domain 3 of VEGFR2 (R2D3) comprise one or more amino acid differences (e.g., 1, 2, 3, 4, 5, 6, 7, 8, 9, 10, or more amino acid differences) as compared to wild-type ones; the amino acid differences includes amino acid substitutions, insertions, or deletions.

In some embodiments, the amino acid mutations are relative to immunoglobulin-like domain 2 and immunoglobulin-like domain 3 of wild-type VEGFR2, wherein the amino acid sequence of wild-type VEGFR2 is set forth in SEQ ID NO: 1.

In some embodiments, immunoglobulin-like domain 2 and immunoglobulin-like domain 3 of wild-type VEGFR2 have the amino acid sequence set forth at positions 123-327 of SEQ ID NO: 1.

In some embodiments, R2D2-R2D3 has the amino acid sequence set forth at positions 123-327 of SEQ ID NO: 1.

In some embodiments, immunoglobulin-like domain 2 of wild-type VEGFR2 (R2D2) has the amino acid sequence set forth at positions 123-225 (single-underlined) of SEQ ID NO: 1, and immunoglobulin-like domain 3 of wild-type VEGFR2 (R2D3) has the amino acid sequence set forth at positions 226-327 (double-underlined) of SEQ ID NO: 1.

In the context of the present disclosure, the numbering of amino acid positions related to R2D2 and R2D3 uses the natural numbering of the amino acid sequence set forth in SEQ ID NO: 1; that is, the numbering starts at the methionine (M) at position 1 of SEQ ID NO: 1. The amino acid sequence of wild-type VEGFR2:

MQSKVLLAVALWLCVETRAASVGLPSVSLDLPRLSIQKDILTIKANTTLQITCRG
QRDLDWLWPNNQSGSEQRVEVTECSDGLFCKTLTIPKVIGNDTGAYKCFYRETD
LASVIYVYVQDYRSPFIASVSDQHGVVYITENKNKTVVIPCLGSISNLNVSLCAR
YPEKRFVPDGNRISWDSKKGFTIPSYMISYAGMVFCEAKINDESYQSIMYIVVVV
GYRIYDVVLSPSHGIELSVGEKLVLNCTARTELNVGIDFNWEYPSSKHQHKKLV
NRDLKTQSGSEMKKFLSTLTIDGVTRSDQGLYTCAASSGLMTKKNSTFVRVHEK
PFVAFGSGMESLVEATVGERVRIPAKYLGYPPPEIKWYKNGIPLESNHTIKAGHV
LTIMEVSERDTGNYTVILTNPISKEKQSHVVSLVVYVPPQIGEKSLISPVDSYQYG
TTQTLTCTVYAIPPPHHIHWYWQLEEECANEPSQAVSVTNPYPCEEWRSVEDFQ
GGNKIEVNKNQFALIEGKNKTVSTLVIQAANVSALYKCEAVNKVGRGERVISFH
VTRGPEITLQPDMQPTEQESVSLWCTADRSTFENLTWYKLGPQPLPIHVGELPTP
VCKNLDTLWKLNATMFSNSTNDILIMELKNASLQDQGDYVCLAQDRKTKKRH
CVVRQLTVLERVAPTITGNLENQTTSIGESIEVSCTASGNPPPQIMWFKDNETLVE
DSGIVLKDGNRNLTIRRVRKEDEGLYTCQACSVLGCAKVEAFFIIEGAQEKTNLE
IIILVGTAVIAMFFWLLLVIILRTVKRANGGELKTGYLSIVMDPDELPLDEHCERLP
YDASKWEFPRDRLKLGKPLGRGAFGQVIEADAFGIDKTATCRTVAVKMLKEGA
THSEHRALMSELKILIHIGHHLNVVNLLGACTKPGGPLMVIVEFCKFGNLSTYL
RSKRNEFVPYKTKGARFRQGKDYVGAIPVDLKRRLDSITSSQSSASSGFVEEKSL
SDVEEEEAPEDLYKDFLTLEHLICYSFQVAKGMEFLASRKCIHRDLAARNILLSE
KNVVKICDFGLARDIYKDPDYVRKGDARLPLKWMAPETIFDRVYTIQSDVWSF
GVLLWEIFSLGASPYPGVKIDEEFCRRLKEGTRMRAPDYTTPEMYQTMLDCWH
GEPSQRPTFSELVEHLGNLLQANAQQDGKDYIVLPISETLSMEEDSGLSLPTSPV
SCMEEEEVCDPKFHYDNTAGISQYLQNSKRKSRPVSVKTFEDIPLEEPEVKVIPD
DNQTDSGMVLASEELKTLEDRTKLSPSFGGMVPSKSRESVASEGSNQTSGYQSG
YHSDDTDTTVYSSEEAELLKLIEIGVQTGSTAQILQPDSGTTLSSPPV

SEQ ID NO: 1.

In some embodiments, the first VEGF-binding domain consists of R2D2-R2D3.

In some embodiments, the first VEGF-binding domain has the capacity to bind to VEGF-C. In some embodiments, the first VEGF-binding domain has the capacity to bind to VEGF-C and the capacity to bind to VEGF-A.

In some embodiments, the VEGF-binding molecule has increased stability and/or ligand-binding activity.

In some embodiments, the second VEGF-binding domain has the capacity to bind to VEGF-A.

In some embodiments, the second VEGF-binding domain has the capacity to bind to VEGF-A and does not have the capacity to bind to VEGF-C or has a weak capacity to bind to VEGF-C.

In some embodiments, the second VEGF-binding domain comprises or consists of a VEGF Trap.

In some embodiments, the VEGF Trap comprises immunoglobulin-like domain 2 of VEGFR1 (R1D2) and immunoglobulin-like domain 3 of VEGFR2 (R2D3).

In some embodiments, immunoglobulin-like domain 2 of VEGFR1 (R1D2) is a wild-type one or a functional variant of R1D2 having at least 80%, at least 85%, at least 90%, at least 95%, at least 96%, at least 97%, at least 98%, or at least 99% sequence identity to the wild type one.

In some embodiments, wild-type R1D2 has the same amino acid sequence as the immunoglobulin-like domain 2 in the wild-type VEGFR1 set forth in SEQ ID NO: 2.

In some embodiments, wild-type R1D2 has the amino acid sequence set forth at positions 129-231 of SEQ ID NO: 2.

In some embodiments, the functional variant of R1D2 comprises one or more amino acid differences (e.g., 1, 2, 3, 4, 5, 6, 7, 8, 9, 10, or more amino acid differences) as compared to a wild-type one; the amino acid differences includes amino acid substitutions, insertions, or deletions.

In the context of the present disclosure, the numbering of amino acid positions related to R1D2 uses the natural numbering of the amino acid sequence set forth in SEQ ID NO: 2; that is, the numbering starts at the methionine (M) at position 1 of SEQ ID NO: 2.

The amino acid sequence of wild-type VEGFR1:

MVSYWDTGVLLCALLSCLLLTGSSSGSKLKDPELSLKGTQHIMQAGQTLHLQC
RGEAAHKWSLPEMVSKESERLSITKSACGRNGKQFCSTLTLNTAQANHTGFYSC
KYLAVPTSKKKETESAIYIFISDTGRPFVEMYSEIPEIIHMTEGRELVIPCRVTSPNI
TVTLKKFPLDTLIPDGKRIIWDSRKGFIISNATYKEIGLLTCEATVNGHLYKTNYL
THRQTNTIIDVQISTPRPVKLLRGHTLVLNCTATTPLNTRVQMTWSYPDEKNKR
ASVRRRIDQSNSHANIFYSVLTIDKMQNKDKGLYTCRVRSGPSFKSVNTSVHIYD
KAFITVKHRKQQVLETVAGKRSYRLSMKVKAFPSPEVVWLKDGLPATEKSARY
LTRGYSLIIKDVTEEDAGNYTILLSIKQSNVFKNLTATLIVNVKPQIYEKAVSSFPD
PALYPLGSRQILTCTAYGIPQPTIKWFWHPCNHNHSEARCDFCSNNEESFILDADS
NMGNRIESITQRMAIIEGKNKMASTLVVADSRISGIYICIASNKVGTVGRNISFYIT
DVPNGFHVNLEKMPTEGEDLKLSCTVNKFLYRDVTWILLRTVNNRTMHYSISK
QKMAITKEHSITLNLTIMNVSLQDSGTYACRARNVYTGEEILQKKEITIRDQEAP

YLLRNLSDHTVAISSSTTLDCHANGVPEPQITWFKNNHKIQQEPGIILGPGSSTLFI
ERVTEEDEGVYHCKATNQKGSVESSAYLTVQGTSDKSNLELITLTCTCVAATLF
WLLLTLFIRKMKRSSSEIKTDYLSIIMDPDEVPLDEQCERLPYDASKWEFARERL
KLGKSLGRGAFGKVVQASAFGIKKSPTCRTVAVKMLKEGATASEYKALMTELKI
LTHIGHHLNVVNLLGACTKQGGPLMVIVEYCKYGNLSNYLKSKRDLFFLNKDA
ALHMEPKKEKMEPGLEQGKKPRLDSVTSSESFASSGFQEDKSLSDVEEEEDSDG
FYKEPITMEDLISYSFQVARGMEFLSSRKCIHRDLAARNILLSENNVVKICDFGL
ARDIYKNPDYVRKGDTRLPLKWMAPESIFDKIYSTKSDVWSYGVLLWEIFSLGG
SPYPGVQMDEDFCSRLREGMRMRAPEYSTPEIYQIMLDCWHRDPKERPRFAEL
VEKLGDLLQANVQQDGKDYIPINAILTGNSGFTYSTPAFSEDFFKESISAPKFNSG
SSDDVRYVNAFKFMSLERIKTFEELLPNATSMFDDYQGDSSTLLASPMLKRFTW
TDSKPKASLKIDLRVTSKSKESGLSDVSRPSFCHSSCGHVSEGKRRFTYDHAELE
RKIACCSPPPDYNSVVLYSTPPI

SEQ ID NO: 2.

In some embodiments, the VEGF Trap is aflibercept or conbercept. In some embodiments, the VEGF Trap is a VEGFR portion of aflibercept or a VEGFR portion of conbercept.

In some embodiments, provided is an anti-VEGF-A antibody or an antigen-binding fragment thereof. In some embodiments, the antigen-binding fragment is an scFv, Fv, Fab, or Fab' fragment.

In some embodiments, the anti-VEGF-A antibody is bevacizumab or ranibizumab. In some embodiments, the anti-VEGF-A antibody is a Fab of bevacizumab.

The present disclosure also provides a VEGF-binding molecule comprising immunoglobulin-like domain 2 of VEGFR2 (R2D2) and immunoglobulin-like domain 3 of VEGFR2 (R2D3), wherein the immunoglobulin-like domain 2 of VEGFR2 (R2D2) and/or the immunoglobulin-like domain 3 of VEGFR2 (R2D3) comprise(s) an amino acid mutation at any one or more positions selected from the group consisting of the following: position 162, position 221, position 222, position 274, position 276, position 277, position 280, position 284, position 288, position 289, and position 313. The position of the amino acid mutation is relative to a natural sequence position of the amino acid sequence set forth in SEQ ID NO: 1.

In some embodiments, the R2D2 and/or R2D3 comprise(s) an amino acid mutation at any one or more sets of positions selected from the group consisting of the following:

- position 162;
- position 162 and position 221;
- position 162 and position 222;
- position 162 and position 274;
- position 162 and position 276;
- position 162 and position 277;
- position 162 and position 289;
- position 162 and position 313;
- position 221 and position 222;
- position 277 and position 289;
- position 162, position 221, and position 222;
- position 162, position 277, and position 289; and
- position 162, position 221, position 222, and position 274.

In some embodiments, the R2D2 and/or R2D3 comprise(s) an amino acid mutation at any one set of positions selected from the group consisting of the following:

- position 162; and
- position 162, position 221, position 222, and position 274.

In some embodiments, the R2D2 and/or R2D3 comprise(s) any one or more sets of amino acid mutations selected from the group consisting of the following:

- 162A, 162V, 162S, 162I, or 162L;
- 221S or 221N;
- 222K;
- 274F or 274I;
- 276Q;
- 277R;
- 280E;
- 284H;
- 288Y;
- 289Y; and
- 313R.

In some embodiments, the R2D2 and/or R2D3 comprise(s) any one or more sets of amino acid mutations selected from the group consisting of the following:

- C162A, C 162 V, C162S, C162I, or C162L;
- Y221S;
- R222K;
- N274F or N274I;
- D276Q;
- L277R;
- Q280E;
- E284H;
- F288Y;
- L289Y; and
- L313R.

In some embodiments, the R2D2 and/or R2D3 comprise(s) any one or more sets of amino acid mutations selected from the group consisting of the following:

- 162V or 162I;
- 162V/274F; 162V/274I; 162I/274F; or 162I/274I;

- 221S/222K;
- 277R/289Y;
- 162V/221S/222K;
- 162V/276Q;
- 162V/289Y;
- 162V/277/289Y;
- 162V/313R; and
- 162V/221S/222K/274F.

In some embodiments, the R2D2 and/or R2D3 comprise(s) any one or more sets of amino acid mutations selected from the group consisting of the following:

- C162V or C162I;
- C162V/N274F;C162V/N274I;C162I/N274F; or C162I/N274I;
- Y221S/R222K;
- L277R/L289Y;
- C162V/Y221 S/R222K;
- C162V/D276Q;
- C162V/L289Y;
- C162V/L277R/L289Y;
- C162V/L313R; and
- C162V/Y221 S/R222K/N274F.

In some embodiments, the R2D2 and/or R2D3 comprise(s) any one or more sets of amino acid mutations selected from the group consisting of the following:

- 162V; and
- 162V/221S/222K/274F.

In some embodiments, the R2D2 and/or R2D3 comprise(s) any one or more sets of amino acid mutations selected from the group consisting of the following:

- C162V; and
- C162V/Y221 S/R222K/N274F.

In some embodiments, the R2D2 and/or R2D3 comprise(s) an amino acid mutation at any one or more positions selected from the group consisting of the following: position 135, position 197, position 199, position 213, position 215, position 219, position 257, position 275, position 277, position 278, position 283, position 289, position 313, and position 314. The position of the amino acid mutation is relative to a natural sequence position of the amino acid sequence set forth in SEQ ID NO: 1.

In some embodiments, the R2D2 and/or R2D3 comprise(s) an amino acid mutation at position 283.

In some embodiments, the R2D2 and/or R2D3 comprise(s) any one or more sets of amino acid mutations selected from the group consisting of the following:

- 135Y;
- 197Y;
- 199Q or 199T;
- 213N;
- 215F or 215H;
- 219I;
- 257Q or 257T;
- 275L or 275E;
- 277R;
- 278D, 278S, or 278E;
- 283T;
- 289Y;
- 313Y; and
- 314S or 314Q.

In some embodiments, the R2D2 and/or R2D3 comprise(s) any one or more sets of amino acid mutations selected from the group consisting of the following:

- V135Y;
- M197Y;
- F199Q or F199T;
- M213N;
- I215F or I215H;
- V219I;
- D257Q or D257T;
- R275L or R275E;
- L277R;
- K278D, K278S, or K278E;
- S283T;
- L289Y;
- L313Y; and
- M314S or M314Q.

In some embodiments, the R2D2 and/or R2D3 comprise(s) an amino acid mutation 283T. In some embodiments, the R2D2 and/or R2D3 comprise(s) the amino acid mutation S283T.

In some embodiments, the R2D2 and/or R2D3 comprise(s) an amino acid mutation at any one or more positions selected from the group consisting of the following: position 162, position 221, position 222, position 274, position 276, position 277, position 280, position 284, position 288, position 289, and position 313; and

comprises an amino acid mutation at any one or more positions selected from the group consisting of the following: position 135, position 197, position 199, position 213, position 215, position 219, position 257, position 275, position 277, position 278, position 283, position 289, position 313, and position 314.

In some embodiments, the R2D2 and/or R2D3 comprise(s) an amino acid mutation at any one or more sets of positions selected from the group consisting of the following:

- position 162 and position 275;
- position 162 and position 283;
- position 162 and position 313;
- position 162, position 274, and position 283;
- position 162, position 221, position 222, and position 283; and
- position 162, position 221, position 222, position 274, and position 283.

In some embodiments, the R2D2 and/or R2D3 comprise(s) an amino acid mutation at any one or more sets of positions selected from the group consisting of the following:

- position 162 and position 283; and
- position 162, position 221, position 222, position 274, and position 283.

In some embodiments, the R2D2 and/or R2D3 comprise(s):

(i) any one or more sets of amino acid mutations selected from the group consisting of the following:

- 162A, 162V, 162S, 162I, or 162L;
- 221S;
- 222K;
- 274F or 274I;
- 276Q;
- 277R;
- 280E;
- 284H;
- 288Y;
- 289Y; and
- 313R; and

(ii) any one or more sets of amino acid mutations selected from the group consisting of the following:

- 135Y;
- 197Y;
- 199Q or 199T;
- 213N;
- 215F or 215H;
- 219I;
- 257Q or 257T;
- 275L or 275E;
- 277R;
- 278D, 278S, or 278E;
- 283T;
- 289Y;
- 313Y; and
- 314S or 314Q.

In some embodiments, the R2D2 and/or R2D3 comprise(s):

(i) any one or more sets of amino acid mutations selected from the group consisting of the following:

- C162A, C 162 V, C162S, C162I, or C162L;
- Y221S;
- R222K;
- N274F or N274I;
- D276Q;
- L277R;
- Q280E;
- E284H;
- F288Y;
- L289Y; and
- L313R; and

(ii) any one or more sets of amino acid mutations selected from the group consisting of the following:

- V135Y;
- M197Y;
- F199Q or F199T;
- M213N;
- I215F or I215H;
- V219I;
- D257Q or D257T;
- R275L or R275E;
- L277R;
- K278D, K278S, or K278E;
- S283T;
- L289Y;
- L313Y; and
- M314S or M314Q.

In some embodiments, the R2D2 and/or R2D3 comprise(s) any one or more sets of amino acid mutations selected from the group consisting of the following:

- 162V/275L;
- 162 V/283 T;
- 162V/313Y;
- 162V/274F/283T;
- 162V/221S/222K/283T; and

- 162V/221S/222K/274F/283T.

In some embodiments, the R2D2 and/or R2D3 comprise(s) any one or more sets of amino acid mutations selected from the group consisting of the following:

- C162V/R275L;
- C162V/S283T;
- C162V/L313Y;
- C162V/N274F/S283T;
- C162V/Y221S/R222K/S283T; and
- C162V/Y221S/R222K/N274F/S283T.

In some embodiments, the R2D2 and/or R2D3 comprise(s) any one set of amino acid mutations selected from the group consisting of the following:

- 162V/283T; and
- 162V/221S/222K/274F/283T.

In some embodiments, the R2D2 and/or R2D3 comprise(s) any one set of amino acid mutations selected from the group consisting of the following:

- C162V/S283T; and
- C162V/Y221S/R222K/N274F/S283T.

In some embodiments, the VEGF-binding molecule consists of R2D2-R2D3.

In some embodiments, the VEGF-binding molecule comprises the amino acid sequence set forth in any one of SEQ ID NOs: 5-6 and SEQ ID NOs: 62-65, or an amino acid sequence having at least 80% sequence identity thereto.

In some embodiments, the VEGF-binding molecule consists of the amino acid sequence set forth in any one of SEQ ID NOs: 5-6 and SEQ ID NOs: 62-65, or an amino acid sequence having at least 80% sequence identity thereto.

In some embodiments, the VEGF-binding molecule has the capacity to bind to VEGF-C. In some embodiments, the VEGF-binding molecule has increased stability and/or ligand-binding activity.

In some embodiments, VEGF-binding molecules comprising amino acid mutations have increased stability and/or ligand-binding activity as compared to wild-type ones.

In some embodiments, any one of the aforementioned VEGF-binding molecules also optionally comprises: a first VEGF-binding domain, a second VEGF-binding domain, and a multimerizing component. In some embodiments, the multimerizing component includes, but is not limited to, an immunoglobulin Fc or a variant thereof.

In some embodiments, the Fc comprises any one of the hinge regions (e.g., upper, middle, and/or lower hinge regions) and CH2 and CH3 domains or any combination thereof. In some embodiments, the Fc comprises at least a CH2 domain and a CH3 domain. In some embodiments, the Fc comprises a hinge region, a CH2 domain, and a CH3 domain.

In some embodiments, the Fc is derived from an immunoglobulin of any species. In some embodiments, the Fc is derived from a human immunoglobulin. In some embodiments, the Fc is derived from immunoglobulins of other mammalian species, including but not limited to rodents (e.g., mice, rats, rabbits, and guinea pigs) or non-human primate (e.g., chimpanzee and macaque) species.

In some embodiments, the Fc is derived from an immunoglobulin of any subtype, including but not limited to: a human IgG1, IgG2, IgG3, IgG4, IgD, IgA, IgE, or IgM antibody. In some embodiments, the Fc is from human IgG1.

In some embodiments, the multimerizing component is an Fc variant whose amino acid sequence differs from a naturally occurring immunoglobulin Fc by no more than 20 amino acids, e.g., no more than 15, no more than 10, or no more than 5 amino acids. In some embodiments, the amino acid differences include amino acid substitutions, insertions, or deletions. In some embodiments, the Fc variant has reduced effector function (e.g., FcyR binding).

In some embodiments, the multimerizing component causes the VEGF-binding molecule to form a dimer, trimer, tetramer, pentamer, hexamer, heptamer, octamer, nonamer, or decamer.

In some embodiments, the VEGF-binding molecule is a dimer (including homodimers and heterodimers).

In some embodiments, the Fc comprises an IgG1 hinge region, a CH2 domain, and a CH3 domain.

In some embodiments, the VEGF-binding domain is fused with a multimerizing component (e.g., a human immunoglobulin Fc) to obtain a fusion protein that has an excellent VEGF-binding effect, good stability, and a long half-life and is capable of multivalently binding to VEGF molecules.

In some embodiments, the VEGF-binding molecule comprises, from N-terminus to C-terminus, any one selected from the group consisting of the following:

R2D2-R2D3-Fc;
R1D2-R2D3-R2D2-R2D3-Fc;
R 1 D2-R2D3 -linker-R2D2-R2D3 -F c;
R2D2-R2D3-R1D2-R2D3-Fc; and
R2D2-R2D3-linker-R1D2-R2D3-Fc.

In some embodiments, the VEGF-binding molecule comprises a heavy chain and a light chain, wherein the heavy chain sequentially comprises, from N-terminus to C-terminus: R2D2-R2D3-Fc-linker-VH-CH1, and the light chain sequentially comprises, from N-terminus to C-terminus: VL-CL, wherein "-" indicates that the two domains are directly or indirectly linked, but their relative positions from N-terminus to C-terminus are unchanged.

In some embodiments, any two adjacent domains in the VEGF-binding molecule of the present disclosure may be linked directly or by a linker.

In some embodiments, the linker is a peptide linker.

In some embodiments, the linker is represented by the following formula: $(GS)_a(GGS)_b(GGGS)_c(GGGGS)_d(GGGGG)_e$, wherein a, b, c, d, and e are independently integers greater than or equal to 0; or the linker is selected from the group consisting of: $(EAAAK)_3$ (SEQ ID NO: 90), $(EAAAR)_3$ (SEQ ID NO: 91), $(EGGGK)_3$ (SEQ ID NO: 92), $(EGGGR)_3$ (SEQ ID NO: 93), $(DAAAR)_3$ (SEQ ID NO: 94), $(DAAAK)_3$ (SEQ ID NO: 95), $(DGGGR)_3$ (SEQ ID NO: 96), and $(DGGGK)_3$; (SEQ ID NO: 97) or the linker is $(G_xS)_y$, wherein x is selected from the group consisting of integers of 1-5, and y is selected from the group consisting of integers of 1-6. GGGS corresponds to SEQ ID NO: 98; GGGGG corresponds to SEQ ID NO: 99.

In some embodiments, the linker is GGGGS (SEQ ID NO: 100). In some embodiments, in some embodiments, from N-terminus to C-terminus, the VEGF-binding molecule is selected from the group consisting of:

R2D2-R2D3-Fc;
R1D2-R2D3-R2D2-R2D3-Fc;
R1D2-R2D3-linker-R2D2-R2D3-Fc;
R2D2-R2D3-R1D2-R2D3-Fc; and
R2D2-R2D3-linker-R1D2-R2D3-Fc.

In some embodiments, the VEGF-binding molecule comprises a heavy chain and a light chain, wherein the heavy chain is, from N-terminus to C-terminus: R2D2-R2D3-Fc-linker-VH-CH1, and the light chain is, from N-terminus to C-terminus: VL-CL.

In some embodiments, the VEGF-binding molecule comprises the amino acid sequence set forth in any one of SEQ ID NOs: 3-4, SEQ ID NOs: 7-49, SEQ ID NOs: 52-61, and SEQ ID NO: 66, or an amino acid sequence having at least 80% sequence identity thereto. In some embodiments, the VEGF-binding molecule consists of the amino acid sequence set forth in any one of SEQ ID NOs: 3-4, SEQ ID NOs: 7-49, SEQ ID NOs: 52-61, and SEQ ID NO: 66, or an amino acid sequence having at least 80% sequence identity thereto. In some embodiments, the heavy chain of the VEGF-binding molecule comprises the amino acid sequence set forth in SEQ ID NO: 50, or an amino acid sequence having at least 80% sequence identity thereto; and the heavy chain of the VEGF-binding molecule comprises a light chain set forth in SEQ ID NO: 51, or an amino acid sequence having at least 80% sequence identity thereto.

In some embodiments, the heavy chain of the VEGF-binding molecule comprises the amino acid sequence set forth in SEQ ID NO: 67, or an amino acid sequence having at least 80% sequence identity thereto; and the light chain of the VEGF-binding molecule comprises the amino acid sequence set forth in SEQ ID NO: 68, or an amino acid sequence having at least 80% sequence identity thereto.

In some embodiments, the VEGF-binding molecule comprises the amino acid sequence set forth in SEQ ID NO: 44 or an amino acid sequence having at least 80% sequence identity thereto, or consists of the amino acid sequence set forth in SEQ ID NO: 44.

In some embodiments, the VEGF-binding molecule comprises the amino acid sequence set forth in SEQ ID NO: 61 or an amino acid sequence having at least 80% sequence identity thereto, or consists of the amino acid sequence set forth in SEQ ID NO: 61.

In some embodiments, the VEGF-binding molecule of the present disclosure includes full-length or partial proteins of any one of the aforementioned VEGF-binding molecules, or muteins, functional derivatives, functional fragments, bioactive peptides, fusion proteins, isotypes, or salts thereof obtained by further mutation on the basis of any one of the aforementioned VEGF-binding molecules, for example, fusion proteins comprising the VEGF-binding molecule, monomers or dimers or trimers or multimers of the VEGF-binding molecule, various modified forms of the VEGF-binding molecule (e.g., PEGylated, glycosylated, albumin-conjugated or -fused, albumin antibody-conjugated or -fused, hydroxyethylated, and de-O-glycosylated), and homologs of the VEGF-binding molecule in various species. The modifications to the VEGF-binding molecule do not result in adverse effects on protein activity associated with treatment.

In some embodiments, the VEGF-binding molecule has increased stability and/or ligand-binding activity.

In some embodiments, VEGF-binding molecules comprising amino acid mutations have increased stability and/or ligand-binding activity as compared to wild-type ones.

In some embodiments, the VEGF-binding molecule of the present disclosure has an HPLC purity of at least 70%, at least 75%, at least 80%, at least 85%, at least 90%, or at least 95% when placed at 40 °C with 75% humidity for two weeks.

In some embodiments, the VEGF-binding molecule of the present disclosure has an HPLC purity of at least 70%, at least 75%, at least 80%, at least 85%, at least 90%, or at least 95% when placed at 40 °C with 75% humidity for four weeks.

In some embodiments, the VEGF-binding molecule of the present disclosure has an HPLC purity of at least 70%, at least 75%, at least 80%, at least 85%, at least 90%, or at least 95% when placed at room temperature with 75% humidity for two weeks.

In some embodiments, the VEGF-binding molecule of the present disclosure has an HPLC purity of at least 70%, at least 75%, at least 80%, at least 85%, at least 90%, or at least 95% when placed at room temperature with 75% humidity for four weeks.

In some embodiments, HPLC purity may be measured using the method of Example 4.

In some embodiments, the binding activity IC50 of the VEGF-binding molecule of the present disclosure to VEGF-A is $\leq$10 nM, $\leq$5 nM, or $\leq$2 nM; for example, the IC50 is 0.01 nM-10 nM, 0.05 nM-5 nM, 0.05 nM-2 nM, 0.05 nM-1.5 nM, 0.05 nM-1.2 nM, or 0.05 nM-1 nM; and/or

the binding activity IC50 of the VEGF-binding molecule of the present disclosure to VEGF-C is $\leq$10 nM, $\leq$5 nM, or $\leq$2 nM; for example, the binding activity IC50 of the VEGF-binding molecule of the present disclosure to VEGF-C is 0.01 nM-10 nM, 0.05 nM-5 nM, 0.05 nM-2 nM, 0.05 nM-1.5 nM, 0.05 nM-1.2 nM, or 0.05 nM-1 nM.

In some embodiments, the binding kinetics $K_D$ of the VEGF-binding molecule of the present disclosure to VEGF-A is $\leq 1 \times 10^{-9}$; for example, $K_D \leq 1 \times 10^{-10}$, $K_D \leq 9 \times 10^{-11}$, $K_D \leq 8 \times 10^{-11}$, $K_D \leq 7 \times 10^{-11}$, $K_D \leq 6 \times 10^{-11}$, $K_D \leq 5 \times 10^{-11}$ or $K_D \leq 4 \times 10^{-11}$;

the binding kinetics $K_D$ of the VEGF-binding molecule of the present disclosure to VEGF-B is $\leq 1 \times 10^{-9}$; for example, $K_D \leq 4 \times 10^{-10}$, $K_D \leq 3 \times 10^{-10}$, $K_D \leq 2 \times 10^{-10}$, $K_D \leq 1 \times 10^{-10}$, $K_D \leq 9 \times 10^{-11}$, $KD \leq 8 \times 10^{-11}$, $K_D \leq 7 \times 10^{-11}$, $K_D \leq 6 \times 10^{-11}$, $K_D \leq 5 \times 10^{-11}$, $KD \leq 4 \times 10^{-11}$, $K_D \leq 3 \times 10^{-11}$, or $K_D \leq 2 \times 10^{-11}$; and/or

the binding kinetics $K_D$ of the VEGF-binding molecule of the present disclosure to VEGF-C is $\leq 1 \times 10^{-9}$; e.g., $K_D \leq 1 \times 10^{-10}$, $K_D \leq 9 \times 10^{-11}$, $K_D \leq 8 \times 10^{-11}$, $K_D \leq 7 \times 10^{-11}$, $K_D \leq 6 \times 10^{-11}$, $K_D \leq 5 \times 10^{-11}$, $K_D \leq 4 \times 10^{-11}$, $K_D \leq 3 \times 10^{-11}$, or $KD \leq 2 \times 10^{-11}$.

In some embodiments, the binding activity of the VEGF-binding molecule of the present disclosure to a ligand can be measured using the method described in Example 3 or Example 5.

**Nucleic Acid and Vector**

The present disclosure also provides a nucleic acid molecule encoding the VEGF-binding molecule of the present disclosure. In some embodiments, the nucleic acid of the present disclosure may be an RNA, DNA, or cDNA. According to some embodiments of the present disclosure, the nucleic acid of the present disclosure is an isolated nucleic acid.

The present disclosure also provides a DNA molecule encoding any of the aforementioned VEGF-binding molecules of the present disclosure.

The nucleic acid of the present disclosure may also be in the form of a vector, and it may be present in the vector and/or may be part of the vector. The vector is, e.g., a plasmid, cosmid, YAC, or viral vector. The vector may especially be an expression vector, i.e., a vector that provides for the *in-vitro* and/or *in-vivo* (i.e., in suitable host cells, host organisms and/or expression systems) expression of the VEGF-binding molecule. The expression vector generally comprises at least one of the nucleic acids of the present disclosure, which is operatively linked to one or more suitable expression regulatory elements (e.g., promoters, enhancers, and terminators). The selection of the elements and their sequences for expression in a particular host is within the knowledge of those skilled in the art. Regulatory elements and other elements useful or necessary for expression of the VEGF-binding molecule of the present disclosure include, for example, promoters, enhancers, terminators, integration factors, selection labels, leader sequences, and reporter genes.

The nucleic acid of the present disclosure may be prepared or obtained by known means (e.g., by automatic DNA synthesis and/or recombinant DNA techniques) based on information on the amino acid sequence of the polypeptide of the present disclosure, and/or may be isolated from a suitable natural source.

In some embodiments, the nucleic acid molecule comprises a transgene or polynucleotide encoding any one of the VEGF-binding molecules of the present disclosure.

In some embodiments, the transgene comprises a nucleic acid sequence having at least 50%, at least 60%, at least 70%, at least 80%, at least 90%, at least 99%, or 100% identity to the nucleic acid sequence of any one of SEQ ID NOs: 78-86, or a codon-optimized variant of the nucleic acid sequences of SEQ ID NOs: 78-86.

In some embodiments, the nucleic acid sequence of the transgene is operatively linked to a constitutive promoter. In other embodiments, the nucleic acid sequence of the transgene is operatively linked to an inducible promoter. In some cases, the nucleic acid sequence of the transgene is operatively linked to a tissue-specific or cell type-specific regulatory element.

In the context of the present disclosure, the transgene refers to the target gene, and a nucleic acid encoding any one of the VEGF-binding molecules of the present disclosure. In some embodiments, the isolated nucleic acid further comprises a promoter operatively linked to the transgene.

In some embodiments, the promoter is selected from the group consisting of a cytomegalovirus (CMV) promoter, a Rous sarcoma virus (RSV) promoter, an MMT promoter, an EF-1alpha promoter, a UB6 promoter, a chicken beta-actin promoter, a CAG promoter, an RPE65 promoter, and an opsin promoter, e.g., a CMV promoter.

In some embodiments, the isolated nucleic acid further comprises an enhancer; for example, the enhancer is a CMV enhancer.

In some embodiments, the isolated nucleic acid further comprises an intron.

In some embodiments, the isolated nucleic acid further comprises a Kozak sequence. In some embodiments, the Kozak sequence is located between the intron and the transgene encoding the VEGF-binding molecule.

In some embodiments, the isolated nucleic acid further comprises a 5'-UTR and a 3'-UTR. In some embodiments, the isolated nucleic acid further comprises a WPRE sequence.

In some embodiments, the isolated nucleic acid further comprises a polyA sequence, e.g., an sv40polyA sequence.

The present disclosure also provides a vector comprising any one of the isolated nucleic acids described above. In some embodiments, the vector is a plasmid or a viral vector. In some embodiments, the virus is selected from the group consisting of an adeno-associated virus (AAV), a helper-dependent adenovirus, a retrovirus, a herpes simplex virus, a lentivirus, an adenovirus, an adeno-associated viral vector, a poxvirus, a hemagglutinating virus of Japan-liposome (HVJ) complex, a Moloney murine leukemia virus, and an HIV-based virus.

In some embodiments, the vector is a recombinant adeno-associated viral vector.

The present disclosure also provides a recombinant adeno-associated viral vector comprising:

(i) the transgene encoding the VEGF-binding molecule defined in any one of the foregoing of the present disclosure;
(ii) an enhancer (e.g., a CMV enhancer);
(iii) a promoter (e.g., a CMV promoter);
(iv) a 5'-UTR and a 3'-UTR;
(v) a post-transcriptional regulatory element (e.g., WPRE); and
(vi) a polyadenylation signal (polyA, e.g., SV40 polyA);

optionally, (vii) a Kozak sequence, and/or, (viii) an intron;
any one or any combination of (ii)-(viii) is operatively linked to the (i).

In some embodiments, the recombinant adeno-associated viral vector comprises a 5' ITR and/or a 3' ITR. The 5' ITR and/or 3' ITR are/is derived from AAV1, AAV2, AAV3, AAV4, AAV5, AAV6, AAV7, AAV8, AAV9, AAV9.47, AAV9 (hu14), AAV10, AAV11, AAV12, AAVrh8, AAVrh10, AAV-DJ, or AAV-DJ8; for example, from AAV2 or AAV9.

In some specific embodiments, the promoter is selected from the group consisting of CMV, CAG, CBh, EFS, EF1 (e.g., EF-1$\alpha$), PGK, SV40, Ubi, and RSV or any combination thereof.

In some specific embodiments, the enhancer is selected from the group consisting of Ubi, CMV, and RSV enhancers or any combination thereof.

In some specific embodiments, the intron is selected from the group consisting of MVM, SV40, $\beta$ Globin, EF1 (e.g., EF-1$\alpha$), and a hybrid intron, or any combination thereof.

In some specific embodiments, the polyA is selected from the group consisting of PA75 polyA, SV40 polyA, hGH polyA, BGH polyA, and rbGlob polyA or any combination thereof.

In some specific embodiments, the post-transcriptional regulatory element is selected from the group consisting of WPRE and HPRE or a combination thereof.

In some embodiments, the recombinant adeno-associated viral vector comprises any one of the aforementioned isolated nucleic acids and a capsid protein.

In some embodiments, the capsid protein is selected from the group consisting of the following serotypes: AAV1, AAV2, AAV3, AAV4, AAV5, AAV6, AAV7, AAV8, AAV9, and AAV 10 or variants thereof.

In some embodiments, the capsid protein has and comprises the amino acid sequence set forth in SEQ ID NO: 77, or an amino acid sequence having at least 70%, at least 75%, at least 80%, at least 85%, at least 90%, at least 95%, or at least 99% sequence identity to SEQ ID NO: 77.

The new-structure VEGF-binding molecule provided by the present disclosure is used as a novel VEGF inhibitor that may be administered in the form of a protein drug or nucleic acid drug. In terms of function, it is capable of binding to VEGF-A and VEGF-C simultaneously and thus has higher VEGF inhibition efficiency. In terms of efficacy, the VEGF-binding molecule has higher stability; in particular, the nucleic acid drug form can effectively reduce administration frequency; it is a drug with good clinical development prospects.

AAV capsid and AAV particle

The polynucleotide according to any one of the foregoing of the present disclosure may be wrapped using an AAV capsid to form an AAV particle.

The AAV capsid comprises or is derived from any natural or recombinant AAV serotype, including but not limited to: PHP.B, PHP.A, AAV1, AAV2, AAV2G9, AAV3, AAV3a, AAV3b, AAV3-3, AAV4, AAV4-4, AAV5, AAV6, AAV6.1, AAV6.2, AAV6.1.2, AAV7, AAV7.2, AAV8, AAV9, AAV9.11, AAV9.13, AAV9.16, AAV9.24, AAV9.45, AAV9.47, AAV9.61, AAV9.68, AAV9.84, AAV9.9, AAV10, AAV11, AAV12, AAV16.3, AAV24.1, AAV27.3, AAV42.12, AAV42-1b, AAV42-2, AAV42-3a, AAV42-3b, AAV42-4, AAV42-5a, AAV42-5b, AAV42-6b, AAV42-8, AAV42-10, AAV42-11, AAV42-12, AAV42-13, AAV42-15, AAV42-aa, AAV43-1, AAV43-12, AAV43-20, AAV43-21, AAV43-23, AAV43-25, AAV43-5, AAV44.1, AAV44.2, AAV44.5, AAV223.1, AAV223.2, AAV223.4, AAV223.5, AAV223.6, AAV223.7, AAV1-7/rh.48, AAV1-8/rh.49, AAV2-15/rh.62, AAV2-3/rh.61, AAV2-4/rh.50, AAV2-5/rh.51, AAV3.1/hu.6, AAV3.1/hu.9, AAV3-9/rh.52, AAV3-11/rh.53, AAV4-8/r11.64, AAV4-9/rh.54, AAV4-19/rh.55, AAV5-3/rh.57, AAV5-22/rh.58, AAV7.3/hu.7, AAV16.8/hu.10, AAV16.12/hu.11, AAV29.3/bb.1, AAV29.5/bb.2, AAV106.1/hu.37, AAV114.3/hu.40, AAV127.2/hu.41, AAV127.5/hu.42, AAV128.3/hu.44, AAV130.4/hu.48, AAV145.1/hu.53, AAV145.5/hu.54, AAV145.6/hu.55, AAV161.10/hu.60, AAV161.6/hu.61, AAV33.12/hu.17, AAV33.4/hu.15, AAV33.8/hu.16, AAV52/hu.19, AAV52.1/hu.20, AAV58.2/hu.25, AAVA3.3, AAVA3.4, AAVA3.5, AAVA3.7, AAVC1, AAVC2, AAVC5, AAV-DJ, AAV-DJ8, AAVF3, AAVF5, AAVH2, AAVrh.72, AAVhu.8, AAVrh.68, AAVrh.70, AAVpi.1, AAVpi.3, AAVpi.2, AAVrh.60, AAVrh.44, AAVrh.65, AAVrh.55, AAVrh.47, AAVrh.69, AAVrh.45, AAVrh.59, AAVhu.12, AAVH6, AAVLK03, AAVH-1/hu.1, AAVH-5/hu.3, AAVLG-10/rh.40, AAVLG-4/rh.38, AAVLG-9/hu.39, AAVN721-8/rh.43, AAVCh.5, AAVCh.5R1, AAVcy.2, AAVcy.3, AAVcy.4, AAVcy.5, AAVCy.5R1, AAVCy.5R2, AAVCy.5R3, AAVCy.5R4, AAVcy.6, AAVhu.1, AAVhu.2, AAVhu.3, AAVhu.4, AAVhu.5, AAVhu.6, AAVhu.7, AAVhu.9, AAVhu.10, AAVhu.11, AAVhu.13, AAVhu.15, AAVhu.16, AAVhu.17, AAVhu.18, AAVhu.20, AAVhu.21, AAVhu.22, AAVhu.23.2, AAVhu.24, AAVhu.25, AAVhu.27, AAVhu.28, AAVhu.29, AAVhu.29R, AAVhu.31, AAVhu.32, AAVhu.34, AAVhu.35, AAVhu.37, AAVhu.39, AAVhu.40, AAVhu.41, AAVhu.42, AAVhu.43, AAVhu.44, AAVhu.44R1, AAVhu.44R2, AAVhu.44R3, AAVhu.45, AAVhu.46, AAVhu.47, AAVhu.48, AAVhu.48R1, AAVhu.48R2, AAVhu.48R3, AAVhu.49, AAVhu.51, AAVhu.52, AAVhu.54, AAVhu.55, AAVhu.56, AAVhu.57, AAVhu.58, AAVhu.60, AAVhu.61, AAVhu.63, AAVhu.64, AAVhu.66, AAVhu.67, AAVhu.68, AAVhu.14/9, AAVhu.t19, AAVrh.2, AAVrh.2R, AAVrh.8, AAVrh.8R, AAVrh.10, AAVrh.12, AAVrh.13, AAVrh.13R, AAVrh.14, AAVrh.17, AAVrh.18, AAVrh.19, AAVrh.20, AAVrh.21, AAVrh.22, AAVrh.23, AAVrh.24, AAVrh.25, AAVrh.31, AAVrh.32, AAVrh.33, AAVrh.34, AAVrh.35, AAVrh.36, AAVrh.37, AAVrh.37R2, AAVrh.38, AAVrh.39, AAVrh.40, AAVrh.46, AAVrh.48, AAVrh.48.1, AAVrh.48.1.2, AAVrh.48.2, AAVrh.49, AAVrh.51, AAVrh.52, AAVrh.53, AAVrh.54, AAVrh.56, AAVrh.57, AAVrh.58, AAVrh.61, AAVrh.64, AAVrh.64R1, AAVrh.64R2, AAVrh.67, AAVrh.73, AAVrh.74, AAVrh8R, AAVrh8R A586R mutant, AAVrh8R R533A mutant, AAAV, BAAV, goat AAV, bovine AAV, ovine AAV, AAVhE1.1, AAVhEr1.5, AAVhER1.14, AAVhEr1.8, AAVhEr1.16, AAVhEr1.18, AAVhEr1.35, AAVhEr1.7, AAVhEr1.36, AAVhEr2.29, AAVhEr2.4, AAVhEr2.16, AAVhEr2.30, AAVhEr2.31, AAVhEr2.36, AAVhER1.23, AAVhEr3.1, AAV2.5T, AAV-PAEC, AAV-LK01, AAV-LK02, AAV-LK03, AAV-LK04, AAV-LK05, AAV-LK06, AAV-LK07, AAV-LK08, AAV-LK09, AAV-LK10, AAV-LK11, AAV-LK12, AAV-LK13, AAV-LK14, AAV-LK15, AAV-LK16, AAV-LK17, AAV-LK18, AAV-LK19, AAV-PAEC2, AAV-PAEC4, AAV-PAEC6, AAV-PAEC7, AAV-PAEC8, AAV-PAEC11, AAV-PAEC12, AAV-2-pre-miRNA-101, AAV-8h, AAV-8b, AAV-h, AAV-b, AAV SM 10-2, AAVShuffle 100-1, AAV Shuffle 100-3, AAV Shuffle 100-7, AAV Shuffle 10-2, AAVShuffle 10-6, AAV Shuffle 10-8, AAV Shuffle 100-2, AAV SM 10-1, AAV SM 10-8, AAVSM 100-3, AAV SM 100-10, BNP61 AAV, BNP62 AAV, BNP63 AAV, AAVrh.50, AAVrh.43, AAVrh.62, AAVrh.48, AAVhu.19, AAVhu.11, AAVhu.53, AAV4-8/rh.64, AAVLG-9/hu.39, AAV54.5/hu.23, AAV54.2/hu.22, AAV54.7/hu.24, AAV54.1/hu.21, AAV54.4R/hu.27, AAV46.2/hu.28, AAV46.6/hu.29, AAV128.1/hu.43, true type AAV (ttAAV), UPENNAAV 10, Japanese AAV 10 serotype, AAV CBr-7.1, AAV CBr-7.10, AAV CBr-7.2, AAV CBr-7.3, AAVCBr-7.4, AAV CBr-7.5, AAV CBr-7.7, AAV CBr-7.8, AAV CBr-B7.3, AAV CBr-B7.4, AAVCBr-E1, AAV CBr-E2, AAV CBr-E3, AAV CBr-E4, AAV CBr-E5, AAV CBr-e5, AAV CBr-E6, AAVCBr-E7, AAV CBr-E8, AAV CHt-1, AAV CHt-2, AAV CHt-3, AAV CHt-6.1, AAV CHt-6.10, AAVCHt-6.5, AAV CHt-6.6, AAV CHt-6.7, AAV CHt-6.8, AAV CHt-P1, AAV CHt-P2, AAV CHt-P5, AAV CHt-P6, AAV CHt-P8, AAV CHt-P9, AAV CKd-1, AAV CKd-10, AAV CKd-2, AAV CKd-3, AAVCKd-4, AAV CKd-6, AAV CKd-7, AAV CKd-8, AAV CKd-B1, AAV CKd-B2, AAV CKd-B3, AAV CKd-B4, AAV CKd-B5, AAV CKd-B6, AAV CKd-B7, AAV CKd-B8, AAV CKd-H1, AAV CKd-H2, AAV CKd-H3, AAV CKd-H4, AAV CKd-H5, AAV CKd-H6, AAV CKd-N3, AAV CKd-N4, AAV CKd-N9, AAV CLg-F1, AAV CLg-F2, AAV CLg-F3, AAV CLg-F4, AAV CLg-F5, AAV CLg-F6, AAV CLg-F7, AAV CLg-F8, AAV CLv-1, AAV CLv1-1, AAV Clv1-10, AAV CLv1-2, AAV CLv1-12, AAV CLv1-3, AAV CLv1-13, AAV CLv1-4, AAV Clv1-7, AAV Clv1-8, AAV Clv1-9, AAV CLv-2, AAV CLv-3, AAV CLv-4, AAV CLv-6, AAV CLv-8, AAV CLv-D1, AAV CLv-D2, AAV CLv-D3, AAV CLv-D4, AAV CLv-D5, AAV CLv-D6, AAV CLv-D7, AAV CLv-D8, AAV CLv-E1, AAV CLv-K1, AAV CLv-K3, AAV CLv-K6, AAV CLv-L4, AAV CLv-L5, AAV CLv-L6, AAV CLv-M1, AAV CLv-M11, AAV CLv-M2, AAV CLv-M5, AAV CLv-M6, AAV CLv-M7, AAV CLv-M8, AAV CLv-M9, AAV CLv-R1, AAV CLv-R2, AAV CLv-R3, AAV CLv-R4, AAV CLv-R5, AAV CLv-R6, AAV CLv-R7, AAV CLv-R8, AAV CLv-R9, AAV CSp-1, AAV CSp-10, AAV CSp-11, AAV CSp-2, AAV CSp-3, AAV CSp-4, AAV CSp-6, AAV CSp-7, AAVCSp-8, AAV CSp-8.10, AAV CSp-8.2, AAV CSp-8.4, AAV CSp-8.5, AAV CSp-8.6, AAV CSp-8.7, AAV CSp-8.8, AAV CSp-8.9, AAV CSp-9, AAVhu.48R3, AAVVR-355, AAV3B, AAV4, AAV5, AAVF1/HSC1, AAVF11/HSC11, AAVF12/HSC12, AAVF13/HSC13, AAVF14/HSC14, AAVF15/HSC15, AAVF16/HSC16, AAVF17/HSC17, AAVF2/HSC2, AAVF3/HSC3, AAVF4/HSC4, AAVF5/HSC5, AAVF6/HSC6, AAVF7/HSC7, AAVF8/HSC8, AAVF9/HSC9, PHP.B(AAV-PHP.B), PHP. A(AAVPHP. A), G2B-26, G2B-13, TH1.1-32, TH1.1-35, AAVPHP.B2, AAVPHP.B3, AAVPHPN/PHPB-DGT, AAVPHP.B-EST, AAVPHP.B-GGT, AAVPHP.B-ATP, AAVPHP.B-ATT-T, AAVPHP.B-DGT-T, AAVPHP.B-GGT-T, AAVPHP.B-SGS, AAVPHP.B-AQP, AAVPHP.B-QQP, AAVPHPB-SNP(3), AAVPHP.B-SNP, AAVPHP.B-QGT, AAVPHP.B-NQT, AAVPHP.B-EGS, AAVPHP.B-SGN, AAVPHP.B-EGT, AAVPHPB-DST, AAVPHPB-DST, AAVPHP.B-STP, AAVPHP.B-PQP, AAVPHP.B-SQP, AAVPUP.B-QLP, AAVPHP.B-TMP, AAVPHP.B-TTP, AAVPHP.S/G2A12, AAVG2A15/G2A3, AAVG2B4, AAVG2B5, AAVDJ8, and variants thereof.

In some embodiments, the AAV capsid may be modified or mutated, for example, containing one or more mutations of Y252F, Y272F, Y444F, Y500F, Y700F, Y704F, Y730F, Y275F, Y281F, Y508F, Y576F, Y612G, Y673F, and Y720F; for example, containing one or more mutations of F129L, D418E, K531E, L584F, V598A, and H642N; for example, containing substitution of at least one of Tyr residues at positions 252, 272, 444, 500, 700, 704, and 730 of AAV2 with, for example, a Phe residue; for example, containing an N587A, E548A, or N708A mutation in the AAV2 capsid; for example, containing a T446F mutation in the AAV9 capsid; for example, containing a V708K mutation in the AAV capsid; for example, the AAV capsid is generated via an AAV9 capsid library having mutations in amino acids at positions 390-627 (VP1 numbering).

The AAV capsids in WO2018232055 (e.g. Table 1 thereof), WO2005033321, WO2015168666, WO2015121501, WO2015038958, WO2016065001, WO2016130589, WO2016049230, WO2016134375, WO2017100671, WO2017083722, WO2017015102, WO2017058892, WO2017066764, US9546112, US7198951, US9233131, US6156303, US9624274, US9475845, US8734809, US20130224836, US20140359799, US20150315612, US20150376240, US20150159173, US20150376607, US20150238550, US20160369298, US20160361439, US20170145405, and NPulicherla et al., (MolecularTherapy19(6):1070-1078(2011)) are incorporated in the present disclosure by reference in their entirety. In some specific embodiments, the AAV capsids used in the present disclosure are the AAV2 and AAV9 capsids in the prior art described above.

In some embodiments, the AAV capsid is engineered; for example, the AAV capsid is a hybrid AAV capsid from two or more parental serotypes. For example, the AAV capsid may be AAV2G9, which comprises sequences from AAV2 and AAV9. The sequence of AAV2G9 in US20160017005 is incorporated herein by reference in its entirety.

In some embodiments, the AAV capsid is an AAV2 or AAV9 capsid.

In some embodiments, the AAV particle may comprise all or part of the vector genome of a nucleotide sequence or a variant of any naturally occurring and/or recombinant AAV capsid, in addition to the encoded heterologous payload.

In some embodiments, the AAV particle may be replication-deficient (e.g., it lacks sequences encoding functional Rep and Cap proteins in the vector genome). In some specific embodiments, the replication-deficient AAV particle may lack most or all of the parental coding sequences, and carry essentially only one or two AAV ITR sequences and a nucleic acid of interest for delivery to a cell, tissue, organ, or organism.

In some embodiments, the AAV particle may be a recombinant AAV (rAAV) particle.

In some embodiments, the AAV particle may be selected from the group consisting of a single-stranded AAV particle (e.g., ssAAV) and a self-complementary AAV particle (e.g., scAAV). By skipping the synthesis of a second strand, scAAV achieves rapid expression in cells.

In some embodiments, the AAV particle has a nucleic acid wrapped therein, wherein a VEGF-binding molecule encoded by the nucleic acid comprises the amino acid sequence set forth in any one of SEQ ID NOs: 3-4, 7-49, 52-61, and 66; the VEGF-binding molecule comprises a heavy chain set forth in SEQ ID NO: 50 and a light chain set forth in SEQ ID NO: 51; or the VEGF-binding molecule comprises a heavy chain set forth in SEQ ID NO: 67 and a light chain set forth in SEQ ID NO: 68.

In some embodiments, the AAV particle has a nucleic acid wrapped herein, wherein the nucleic acid has at least 95% identity to the sequence set forth in any one of SEQ ID NOs: 78-86.

In some embodiments, the AAV particle has a nucleic acid wrapped herein, wherein the nucleic acid comprises the sequence set forth in any one of SEQ ID NOs: 78-86.

In some embodiments, the capsid protein of the AAV particle has and comprises the amino acid sequence set forth in SEQ ID NO: 77, or an amino acid sequence having at least 70%, at least 75%, at least 80%, at least 85%, at least 90%, at least 95%, or at least 99% sequence identity to SEQ ID NO: 77.

Regulatory element

The vector genome (or viral genome) of the nucleic acid or AAV particle according to any one of the aforementioned of the present disclosure comprises at least one regulatory element, such that a target gene (e.g., a VEGF-binding molecule) in a payload can be replicated, transcribed, and translated.

In some embodiments, the regulatory element includes, but is not limited to, sequences for transcription initiation and/or termination, promoter and/or enhancer sequences, efficient RNA processing signals (e.g., splicing and polyadenylation signals), sequences that stabilize cytoplasmic mRNA, sequences that enhance translation efficiency (e.g., Kozak consensus sequences), sequences that enhance protein stability, and/or sequences that enhance protein processing and/or secretion. Exemplary regulatory elements include, but are not limited to, promoters, enhancers, introns, endogenous miRNAs, post-transcriptional regulatory elements (PREs), polyadenylation (PolyA) signal sequences, and upstream enhancers (USEs).

In some embodiments, the regulatory elements described below (e.g., promoters) drive expression of the payload in a target tissue (e.g., in an eye of a subject) for a period of time, for example, for 1 day, 2 days, 3 days, 4 days, 5 days, 6 days, 7 days, 1 week, 2 weeks, 3 weeks, 4 weeks, 1 month, 2 months, 3 months, 4 months, 5 months, 6 months, 7 months, 8 months, 9 months, 10 months, 11 months, 12 months, 1 year, 2 years, 3 years, 4 years, 5 years, 6 years, 7 years, 8 years, 9 years, 10 years, 20 years, 30 years, 40 years, 50 years, 60 years or more, as another example, for 1-2 weeks, 1-3 weeks, 1-4 weeks, 1-2 months, 1-4 months, 1-6 months, 2-6 months, 3-6 months, 3-9 months, 4-8 months, 6-12 months, 1-2 years, 1-5 years, 2-5 years, 3-6 years, 3-8 years, 4-8 years, 5-10 years, 10-20 years, 10-30 years, 20-40 years, 20-50 years, or the lifetime of the subject.

Inverted terminal repeat (ITR)

The vector genome (or viral genome) of the polynucleotide or AAV particle according to any one of the foregoing of the present disclosure comprises at least one ITR, e.g., has two ITRs flanking a payload at the 5' end and the 3' end, respectively.

In some embodiments, the ITRs have origin of replication function.

In some embodiments, the ITRs comprise sequence regions that may be complementary and symmetrically arranged.

In some embodiments, the ITRs may consist of naturally occurring polynucleotide sequences or recombinantly derived polynucleotide sequences.

In some embodiments, the ITRs may be derived from the same or different serotype as the capsid. In some embodiments, the 5' ITR and the 3' ITR may be derived from the same serotype or may be derived from different serotypes. For example, both 5' ITR and 3' ITR are from AAV2, or both are from AAV9. As another example, the 5' ITR comprises the sequence set forth in SEQ ID NO: 76, and/or the 3' ITR comprises the nucleotide sequence set forth in SEQ ID NO: 87.

In some embodiments, the length of each ITR may be about 100 to about 150 nucleotides, such as 100-105 nucleotides, 106-110 nucleotides, 111-115 nucleotides, 116-120 nucleotides, 121-125 nucleotides, 126-130 nucleotides, 131-135 nucleotides, 136-140 nucleotides, 141-145 nucleotides, or 146-150 nucleotides. In one embodiment, the length of the ITR is 140-142 nucleotides, such as 141 nucleotides. Non-limiting examples of the length of the ITR are 102 nucleotides, 140 nucleotides, 141 nucleotides, 142 nucleotides, 145 nucleotides, and nucleotides having at least 95% identity thereto.

Promoter

The vector genome (or viral genome) of the polynucleotide or AAV particle according to any one of the foregoing of the present disclosure comprises at least one promoter, including but not limited to, species-specific promoters, inducible promoters, tissue-specific promoters, or cell cycle-specific promoters.

In some embodiments, the promoter drives expression of a protein or polypeptide (e.g., a VEGF-binding molecule) encoded in a payload of the vector genome of the AAV particle. In some embodiments, the promoter is specific to or has tropism for a target tissue, such as a promoter capable of expressing a payload in an eye tissue.

In some embodiments, the promoter may be a viral promoter, a plant promoter, a mammalian promoter, or a human promoter.

In some embodiments, the promoter includes, but is not limited to, CMV, CBA (including derivatives CAG, CBh, etc.), EF-1α, PGK, UBC, RSV, EFS, EF1, GUSB (hGBp), UCOE (the promoter of HNRPA2B1-CBX3), NSE, Synapsin, MeCP2, MeP418, MeP426, VMD2, MRHO, TRE, Ac5, Polyhedrin, CaMKIIa, Gall, TEF1, GDS, ADHI, Ubi, GFAP, or PKG promoters, and may be selected from the group consisting of neurofilament light (NFL) promoters, neurofilament heavy (NFH) promoters, SCN8A promoters, frataxin (FXN) promoters (or known as FRDA promoters), H1 promoters, RNA pol III promoters (e.g., U6 or H1), and small nuclear RNA (ULB or ULA) promoters. In some embodiments, the promoter is a liver or skeletal muscle promoter; the liver promoter is, for example, human $\alpha$-1-antitrypsin (hAAT) and thyroxine-binding globulin (TBG), and the skeletal muscle promoter is, for example, desmin, MCK, or synthetic C5-12.

In some embodiments, the AAV vector genome of the present disclosure comprises two promoters, for example, a CMV promoter and a CBA promoter, the CMV sequence being set forth in SEQ ID NO: 75; as another example, an EF1$\alpha$ promoter and a CMV promoter, or a combination of any two of the aforementioned promoters.

In some embodiments, the promoter is a tissue-specific expression element that can limit expression to certain cell types, including but not limited to, a muscle-specific promoter, a B cell promoter, a monocyte promoter, a leukocyte promoter, a macrophage promoter, a pancreatic acinar cell promoter, an endothelial cell promoter, a lung tissue promoter, an astrocyte promoter, or a nervous system promoter, which can be used to limit expression to neurons, astrocytes, or oligodendrocytes.

In some embodiments, the promoter is a truncation or a variant of the aforementioned promoters, having a length of less than 1 kb, such as 200-300, 200-400, 300-400, 200-500, 200-600 or more.

In some embodiments, the promoter may be a combination of two or more components of the same or different initial or parental promoters, such as CMV and CBA.

Enhancer

The vector genome (or viral genome) of the polynucleotide or AAV particle according to any one of the foregoing of the present disclosure comprises at least one enhancer, including but not limited to, species-specific enhancers, inducible enhancers, tissue-specific enhancers, or cell cycle-specific enhancers.

In some embodiments, the enhancer is specific to or has tropism for a target tissue, such as an enhancer capable of regulating expression of a payload in a neural tissue.

In some embodiments, the enhancer may be or be derived from a viral enhancer, a plant enhancer, a mammalian enhancer, or a human enhancer.

In some embodiments, the enhancer may be located upstream or downstream of a promoter and operatively linked to the promoter. Expression of a target gene (e.g., a VEGF-binding molecule) is enhanced by at least 20%, at least 50%, at least 80%, at least 100%, or at least several times (including 1, 2, 3, 4, 5, 6, 7, 8, 9, 10 times or more) when the enhancer is present as compared to when the enhancer is not present.

In some embodiments, the enhancer includes, but is not limited to, EF-1$\alpha$, Ubc, humanb-actin, CAG, TRE, Ac5, Polyhedrin, CaMKIIa, Gall, TEF1, GDS, ADH1, Ubi, and $\alpha$-1-antitrypsin (hAAT), or variants or fragments of the above enhancers. In some embodiments, the enhancer is selected from the group consisting of IRBP, RSV, or CMV enhancers, or variants or fragments thereof.

In some embodiments, the enhancer comprises or is an enhancer set forth in SEQ ID NO: 74 or a variant or fragment thereof.

Intron

An intron or a portion thereof may be comprised in the vector genome (or viral genome) of the polynucleotide or AAV particle according to any one of the foregoing of the present disclosure.

In some embodiments, the intron includes, but is not limited to, MVM (67-97 bp), F.IX truncated intron 1 (about 300 bp), $\beta$-globin SD/immunoglobulin heavy chain splice acceptor (about 250 bp), adenovirus splice donor/immunoglobulin splice acceptor (about 500 bp), SV40 late splice donor/splice acceptor (19S/16S) (180 bp) and hybrid adenovirus splice donor/IgG splice acceptor (about 230 bp), hemoglobin introns, hybrid introns, $\beta$-globin introns, or variants or fragments of the above introns.

In some embodiments, the length of the intron is 100-800 nucleotides, such as about 100 nucleotides, about 200 nucleotides, about 300 nucleotides, about 400 nucleotides, about 500 nucleotides, and about 600 nucleotides, and further such as 200-250 nucleotides, 200-300 nucleotides, 100-200 nucleotides, 300-500 nucleotides, 500-600 nucleotides, 400-600 nucleotides, and 100-200 nucleotides.

In some embodiments, the intron in the technical solutions of the present disclosure is selected from the group consisting of a hybrid intron and a $\beta$-globin intron.

Untranslated region (UTR)

An untranslated region (UTR) may be comprised in the vector genome (or viral genome) of the polynucleotide or AAV particle according to any one of the foregoing of the present disclosure, wherein the UTR is selected from the group consisting of a 5' UTR and/or a 3' UTR, to regulate (e.g., increase or decrease) polynucleotide stability and protein production. Typically, the 5' UTR starts at a transcription start site and terminates at a start codon, and the 3' UTR starts immediately after a stop codon and terminates at a transcription termination signal. The UTR may be a wild type, a variant thereof, or an artificial UTR.

In some embodiments, the 5' UTR comprises a Kozak sequence. In other embodiments, the 5' UTR does not comprise a Kozak sequence.

In some embodiments, the 3' UTR is rich in AU. In some embodiments, the 3' UTR is selected from the group consisting of: class I AREs, for example, but not limited to, c-Myc and MyoD, containing several dispersed copies of AUUUA motifs within the U-rich region; class II AREs, for example, but not limited to, GM-CSF and TNF-a, possessing two or more overlapping UUAUUUA(U/A)(U/A) nonamers; and class III ARES, for example, but not limited to, c-Jun and myogenin.

In some embodiments, the 3' UTR may comprise an oligomeric (dT) sequence for templated addition of a polyadenylation sequence (PolyA).

Polyadenylation sequence (PolyA)

A sequence encoding a PolyA may be comprised in the vector genome (or viral genome) of the polynucleotide or AAV particle according to any one of the foregoing of the present disclosure, wherein the PolyA may be a wild type, a variant thereof, or a modified PolyA, for protein translation.

In some embodiments, the sequence encoding the PolyA is located between the 3' end of a coding sequence of a payload and the 5' end of a 3' ITR.

In some embodiments, the length of the sequence encoding the PolyA is 0-500 nucleotides, such as about 75 nucleotides, about 100 nucleotides, about 110 nucleotides, about 120 nucleotides, about 150 nucleotides, about 160 nucleotides, about 200 nucleotides, or about 300 nucleotides, further such as 50-100, 50-150, 50-160, 50-200, 60-100, 60-150, 60-160, 60-200, 70-100, 70-150, 70-160, 70-200, 80-100, 80-150, 80-160, 80-200, 90-100, 90-150, 90-160, or 90-200.

In some embodiments, the encoding PolyA may encode continuous PolyA or discontinuous PolyA. When the discontinuous PolyA is encoded, the coding sequence may be interrupted or separated by other nucleotides. For example, the polyA has at least two 60-adenylate fragments that are separated by a sequence comprising 10-90 nucleotides.

In some embodiments, the sequence encoding the PolyA or polyA in the technical solutions of the present disclosure is selected from the group consisting of β-globin polyA, SV40 polyA, bGH polyA, PA75 polyA, MeCP2 polyA, RDH1 polyA, BGH poly A, SPA49 poly A, sNRP-TK65 poly A, sNRP poly A, TK65 poly A, or variants or fragments of the above PolyA.

In some embodiments, the polyA set forth in SEQ ID NO: 89 or a variant or fragment thereof in the technical solutions of the present disclosure.

polyA or coding sequences thereof in WO2016005324, WO2016005004, WO2016091391, WO2019036513, and WO2020074642 are incorporated herein by reference in their entirety, all of which may be used in the technical solutions of the present disclosure.

Stuffer sequence

A stuffer sequence may be comprised in the vector genome (or viral genome) of the polynucleotide or AAV particle according to any one of the foregoing of the present disclosure, such that the length of the vector genome is the optimal size for packaging, for example, such that the length of the vector genome is about 2.3 kb, about 4.6 kb, about 4.7 kb, or about 5.1 kb.

In some embodiments, the vector genome is a single-stranded or double-stranded genome, and the packaged AAV particle is ssAAV or scAAV.

In some embodiments, one vector genome may comprise one or more (e.g., 1, 2, 3, 4, 5, 6, 7, 8, 9, or 10) stuffer sequences.

In some embodiments, the stuffer sequence may be located between or within a plurality of regulatory elements, e.g., between two introns, at 3' of a 5' ITR sequence, at 5' of a 5' ITR sequence, at 5' of a 3' ITR sequence, at 3' of a 3' ITR sequence, and before or after a promoter, an intron, an enhancer, a polyA, a multiple cloning site (MCS) region, an exon, or other regions.

Production or Preparation Method for Recombinant Adeno-Associated Viral (rAAV) Particles

The present disclosure provides methods for producing, preparing, and/or modifying rAAV particles. The production, preparation, and/or modification methods for rAAV particles in WO200028004, WO200123001, WO2004112727, WO 2005005610, WO2005072364, WO2013123503, WO2015191508, and US20130195801 are incorporated herein by reference in their entirety. The rAAV particles may have enhanced delivery efficiency, may be efficiently packaged, and may successfully infect a target cell (e.g., a mammalian or human cell) with high frequency and minimum toxicity.

In some embodiments, provided is a production method for rAAV particles, comprising packaging any of the nucleic acids or vector genomes (or viral genomes) or vectors of the present disclosure into an AAV capsid. In some specific embodiments, the method comprises the following steps:

1) co-transfecting competent bacterial cells with a baculovirus vector and a viral construct vector and/or an AAV payload construct vector,

2) isolating the resulting viral construct expression vector and AAV payload construct expression vector and separately transfecting viral replication cells,

3) isolating and purifying the resulting payload and viral construct particles comprising the viral construct expression vector or AAV payload construct expression vector,

4) co-infecting viral replication cells with both the AAV payload and the viral construct particles comprising the viral construct expression vector or AAV payload construct expression vector, and

5) harvesting and purifying AAV particles comprising a viral genome.

In some embodiments, provided is a production method for rAAV particles, comprising the following steps:

1) co-transfecting a mammalian cell (e.g., an HEK293 cell) with any of the nucleic acids or vector genomes (or viral genomes) or vectors of the present disclosure, and a construct expressing Rep and Cap genes and a helper construct (to achieve helper functions) simultaneously; and

2) harvesting and purifying rAAV particles comprising a viral genome.

In some embodiments, the viral genome of the aforementioned rAAV particles optionally encodes a selection marker. The selection marker may include a cell surface marker, such as any protein expressed on the cell surface, including but not limited to, a receptor CD marker, a lectin, an integrin, or truncated forms thereof.

In some embodiments, provided is an AAV production system for producing the rAAV particles of the present disclosure, wherein the production system comprises:

1) a nucleic acid sequence encoding an AAV capsid;

2) any of the nucleic acids or vector genomes (or viral genomes) or vectors of the present disclosure; and

3) a helper packaging element having sufficient AAV rep function and helper function that allow packaging of the nucleic acid or vector genome (or viral genome) or vector in (b) into an AAV capsid.

The nucleic acid or vector genome (or viral genome) or vector comprises any of the aforementioned nucleic acids encoding the VEGF-binding molecule of the present disclosure.

The AAV capsid is selected from the group consisting of any of the aforementioned AAV capsids of the present disclosure, e.g., AAV1, AAV2, AAV3, AAV4, AAV5, AAV6, AAV7, AAV8, AAV9, AAV9.47, AAV9 (hu14), AAV10, AAV11, AAV12, AAVrh8, AAVrh10, AAV-DJ, AAV-DJ8, or a variant of any one of the foregoing.

In some specific embodiments, the sufficient AAV rep function and helper function of the helper packaging plasmid including element are provided by a packaging cell, wherein the packaging cell may comprise any one or more of three plasmids pHelper, pRC9, and pGOI.

Illustratively, "helper function" or "helper virus function" refers to a function encoded in a helper virus genome which allows AAV replication and packaging (in conjunction with other requirements for replication and packaging described herein). In the present disclosure, "helper virus function" can be provided in a number of ways, including by providing a helper virus or providing, for example, polynucleotide sequences encoding the requisite functions to a producer cell in transport.

Illustratively, "AAV rep function" refers to functions provided by Rep genes and/or Cap genes.

In some embodiments, the Rep genes encode the non-structural proteins that regulate functions such as the replication of the AAV genome, and may be selected from the group consisting of Rep78, Rep68, Rep52, and Rep40. Rep78 and Rep68 are generally transcribed from the p5 promoter, while Rep52 and Rep40 are generally transcribed from the p19 promoter. The Cap genes encode the structural proteins VP1, VP2, and/or VP3 that assemble to form the viral capsid. The Cap genes are generally transcribed from the p40 promoter.

In some embodiments, provided are rAAV particles produced by the AAV production system described above.

## Host Cell and Preparation Method

The present disclosure further provides a host cell, e.g., an isolated (genetically modified) host cell, comprising any one of the nucleic acids described above. The host cell may be an isolated cell, e.g., a cell cultured *in vitro.* The host cell is capable of expressing one or more of the VEGF-binding molecules of the present disclosure or conjugates thereof, and/or comprising the nucleic acid or vector of the present disclosure.

In some embodiments, the host cell includes prokaryotic microorganisms (e.g., Escherichia coli) or various eukaryotic cells (e.g., Chinese hamster ovary cells (CHO), human embryonic kidney (HEK) cells, or lymphocytes (e.g., Y0, NS0, and Sp20 cells), and insect cells).

In some embodiments, host cells expressing glycosylated polypeptides may be used; the host cells are derived from multicellular organisms (including, for example, invertebrates and vertebrates), such as plant and insect cells. Vertebrate cells may also be used as host cells, for example, mammalian cell lines grown in suspension, monkey kidney CV1 lines (COS-7), human embryonic kidney lines (293 or 293T cells), baby hamster kidney cells (BHK), mouse sertoli cells (TM4 cells), monkey kidney cells (CV1), African green monkey kidney cells (VERO-76), human cervical cancer cells (HELA), canine kidney cells (MDCK), buffalo rat liver cells (BRL3A), human lung cells (W138), human liver cells (Hep G2), mouse mammary tumor cells (MMT060562), TRI cells (as described, for example, in Mather et al., Annals N.YAcad Sci383, 44-68(1982)), MRC5 cells and FS4 cells, Chinese hamster ovary (CHO) cells, myeloma cell lines such as YO, NS0, P3X63, and Sp2/0, and cells contained in transgenic animals, transgenic plants, or cultivated plant or animal tissues.

As described below, the host cell can be used to produce the subject rAAV virion. When the host cell is used to produce the subject rAAV virion, it is referred to as a "packaging cell". In some embodiments, the host cell is stably genetically modified with any one of the foregoing. In other embodiments, the host cell is transiently genetically modified with any one of the foregoing.

Any one of the foregoing is introduced stably or transiently into the host cell using established techniques, including, but not limited to, electroporation, calcium phosphate precipitation, liposome-mediated transfection, and the like. For stable transformation, any one of the foregoing will generally further comprise a selectable marker, such as any one of several well-known selectable markers, such as Normocin resistance.

The subject host cell is generated by introducing any one of the foregoing into any one of a variety of cells, e.g., mammalian cells, including (for example) murine cells and primate cells (e.g., human cells). Suitable mammalian cells include, but are not limited to, primary cells and cell lines, wherein suitable cell lines include, but are not limited to, 293 cells, COS cells, HeLa cells, Vero cells, 3T3 mouse fibroblasts, C3H10T1/2 fibroblasts, CHO cells, and the like. Non-limiting examples of suitable host cells include (for example) HeLa cells (e.g., American Type Culture Collection (ATCC) No. CCL-2), CHO cells (e.g., ATCC Nos. CRL9618, CCL61, and CRL9096), 293 cells (e.g., ATCC No. CRL-1573), Vero cells, NIH3T3 cells (e.g., ATCC No. CRL-1658), Huh-7 cells, BHK cells (e.g., ATCC No. CCL10), PC12 cells (ATCC No. CRL1721), COS cells, COS-7 cells (ATCC No. CRL1651), RAT1 cells, mouse L cells (ATCC No. CCLI.3), human embryonic kidney (HEK) cells (ATCC No. CRL1573), HLHepG2 cells, and the like. The subject host cell may also be prepared using a baculovirus to infect AAV-producing insect cells, such as Sf9 cells (see, e.g., U.S. Pat. No. 7,271,002; U.S. Pat. Publication 12/297,958).

In some embodiments, the genetically modified subject host cell also comprises, in addition to a nucleic acid comprising a nucleotide sequence encoding a variant AAV capsid protein as described above, a nucleic acid comprising a nucleotide sequence encoding one or more AAV rep proteins. In other embodiments, the subject host cell further comprises an rAAV vector. An rAAV virion can be generated using the host cell. Methods of generating an rAAV virion are described in, e.g., U.S. Pat. Publication No. 2005/0053922 and U.S. Pat. Publication No. 2009/0202490.

In some embodiments, a unit dose of the pharmaceutical composition may comprise 0.01 to 99 weight% of a polynucleotide (including a VEGF-binding molecule) or rAAV particles. In some other specific embodiments, a unit dose of the pharmaceutical composition comprises 0.1 to $10 \times 10^{13}$ copies of the VEGF-binding molecule transgene.

## Preparation Method

The present disclosure also provides a method for preparing the VEGF-binding molecule of the present disclosure, comprising:

- culturing the host cell of the present disclosure under conditions that allow expression of the VEGF-binding molecule of the present disclosure; and
- collecting the VEGF-binding molecule expressed by the host cell from the culture; and
- optionally, further purifying and/or modifying the VEGF-binding molecule of the present disclosure.

The VEGF-binding molecule of the present disclosure can be produced intracellularly (e.g., in the cytoplasm, in the periplasm, or in inclusion bodies) in a cell as described above, followed by isolation from the host cell and optionally further purification; or it may be produced extracellularly (e.g., in the medium in which the host cell is cultured), followed by isolation from the medium and optionally further purification. For example, purification is performed using an A or G Sepharose FF column containing an adjusted buffer to wash off non-specifically bound components, and then bound antibodies are eluted by a pH gradient method, detected by SDS-PAGE, and collected. Optionally, the antibody solution can be filtered and concentrated by conventional methods. Soluble mixtures and polymers can also be removed by conventional methods, such as molecular sieves and ion exchange. The resulting product needs to be immediately frozen, e.g., at - 70 °C, or lyophilized.

Methods and reagents for recombinant production of polypeptides, e.g., specific suitable expression vectors, transformation or transfection methods, selection labels, methods for inducing protein expression, culture conditions, and the like, are known in the art. Similarly, protein isolation and purification techniques suitable for use in the method for producing the VEGF-binding molecule of the present disclosure are well known to those of skill in the art.

## Composition

The present disclosure also provides a composition, e.g., a pharmaceutical composition, comprising an effective amount of the VEGF-binding molecule, isolated nucleic acid, or recombinant adeno-associated viral vector of the present disclosure, and one or more pharmaceutically acceptable carriers, diluents, buffers, or excipients.

In some specific embodiments, a unit dose of the pharmaceutical composition may comprise 0.01 to 99 weight% of the isolated nucleic acid or the recombinant adeno-associated viral vector, or a unit dose of the pharmaceutical composition comprises 0.1-2000 mg (e.g., 1-1000 mg) of the isolated nucleic acid or the recombinant adeno-associated viral vector.

In some specific embodiments, the pharmaceutical composition may be a lyophilized formulation or an injectable solution.

## Treatment Method and Pharmaceutical Use

The present disclosure also provides use of any one of the aforementioned VEGF-binding molecules, isolated nucleic acids, or recombinant adeno-associated viral vectors in the preparation of a medicament for treating and/or preventing a disease associated with abnormal angiogenesis.

The present disclosure also provides a method for inhibiting VEGF receptor ligand activity in a subject, comprising administering to a subject in need thereof a therapeutically effective amount of any one of the aforementioned VEGF-binding molecules, isolated nucleic acids, recombinant adeno-associated viral vectors, or pharmaceutical compositions.

The present disclosure also provides a method for treating and/or preventing a disease associated with abnormal angiogenesis, comprising administering to a subject in need thereof a therapeutically effective amount of any one of the aforementioned VEGF-binding molecules, isolated nucleic acids, recombinant adeno-associated viral vectors, or pharmaceutical compositions.

The present disclosure also provides a VEGF-binding molecule, an isolated nucleic acid, or a recombinant adeno-associated viral vector for use as a medicament.

The present disclosure also provides a VEGF-binding molecule, an isolated nucleic acid, or a recombinant adeno-associated viral vector, or a pharmaceutical composition comprising same for use in treating and/or preventing a disease associated with abnormal angiogenesis.

In some embodiments, the subject is a mammal.

In some embodiments, the subject is a human.

In some embodiments, the disease associated with abnormal angiogenesis is an angiogenic ocular disease or cancer.

In some embodiments, the angiogenic ocular disease is selected from the group consisting of macular degeneration (e.g., age-related macular degeneration (AMD), e.g., wet age-related macular degeneration or dry age-related macular degeneration), macular edema (e.g., macular edema following retinal vein occlusion, or diabetic macular edema), retinal vein occlusion (e.g., central retinal vein occlusion, branch retinal vein occlusion), choroidal neovascularization, iris neovascularization, neovascular glaucoma, postoperative fibrosis in glaucoma, proliferative vitreoretinopathy, optic disc neovascularization, corneal neovascularization, retinal neovascularization, vitreous neovascularization, pannus, pterygium, choroidal retinopathy, retinopathy of prematurity, vascular retinopathy, diabetic retinopathy, non-proliferative diabetic retinopathy, and proliferative diabetic retinopathy.

In some embodiments, the disease associated with abnormal angiogenesis is wet AMD or dry AMD.

In some embodiments, the cancer includes, but is not limited to, non-small cell lung cancer, kidney cancer, uterine cancer, prostate cancer, bladder cancer, ovarian cancer, colon cancer, breast cancer, leukemia, lymphoma, myeloma, sarcoma (e.g., fibrosarcoma, myxosarcoma, liposarcoma, lymphatic endotheliosarcoma, angiosarcoma, endotheliosarcoma, chondrosarcoma, osteosarcoma, chordoma, lymphangiosarcoma, synovioma, mesothelioma, leiomyosarcoma, or rhabdomyosarcoma), neuroglioma, pancreatic cancer, squamous cell carcinoma, basal cell carcinoma, adenocarcinoma, sweat gland cancer, sebaceous carcinoma, papillary carcinoma, papillary adenocarcinoma, cystadenocarcinoma, medullary carcinoma, bronchopulmonary carcinoma, choriocarcinoma, renal cell carcinoma, liver cancer, bile duct cancer, seminoma, embryonal carcinoma, cervical cancer, testicular tumor, lung cancer, small cell lung cancer, bladder cancer, epithelial cancer, neuroglioma, astrocytoma, ependymoma, pinealoma, hemangioblastoma, acoustic neuroma, medulloblastoma, craniopharyngioma, oligodendroglioma, meningioma, melanoma, neutrophilic tumor, and retinoblastoma.

The present disclosure also provides a method for inhibiting abnormal angiogenesis, comprising administering to a subject in need thereof a therapeutically effective amount of any one of the aforementioned VEGF-binding molecules, isolated nucleic acids, or recombinant adeno-associated viral vectors or pharmaceutical compositions.

The methods and use described herein may be practiced in combination with other therapeutic agents or therapies (e.g., radiotherapy).

**Kit and Device**

The present disclosure provides a kit and a device comprising any of the aforementioned VEGF-binding molecules, nucleic acids, rAAV particles, and pharmaceutical compositions of the aforementioned disclosure.

In some embodiments, the kit comprises a container device generally including at least one vial, test tube, flask, bottle, syringe, or other container devices, into which the nucleic acid, rAAV particles, or pharmaceutical composition may be placed. Generally, the kit may also comprise a second, third, or other additional containers in which additional components may be separately placed, for example, for containing sterile pharmaceutically acceptable buffers and/or other diluents. In some embodiments, the kit comprises a package insert.

In some embodiments, the VEGF-binding molecule, nucleic acid, rAAV particles, and pharmaceutical composition of the present disclosure may be combined with, applied to, or embedded in the device. The device may include, but is not limited to, a stent, a pump, and/or other implantable therapeutic devices. In addition, the nucleic acid, rAAV particles, and pharmaceutical composition may be delivered to a subject when the subject uses a compression device, for example, but not limited to, a compression device to reduce the chance of deep vein thrombosis (DVT) in the subject.

In some embodiments, the nucleic acid, rAAV particles, or pharmaceutical composition of the present disclosure may be delivered using devices, including but not limited to, stents, tubes (including catheters, pipelines, and straws), and gene guns. The nucleic acid, rAAV particles, or pharmaceutical composition of the present disclosure may be administered to a subject using a delivery system that integrates image-guided therapy and integrates imaging, including but not limited to, a laser, MRgFUS, an endoscope, and a robotic surgical device. The nucleic acid, rAAV particles, or pharmaceutical composition of the present disclosure may be administered to a subject using an automated delivery system. The nucleic acid, rAAV particles, or pharmaceutical composition of the present disclosure may be delivered using device localization to a target site of a subject, an enhanced delivery device, or an MIR guide device.

**BRIEF DESCRIPTION OF THE DRAWINGS**

FIG. 1: a schematic diagram of molecular formats of VEGF-binding molecules.
FIG. 2: a schematic diagram of the structure of a vector comprising a nucleic acid molecule expressing a VEGF-binding molecule.
FIG. 3: a schematic diagram of an AAV packaging plasmid.
FIG. 4: stability test results for VEGF-binding molecules in the vitreous humor of both eyes of rabbits.
FIG. 5A: fluorescein fundus angiography (FFA) results 7 days after rat CNV modeling and 14 days after rat CNV model administration.
FIG. 5B: the inhibition of choroidal neovascularization fluorescein sodium leakage 14 days after rat CNV model administration.
FIG. 6: the expression of AAV-1174 and ADVM-022 in the aqueous humor, the vitreous humor, the retina, and the choroid of rabbit eyes.
FIG. 7: the inhibition of choroidal neovascularization fluorescein sodium leakage after administration of AAV-1174 and ADVM-022 to a mouse CNV model.

## DETAILED DESCRIPTION

### Definition

In order to facilitate the understanding of the present disclosure, some technical and scientific terms are specifically defined below. Unless otherwise specifically defined herein, all other technical and scientific terms used herein have the meanings generally understood by those of ordinary skills in the art to which the present disclosure belongs. VEGFR-1, VEGFR1, Flt1, and Flt-1 are used interchangeably and all refer to one VEGF receptor. The amino acid sequence of wild-type VEGFR-1 is set forth in SEQ ID NO: 2. VEGFR-2, VEGFR2, Flk-1, and KDR are used interchangeably and all refer to another VEGF receptor. The amino acid sequence of wild-type VEGFR-2 is set forth in SEQ ID NO: 1.

"Wild-type" refers to a molecular format that is identical to the VEGF-binding molecule of the present disclosure in other aspects (such as domain composition, sequence length, and linkage mode), except that it has a wild-type amino acid at each amino acid position of the VEGF-binding molecule. For example, if the VEGF-binding molecule is a polypeptide of molecular format 1 (VEGFR1 D2 + VEGFR2 D3 + VEGFR2 D2D3 + the hinge region and CH2CH3 constant region of human IgG1), then the corresponding wild-type one is a polypeptide that has this molecular format 1 and each domain is a native amino acid sequence.

"Amino acid mutations" include amino acid substitutions, deletions, insertions, modifications, and any combination thereof, to obtain a final construct that possesses desired properties, such as enhanced stability. Amino acid sequence deletions and insertions include amino-terminal and/or carboxyl-terminal deletions and amino acid insertions. To alter the binding properties of, for example, a VEGF-binding molecule (or a VEGFR fusion protein), non-conservative amino acid substitutions can be made; that is, one amino acid is replaced with another amino acid that has different structural and/or chemical properties. Preferred amino acid substitutions include the replacement of hydrophobic amino acids with hydrophilic amino acids. Amino acid substitutions include the replacement with non-naturally occurring amino acids or with naturally occurring amino acid derivatives of the 20 standard amino acids (e.g., 4-hydroxyproline, 3-methylhistidine, ornithine, homoserine and 5-hydroxylysine). Amino acid mutations can be generated using genetic or chemical methods well known in the art, including site-directed mutagenesis, PCR, gene synthesis, chemical modification, and the like. In case of amino acid replacements, the replacements will preferably be conservative amino acid replacements. The conservative amino acid means that an amino acid residue is substituted with another amino acid residue that is similar in chemical structure and has little or essentially no influence on the function, activity, or other biological properties of the polypeptide. The conservative amino acid replacements are well known in the art; for example, conservative amino acid substitutions are preferably substitution of one amino acid from the following groups (i)-(v) with another amino acid residue from the same group:

(i) relatively small, aliphatic, nonpolar or weakly polar residues: Ala, Ser, Thr, Pro, and Gly;
(ii) polar, negatively charged residues and (uncharged) amides thereof: Asp, Asn, Glu, and Gln;
(iii) polar, positively charged residues: His, Arg, and Lys;
(iv) relatively large, aliphatic, nonpolar residues: Met, Leu, Ile, Val, and Cys; and
(v) aromatic residues: Phe, Tyr, and Trp.

For example, conservative amino acid substitutions are as follows: substitution of Ala with Gly or Ser; substitution of Arg with Lys; substitution of Asn with Gln or His; substitution of Asp with Glu; substitution of Cys with Ser; substitution of Gln with Asn; substitution of Glu with Asp; substitution of Gly with Ala or Pro; substitution of His with Asn or Gin; substitution of Ile with Leu or Val; substitution of Leu with Ile or Val; substitution of Lys with Arg, Gln, or Glu; substitution of Met with Leu, Tyr, or Ile; substitution of Phe with Met, Leu, or Tyr; substitution of Ser with Thr; substitution of Thr with Ser; substitution of Trp with Tyr; substitution of Tyr with Trp or Phe; and substitution of Val with Ile or Leu.

"Homology", "identity", or "sequence identity" refers to the overall correlation between polymer molecules (e.g., oligonucleotides, polynucleotides, or polypeptides). For example, the percent identity of two polynucleotide sequences may be calculated by aligning the two. The numerical value of identity may differ due to gaps and penalties introduced in the calculation, but the best way may be selected (e.g., a gap may be introduced in one or both of two sequences to be aligned to achieve the best alignment, and the different sequences may be ignored to achieve the comparison, one of the sequences being a target sequence to be aligned and the other being a reference sequence). In some embodiments, the length of the target sequence to be aligned may be at least 30%, at least 40%, at least 50%, at least 60%, at least 70%, at least 80%, at least 90%, at least 95%, or 100% of the length of the reference sequence, and then the nucleotides at the corresponding nucleotide positions are compared. When a position in the target sequence to be aligned is occupied by the identical nucleotide at the corresponding position in the reference sequence, the two are identical at this position. The percent identity between two sequences is a function of the number of identical positions shared by the sequences, taking into account the number of gaps and the length of each gap, wherein the gaps need to be introduced to achieve the optimal alignment of the two sequences. The comparison of sequences and determination of percent identity between two sequences may be accomplished using a mathematical algorithm. For example, the percent identity between two polynucleotide sequences may be determined using methods such as those described in the following: Computational Molecular Biology, Lesk, A.M., ed., Oxford University Press, New York, 1988; Biocomputing: Informatics and Genome Projects, Smith, D.W., ed., Academic Press, New York, 1993; Sequence Analysis in Molecular Biology, von Heinje, G., Academic Press, 1987; Computer Analysis of Sequence Data, Part I, Griffin, A.M., and Griffin, H.G., eds., Humana Press, New Jersey, 1994; and Sequence Analysis Primer, Gribskov, M. and Devereux, J., eds., M Stockton Press, New York, 1991 (all incorporated in the present disclosure in their entirety). For example, the percent identity between two nucleotide sequences may be determined using the Meyers and Miller (CABIOS, 1989, 4:11-17) algorithm, which has been incorporated into the ALIGN program (version 2.0), and using a PAM120 weight residue table with a gap length penalty of 12 and a gap penalty of 4. Alternatively, the percent identity between two nucleotide sequences may be determined using NWSgapdna.CMP matrix and using the GAP program in the GCG software package. Methods commonly used to determine the percent identity between sequences include, but are not limited to, Carillo, H., and Lipman, D., SIAM J Applied Math., 48:1073.

"Binding activity" or "affinity" refers to the strength of the sum of noncovalent interactions between a single binding site of a molecule (e.g., a receptor or the binding molecule of the present disclosure) and its binding partner (e.g., a ligand). Unless indicated otherwise, the "binding affinity" herein refers to intrinsic affinity which reflects a 1:1 interaction between members of a binding pair (e.g., an antibody and an antigen). The binding activity (affinity) of a molecule X for its partner Y can generally be represented by the dissociation constant (KD), which is the ratio of the dissociation rate constant to the association rate constant ($K_d$ and $K_a$, respectively). Thus, equal binding activity (affinity) may comprise different rate constants, as long as the ratio of rate constants remains the same. Binding activity (affinity) can be measured by conventional methods in the art, including the methods described in Example 3 or Example 5 herein. "Stability" means that the VEGF-binding molecule can achieve the effect of being stable: after a period (e.g., 2 weeks, 4 weeks, 8 weeks, 12 weeks, or even longer) of storage, it essentially retains its physical stability and/or chemical stability and/or biological activity; for example, it essentially retains its physical and chemical stability and its biological activity. There are a variety of analytical techniques currently available for measuring polypeptide stability, and the stability after storage for a selected period of time at a selected temperature can be measured. A stable VEGF-binding molecule is a substance in which no significant change is observed under the following conditions: storage at refrigeration temperature (2-8 °C) for at least 1 month, at least 3 months, at least 6 months, at least 1 year, at least 2 years, or even longer. Stable VEGF-binding molecules also include exhibiting desired characteristics after storage at a temperature of 25 °C or 40 °C for at least 1 month, at least 2 months, at least 3 months, at least 4 months, at least 5 months, at least 6 months, or even longer. Typical acceptable criteria for stability are as follows: aqueous solutions or formulations of VEGF-binding molecules are colorless, or clear to slightly opalescent, by visual analysis. The concentration, pH and osmolality of the formulation have changes of no more than ±10%. Generally, no more than about 10%, preferably no more than about 5%, of clipping is observed. Generally, no more than about 10%, preferably no more than about 5%, of aggregation is formed. A polypeptide "retains its physical stability" if it shows no significant increase in aggregation, precipitation, and/or denaturation upon visual inspection of color and/or clarity, or as determined by UV light scattering, size exclusion chromatography (SEC), and dynamic light scattering (DLS). Changes in the conformation of a polypeptide can be evaluated by fluorescence spectroscopy (which determines the tertiary structure of the polypeptide) and by FTIR spectroscopy (which determines the secondary structure of the polypeptide). A polypeptide "retains its chemical stability" if it shows no significant chemical alteration. Chemical stability can be assessed by detecting and quantifying chemically altered forms of the polypeptide. Degradation processes that often alter the chemical structure of polypeptides include hydrolysis or clipping (evaluated by methods such as size exclusion chromatography and SDS-PAGE), oxidation (evaluated by methods such as peptide mapping in conjunction with mass spectroscopy or MALDI/TOF/MS), deamidation (evaluated by methods such as ion-exchange chromatography, capillary isoelectric focusing, peptide mapping, isoaspartic acid measurement), and isomerization (evaluated by measuring the isoaspartic acid content, peptide mapping, etc.). A polypeptide "retains its biological activity" if the biological activity of the polypeptide at a given time is within a predetermined range. The biological activity of a polypeptide can be determined, for example, by an antigen-binding assay.

"Antibody" is used herein in the broadest sense and encompasses a variety of antibody structures as long as they exhibit the desired biological activity. The antibodies include, but are not limited to, monoclonal antibodies, polyclonal antibodies, multispecific antibodies (e.g., bispecific antibodies), and antigen-binding fragments. Antibodies may include murine, human, humanized, chimeric, and camelid antibodies. By way of example, an antibody may be an immunoglobulin, a four-peptide-chain structure formed by linking two heavy chains and two light chains by interchain disulfide bonds. The heavy chain constant regions of an immunoglobulin differ in their amino acid composition and arrangement, and thus in their antigenicity. Accordingly, immunoglobulins can be divided into five classes, otherwise called isotypes of immunoglobulins, namely IgM, IgD, IgG, IgA and IgE, with their corresponding heavy chains being μ chain, δ chain, γ chain, α chain, and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3, and IgG4. Light chains are divided into x or λ chains according to differences in the constant regions. Each of the five classes of Ig may have a x chain or λ chain.

"Antigen-binding fragment" refers to a Fab fragment, Fab' fragment, F(ab')2 fragment, single-chain Fv (i.e., sFv), nanobody (i.e., VHH), or VH/VL domain that has antigen-binding activity. The Fv fragment comprises the heavy chain variable region and the light chain variable region of the antibody but does not comprise the constant region, and has the smallest antigen-binding fragment of the entire antigen-binding sites. Generally, the Fv antibody also comprises a polypeptide linker between the VH and VL domains, and is capable of forming the structure required for antigen binding. Two antibody variable regions can also be linked into a single polypeptide chain using different linkers, which is referred to as a single-chain antibody or single-chain Fv (sFv).

"Fc" refers to and includes polypeptides that comprise the constant region of an antibody but do not comprise the first constant region immunoglobulin domain. That is, Fc refers to the last two constant region immunoglobulin domains of IgA, IgD, and IgG, and the last three constant region immunoglobulin domains of IgE and IgM, and the N-terminal flexible hinge regions of these domains. For IgA and IgM, Fc may include a J chain. For IgG, Fc includes immunoglobulin domains Cγ2 and Cγ3 and the hinge between Cγ1 and Cγ2. Although the boundaries of the Fc region may vary, the human IgG heavy chain Fc region is usually defined to comprise residues C226 or P230 at its carboxyl-terminus, wherein the numbering is according to the EU index described in Kabat et al. (1991, NIH Publication 91-3242, National Technical Information Service, Springfield, VA). "EU index described in Kabat" refers to the residue numbering of the human IgG1 EU antibody as described in Kabat et al., supra. Fc may refer to this region in isolation, or this region in an antibody, antibody fragment, or Fc fusion protein. An Fc variant protein may be an antibody, Fc fusion, or any protein or protein domain that comprises an Fc region. Particularly preferred are proteins comprising variant Fc regions, i.e., non-naturally occurring Fc variants. Note: Polymorphism has been observed at many Fc positions, including but not limited to Kabat 270, 272, 312, 315, 356, and 358, and thus the presence of slight differences between the sequence and sequences in the prior art is allowed.

"Nucleic acid molecule" or "nucleic acid" refers to DNA molecules and RNA molecules. A nucleic acid molecule may be single-stranded or double-stranded, and it is preferably a double-stranded DNA. A nucleic acid is "operably linked" when it is placed into a functional relationship with another nucleic acid sequence. For example, a promoter or enhancer is operably linked to a coding sequence if it affects the transcription of the coding sequence.

An "expression vector" or "expression construct" or "cassette" or "plasmid" or simply "vector" may include any genetic construct type, including AAV or rAAV vectors, containing a nucleic acid or polynucleotide encoding a gene product in which part or all of the nucleic acid encoding sequence is capable of being transcribed and is suitable for gene therapy. The transcript can be translated into a protein. In some cases, it may be partially translated or not translated. In certain aspects, expression includes both gene transcription and translation of mRNA into a gene product. In other aspects, expression only includes transcription of the nucleic acid encoding genes of interest. An expression vector may also comprise control elements operatively linked to the encoding region to facilitate the expression of the protein in target cells. The combination of control elements and one or more genes to which they are operatively linked for expression can sometimes be referred to as an "expression cassette", a large number of which are known and available in the art or can be readily constructed from available components in the art.

"Host cell", "host cell line", and "host cell culture" are used interchangeably and refer to cells into which exogenous nucleic acids are introduced, including progeny of such cells. Host cells include "transformants" and "transformed cells", which include initial transformed cells and progeny derived therefrom (regardless of the number of passages). Progeny may not be exactly the same as the parent cell in terms of nucleic acid content but may contain mutations. Mutant progeny that have function or biological activity that is the same as the function or biological activity screened or selected in primary transformed cells are included herein.

"Pharmaceutical composition" refers to a mixture containing one or more of the compounds or the physiologically/pharmaceutically acceptable salts or prodrugs thereof described in the present disclosure, and other chemical components, for example, physiologically/pharmaceutically acceptable carriers and excipients. The pharmaceutical composition is intended to promote administration to an organism and facilitate the absorption of the active ingredient so that it can exert its biological activity.

"Administered", "administering", or "administration", when applied to animals, humans, experimental subjects, cells, tissue, organs, or biological fluids, refers to contact of an exogenous drug, a therapeutic agent, a diagnostic agent, or a composition with the animals, humans, subjects, cells, tissue, organs, or biological fluids. "Administered", "administering", or "administration" can refer to, for example, therapeutic, pharmacokinetic, diagnostic, research, and experimental methods. The treatment of cells comprises contacting the reagent with the cells and contacting the reagent with fluid, where the fluid is in contact with the cells. "Administered", "administering", or "administration" also means treating, e.g., cells by reagents, diagnosis, binding compositions or by another cell *in vitro* and *ex vivo*. "Administered", "administering", or "administration", when applied to humans, veterinary or research subjects, refer to therapeutic treatment, preventive or prophylactic measures, and research and diagnostic applications.

"Treatment" means administering a therapeutic agent, such as a composition comprising any one of the VEGF-binding molecules of the present disclosure, either internally or externally to a subject who is diagnosed as having, suspected of having, or predisposed to one or more disease symptoms on which the therapeutic agent is known to have a therapeutic effect. Generally, the therapeutic agent is administered in an amount effective to alleviate one or more disease symptoms in the subject or population being treated, whether by inducing regression of such symptoms or inhibiting such symptoms from progressing to any clinically unmeasurable degree.

The amount of therapeutic agent effective to alleviate any particular disease symptom (also referred to as the "therapeutically effective amount") may vary depending on factors such as the disease state, age and weight of the subject, and the capacity of the drug to produce a desired therapeutic effect in the subject. Whether a disease symptom has been alleviated can be evaluated by any clinical testing methods commonly used by doctors or other health care professionals to evaluate the severity or progression of the symptom. Although embodiments of the present disclosure (e.g., treatment methods or products) may be ineffective in alleviating the target disease symptom that every patient has, they shall alleviate the symptoms of the disease of interest in a statistically significant number of subjects as determined by any statistical test method known in the art, such as the Student's t-test, chi-square test, U-test by Mann and Whitney, Kruskal-Wallis test (H-test), Jonckheere-Terpstra test and Wilcoxon test.

"Effective amount" comprises an amount sufficient to ameliorate or prevent a symptom or sign of a medical disorder. An effective amount also means an amount sufficient to allow or facilitate diagnosis. The effective amount for a particular subject or veterinary subject may vary depending on factors such as the disorder to be treated, the general health of the subject, the method and route and dosage of administration, and the severity of side effects. An effective amount may be the maximum dose or administration regimen to avoid significant side effects or toxic effects.

The three-letter and single-letter codes for amino acids used in the present disclosure are as described in J. biol. chem., 243, p3558 (1968).

"Comprise", "have", or "include" includes "contain", "mainly consist of", "essentially consist of', and "consist of"; "mainly consist of", "essentially consist of', and "consist of" belong to the subordinate concepts of "comprise", "have", or "include".

"Fusion protein" is an isolated protein unrelated to other proteins, polypeptides, or molecules, and it is a purified product of the culture of recombinant host cells or is used as a purified extract. Techniques for the construction of fusion proteins are based on molecular cloning. The specific experimental method can be found in experimental handbooks such as the second and third editions of Molecular Cloning. The DNA encoding the fusion protein described above can be obtained by conventional techniques, e.g., by whole gene synthesis or is formed by splicing of fragments of Flt1 and KDR. The vector used may be a plasmid vector commonly used in molecular biology. A protein secretion signal sequence is added before the amino-terminus of each fusion protein to ensure the secretion of proteins from cells. The vector sequence includes a promoter for driving gene expression, protein translation initiation and termination signals, and a polyadenylation (PolyA) sequence. The vector contains an antibiotic resistance gene to facilitate plasmid propagation in bacteria. Additionally, the vector also contains a eukaryotic cell selective gene for screening for stably transfected cell strains. After the construction of the plasmids for the various fusion proteins described above is complete, the plasmid DNA can be used to transfect cells to express the corresponding proteins. There are a variety of expression systems available for expressing these fusion proteins, including (but not limited to) mammalian cells, bacteria, yeast, insect cells, etc. Proteins expressed from mammalian cells have glycosylation modifications. Since the amino acid sequences of the fusion proteins of the present disclosure include glycosylatable amino acids, mammalian cells are the best cells for expressing these proteins. A variety of mammalian cells can be used for large-scale protein expression, such as 293 cells, CHO cells, SP20 cells, NSO cells, COS cells, BHK cells, and PerC6 cells. Many other cells can also be used for the expression and production of these proteins, and thus are included among the cells usable in the present disclosure. Plasmids encoding polypeptides can be transfected into cells. Transfection methods include (but are not limited to): electroporation, liposome-mediated transfection, etc. After the fusion proteins are expressed, the concentration of the fusion proteins in the cell culture can be measured using enzyme-linked immunosorbent assay (ELISA) or other methods. Since these fusion proteins have an immunoglobulin Fc fragment, the expressed fusion proteins can be extracted using protein A affinity chromatography. Since the primary function of the various fusion proteins of the present disclosure is to block the VEGF signaling pathway, these fusion proteins may be used in diseases associated with angiogenesis or VEGF. The fusion protein DNA sequences can also be inserted into appropriate vectors and expressed in patients using gene therapy or cell therapy. Therefore, the methods of using the fusion proteins contemplated by the present disclosure can take a variety of forms, including not only the proteins themselves but also the corresponding DNA sequences encoding the amino acid sequences of the fusion proteins.

"Payload" refers to one or more polynucleotides or polynucleotide regions encoded by or encoded within a viral genome, or expression products of such polynucleotides or polynucleotide regions, e.g., a transgene, a polynucleotide encoding a polypeptide, or a regulatory polynucleotide. In some embodiments of the present disclosure, the payload encodes or expresses a target gene, and the target gene is, for example, a transgene encoding a VEGF-binding molecule. "Payload construct" is one or more polynucleotide regions encoding or comprising a payload flanked on one or both sides by inverted terminal repeat (ITR) sequences, and the payload construct is a template that is replicated in a viral production cell to produce a viral genome. "Payload construct vector" is a vector that encodes or comprises a payload construct and regulatory regions for replication and expression in bacteria and cells. "Payload construct expression vector" is a vector that encodes or comprises a payload construct, and it also comprises one or more polynucleotide regions encoding or comprising components for viral expression in a viral replication cell.

"AAV vector" or "rAAV vector" refers to an adeno-associated viral (AAV) vector or a recombinant AAV (rAAV) vector comprising a polynucleotide sequence not of AAV origin (i.e., a polynucleotide heterologous to AAV, such as a nucleic acid sequence that encodes a therapeutic transgene (e.g., aflibercept)), typically a sequence of interest for the genetic transformation of a cell. In general, the heterologous polynucleotide is flanked by at least one, and generally by two AAV inverted terminal repeats (ITRs). The term rAAV vector includes both rAAV vector particles and rAAV vector plasmids. A rAAV vector may be single-stranded (ssAAV) or self-complementary (scAAV). "AAV virus" or "rAAV vector particle" refers to a viral particle consisting of at least one AAV capsid protein (typically of all of the capsid proteins of a wild-type AAV) and a polynucleotide rAAV vector. If the particle comprises a heterologous polynucleotide (i.e. a polynucleotide other than a wild-type AAV genome, such as a transgene to be delivered to a mammalian cell), it is typically referred to as an "rAAV vector particle" or simply an "rAAV vector". Thus, production of rAAV particles necessarily includes production of rAAV vectors, as such vectors are contained within rAAV particles.

"Operatively linked" can refer to the juxtaposition of genetic elements, wherein the elements are in a relationship permitting them to operate in an expected manner. For example, a promoter can be operatively linked to a coding region if the promoter helps initiate transcription of the coding sequence. Spacer residues may be present between the promoter and the coding region as long as their functional relationship is maintained.

In the present disclosure, "VEGFR2 D2D3" is used interchangeably with "R2D2-R2D3" and "R2D2 R2D3". "VEGFR1D2-VEGFR2D3" is used interchangeably with "R1D2-R2D3" and "R1D2 R2D3".

## Examples

The present disclosure is further described below with reference to examples; however, these examples are not intended to limit the scope of the present disclosure. The experimental methods in the examples of the present disclosure in which specific conditions are not specified are generally performed under conventional conditions such as Antibodies: A Laboratory Manual and Molecular Cloning: A Laboratory Manual by Cold Spring Harbor Laboratory, or under conditions recommended by the manufacturer of the raw material or the goods. Reagents without specific origins indicated are commercially available conventional reagents.

### Example 1: VEGF-Binding Molecule and Point Mutation Design

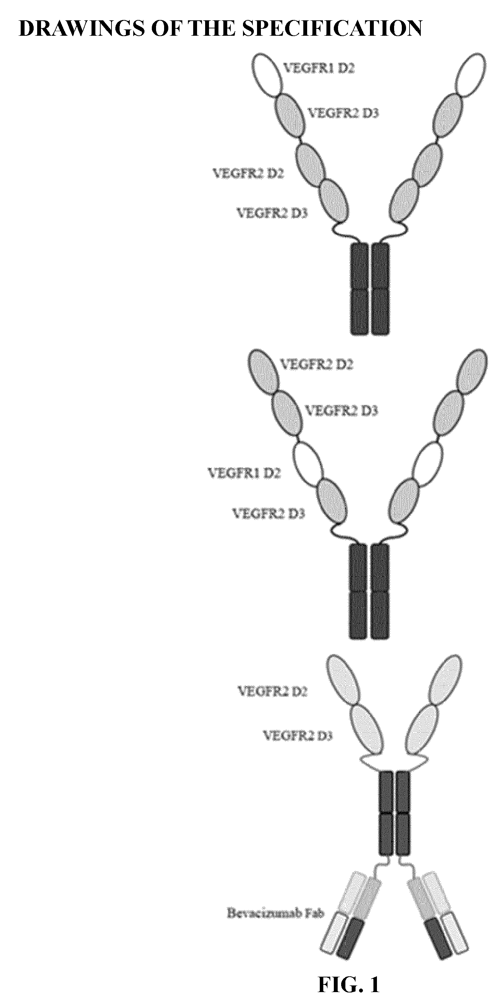

In this example, three molecular formats of VEGF-binding molecules were designed (FIG. 1), and they were, from N-terminus to C-terminus:

molecular format 1: VEGFR1 D2 + VEGFR2 D3 + VEGFR2 D2D3 + hinge region of human IgG1 and CH2CH3 constant region;

molecular format 2: VEGFR2 D2D3 + VEGFR1 D2 + VEGFR2 D3 + hinge region of human IgG1 and CH2CH3 constant region;

molecular format 3: heavy chain: VEGFR2 D2D3 + hinge region of human IgG1 and CH2CH3 constant region + bevacizumab VH + CH1; light chain: bevacizumab VL + CL. To improve the stability and ligand affinity of fusion proteins, a series of point mutations were designed in the VEGF trap backbone molecule (i.e., the VEGFR2 D2D3 domain, underlined above) in the VEGF-binding molecules:

1. Based on the VEGFR2 D2D3 sequence (SEQ ID NO: 69), 5 mutants were designed by mutating the cysteine at position 162 (C162) into A/V/S/I/L, respectively.
Wild-type VEGFR2 D2D3 sequence:

SPFIASVSDQHGVVYITENKNKTVVIPCLGSISNLNVSLCARYPEKRFVPDGNRIS
WDSKKGFTIPSYMISYAGMVFCEAKINDESYQSIMYIVVVVGYRIYDVVLSPSH
GIELSVGEKLVLNCTARTELNVGIDFNWEYPSSKHQHKKLVNRDLKTQSGSEMK
KFLSTLTIDGVTRSDQGLYTCAASSGLMTKKNSTFVRVHEK

SEQ ID NO: 69

2. Based on the VEGFR2 D2D3 sequence (SEQ ID NO: 69), 6 single point mutations and 2 combination mutations were designed (Table 1).

3. Based on the VEGFR2 D2D3 sequence (SEQ ID NO: 69), single point mutations were designed in the D2 or D3 domain (Table 2 and Table 3).

Table 1: VEGF trap backbone molecule VEGFR2 D3 mutations

| Residue No. | Wild type | Residue after mutation |
|---|---|---|
| 274 | N | F or I |
| 276 | D | Q |
| 280 | Q | E |
| 284 | E | H |
| 288 | F | Y |
| 313 | L | R |
| 221&222 | Y&R | S or N & K |
| 277&289 | L&L | R&Y |

Table 2: VEGF trap backbone molecule VEGFR2 D2 mutations

| Residue No. | Wild type | Residue after mutation |
|---|---|---|
| 135 | V | Y |
| 197 | M | Y |
| 199 | F | Q or T |
| 213 | M | N |
| 215 | I | F or H |
| 219 | V | I |

Table 3: VEGF trap backbone molecule VEGFR2 D3 mutations

| Residue No. | Wild type | Residue after mutation |
|---|---|---|
| 257 | D | Q or T |
| 275 | R | L or E |
| 277 | L | R |
| 278 | K | D or S or E |
| 283 | S | T |
| 289 | L | Y |
| 313 | L | Y |
| 314 | M | S or Q |

**Example 2: Plasmid Construction for VEGF-Binding Molecules and Protein Expression and Purification**

Using whole-gene synthesis, the VEGF-binding molecules designed in Example 1 were synthesized. The antagonistic activity against VEGF-C is mainly provided by VEGFR2 D2D3, and the antagonistic activity against VEGF-A is mainly provided by VEGFR1D2-VEGFR2D3 (aflibercept) or the Fab of bevacizumab.

After the amino acid sequences were optimized for the expression host, the genes encoding the nucleotide sequences described above were inserted into the pTT5 vector. The expression vectors were synthesized according to the sequence design described above and verified by sequencing.

Expression and purification of VEGF-binding molecules: The qualified plasmids were transfected into human 293F cells using PEI. The 293F cells were continuously cultured in serum-free culture medium (Shanghai OPM Biosciences, OPM-293 CD03) until they reached the logarithmic growth phase for cell transfection. 45 $\mu$ of plasmids was dissolved in 10 mL of Opti-MEM® I Reduced Serum Medium (GIBCO, 31985-070) and thoroughly mixed, and 200 $\mu$g of PEI was added and thoroughly mixed. The mixture was incubated at room temperature for 15 min and added to 50 mL of cells. The cell culture conditions were: 5% $CO_2$, 37 °C, 125 rpm/min. During the culture, a feeding was added at day 1 and day 3. When the cell viability was less than 70%, the cell supernatant was collected and filtered by centrifugation. The filtered cell culture supernatant was loaded onto a protein A column. The column was washed with phosphate buffer, and elution was performed with glycine hydrochloride buffer (pH 2.7, 0.1 M Gly-HCl). The eluate was neutralized with 1 M Tris-hydrochloride pH 9.0 and dialyzed against phosphate buffer to obtain finally the purified protein. SDS-PAGE and SEC-HPLC analyses confirmed the obtaining of the target fusion proteins.

The specific fusion protein sequences synthesized in this example are as follows:

> Aflibercept (PR1076)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKDKTHTCPPCPAPELLGG
PSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPR
EPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPG

SEQ ID NO: 70

> OPT-302 (HRAMD-C3)

YSMTPPTLNITEESHVIDTGDSLSISCRGQHPLEWAWPGAQEAPATGDKDSEDTG
VVRDCEGTDARPYCKVLLLHEVHAQDTGSYVCYYKYIKARIEGTTAASSYVFV
RDFEQPFINKPDTLLVNRKDAMWVPCLVSIPGLNVTLRSQSSVLWPDGQEVVW
DDRRGMLVSTPLLHDALYLQCETTWGDQDFLSNPFLVHITGNELYDIQLLPRKS
LELLVGEKLVLNCTVWAEFNSGVTFDWDYPGKQAERGKWVPERRSQQTHTEL
SSILTIHNVSQHDLGSYVCKANNGIQRFRESTEVIVHEEPKSCDKTHTCPPCPAPE
LLGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKA
KGQPREPQVYTLPPSRDELTKNQVSLTCLVKGFYPSDIAVEWESNGQPENNYKT

TPPVLDSDGSFFLYSKLTVDKSRWQQGNVFSCSVMHEALHNHYTQKSLSLSPGK

SEQ ID NO: 71

> Bevacizumab

Heavy chain:

EVQLVESGGGLVQPGGSLRLSCAASGYTFTNYGMNWVRQAPGKGLEWVGWIN
TYTGEPTYAADFKRRFTFSLDTSKSTAYLQMNSLRAEDTAVYYCAKYPHYYGSS
HWYFDVWGQGTLVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNT
KVDKKVEPKSCDKTHTCPPCPAPEAAGGPSVFLFPPKPKDTLMISRTPEVTCVV
VDVSHEDPEVKFNWYVDGVEVHNAKTKPREEQYNSTYRVVSVLTVLHQDWL
NGKEYKCKVSNKALPAPIEKTISKAKGQPREPQVYTLPPSREEMTKNQVSLTCL
VKGFYPSDIAVEWESNGQPENNYKTTPPVLDSDGSFFLYSKLTVDKSRWQQGN
VFSCSVMHEALHNHYTQKSLSLSPGK

SEQ ID NO: 72

Light chain:

DIQMTQSPSSLSASVGDRVTITCSASQDISNYLNWYQQKPGKAPKVLIYFTSSLH
SGVPSRFSGSGSGTDFTLTISSLQPEDFATYYCQQYSTVPWTFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

SEQ ID NO: 73

> PR1010 (RID2-R2D3-linker-R2D2-R2D3-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEK*GGGGS*SPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
NCTARTELNVGIDFNWEYPSSKHQHKKLVNRDLKTQSGSEMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 3

> PR1011 (R2D2-R2D3-linker-R1D2-R2D3-Fc)

SPFIASVSDQHGVVYITENKNKTVVIPCLGSISNLNVSLCARYPEKRFVPDGNRIS
WDSKKGFTIPSYMISYAGMVFCEAKINDESYQSIMYIVVVVGYRIYDVVLSPSH
GIELSVGEKLVLNCTARTELNVGIDFNWEYPSSKHQHKKLVNRDLKTQSGSEMK
KFLSTLTIDGVTRSDQGLYTCAASSGLMTKKNSTFVRVHEK*GGGGS*SDTGRPFV
EMYSEIPEIIHMTEGRELVIPCRVTSPNITVTLKKFPLDTLIPDGKRIIWDSRKGFII
SNATYKEIGLLTCEATVNGHLYKTNYLTHRQTNTIIDVVLSPSHGIELSVGEKLVL

NCTARTELNVGIDFNWEYPSSKHQHKKLVNRDLKTQSGSEMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 4

> PR1088 (R2D2-R2D3-Fc)

SPFIASVSDQHGVVYITENKNKTVVIPCLGSISNLNVSLCARYPEKRFVPDGNRIS
WDSKKGFTIPSYMISYAGMVFCEAKINDESYQSIMYIVVVVGYRIYDVVLSPSH
GIELSVGEKLVLNCTARTELNVGIDFNWEYPSSKHQHKKLVNRDLKTQSGSEMK
KFLSTLTIDGVTRSDQGLYTCAASSGLMTKKNSTFVRVHEKDKTHTCPPCPAPE
AAGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLSLSPGK

SEQ ID NO: 5

> PR1160 (R2D2 (C162V)-R2D3-Fc)

SPFIASVSDQHGVVYITENKNKTVVIPCLGSISNLNVSLVARYPEKRFVPDGNRIS
WDSKKGFTIPSYMISYAGMVFCEAKINDESYQSIMYIVVVVGYRIYDVVLSPSH
GIELSVGEKLVLNCTARTELNVGIDFNWEYPSSKHQHKKLVNRDLKTQSGSEMK
KFLSTLTIDGVTRSDQGLYTCAASSGLMTKKNSTFVRVHEKDKTHTCPPCPAPE
AAGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLSLSPGK

SEQ ID NO: 6

> PR1090 (R1D2-R2D3-linker-R2D2 (V135Y)-R2D3-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
YVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
NCTARTELNVGIDFNWEYPSSKHQHKKLVNRDLKTQSGSEMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 7

> PR1094 (R1D2-R2D3-linker-R2D2 (C162V)-R2D3-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLVARYPEKRFVPDGNRISWDSKKGFTIPSY
MISYAGMVFCEAKINDESYQSIMYIVVVVGYRIYDVVLSPSHGIELSVGEKLVLN
CTARTELNVGIDFNWEYPSSKHQHKKLVNRDLKTQSGSEMKKFLSTLTIDGVTR
SDQGLYTCAASSGLMTKKNSTFVRVHEKDKTHTCPPCPAPEAAGGPSVFLFPPK
PKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK

SEQ ID NO: 8

> PR1101 (R1D2-R2D3-linker-R2D2 (M197Y)-R2D3-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGYVFCEAKINDESYQSIMYIVVVVGYRIYDVVLSPSHGIELSVGEKLVL
NCTARTELNVGIDFNWEYPSSKHQHKKLVNRDLKTQSGSEMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

<div align="right">SEQ ID NO: 9</div>

> PR1103 (R1D2-R2D3-linker-R2D2 (F199Q)-R2D3-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVQCEAKINDESYQSIMYIVVVVGYRIYDVVLSPSHGIELSVGEKLV
LNCTARTELNVGIDFNWEYPSSKHQHKKLVNRDLKTQSGSEMKKFLSTLTIDGV
TRSDQGLYTCAASSGLMTKKNSTFVRVHEKDKTHTCPPCPAPEAAGGPSVFLFP
PKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVY
TLPPSREEMTKNQVSLTCLVKGFYPSDIAVEWESNGQPENNYKTTPPVLDSDGSF
FLYSKLTVDKSRWQQGNVFSCSVMHEALHNHYTQKSLSLSPGK

<div align="center">SEQ ID NO: 10</div>

> PR1106 (R1D2-R2D3-linker-R2D2 (M213N)-R2D3-Fc)

<div align="center">38</div>

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSINYIVVVVGYRIYDVVLSPSHGIELSVGEKLVL
NCTARTELNVGIDFNWEYPSSKHQHKKLVNRDLKTQSGSEMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 11

> PR1107 (R1D2-R2D3-linker-R2D2 (I215F)-R2D3-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYFVVVVGYRIYDVVLSPSHGIELSVGEKLV
LNCTARTELNVGIDFNWEYPSSKHQHKKLVNRDLKTQSGSEMKKFLSTLTIDGV
TRSDQGLYTCAASSGLMTKKNSTFVRVHEKDKTHTCPPCPAPEAAGGPSVFLFP
PKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVY
TLPPSREEMTKNQVSLTCLVKGFYPSDIAVEWESNGQPENNYKTTPPVLDSDGSF
FLYSKLTVDKSRWQQGNVFSCSVMHEALHNHYTQKSLSLSPGK

SEQ ID NO: 12

> PR1108 (R1D2-R2D3-linker-R2D2 (I215H)-R2D3-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYHVVVVGYRIYDVVLSPSHGIELSVGEKLV
LNCTARTELNVGIDFNWEYPSSKHQHKKLVNRDLKTQSGSEMKKFLSTLTIDGV
TRSDQGLYTCAASSGLMTKKNSTFVRVHEKDKTHTCPPCPAPEAAGGPSVFLFP
PKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVY
TLPPSREEMTKNQVSLTCLVKGFYPSDIAVEWESNGQPENNYKTTPPVLDSDGSF

FLYSKLTVDKSRWQQGNVFSCSVMHEALHNHYTQKSLSLSPGK

SEQ ID NO: 13

> PR1109 (R1D2-R2D3-linker-R2D2 (V219I)-R2D3-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVIGYRIYDVVLSPSHGIELSVGEKLVL
NCTARTELNVGIDFNWEYPSSKHQHKKLVNRDLKTQSGSEMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 14

> PR1110 (R1D2-R2D3-linker-R2D2 (Y221N)-R2D3-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGNRIYDVVLSPSHGIELSVGEKLVL
NCTARTELNVGIDFNWEYPSSKHQHKKLVNRDLKTQSGSEMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 15

> PR1111 (R1D2-R2D3-linker-R2D2-R2D3 (D257Q)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
NCTARTELNVGIQFNWEYPSSKHQHKKLVNRDLKTQSGSEMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL

PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 16

> PR1112 (R1D2-R2D3-linker-R2D2-R2D3 (D257T)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
NCTARTELNVGITFNWEYPSSKHQHKKLVNRDLKTQSGSEMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 17

> PR1113 (R1D2-R2D3-linker-R2D2-R2D3 (N274F)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
NCTARTELNVGIDFNWEYPSSKHQHKKLVFRDLKTQSGSEMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 18

> PR1114 (R1D2-R2D3-linker-R2D2-R2D3 (N274I)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
NCTARTELNVGIDFNWEYPSSKHQHKKLVIRDLKTQSGSEMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN

STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 19

> PR1115 (R1D2-R2D3-linker-R2D2-R2D3 (R275L)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
NCTARTELNVGIDFNWEYPSSKHQHKKLVNLDLKTQSGSEMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 20

> PR1116 (R1D2-R2D3-linker-R2D2-R2D3 (R275E)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
NCTARTELNVGIDFNWEYPSSKHQHKKLVNEDLKTQSGSEMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 21

> PR1118 R1D2-R2D3-linker-R2D2-R2D3 (D276Q)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
NCTARTELNVGIDFNWEYPSSKHQHKKLVNRQLKTQSGSEMKKFLSTLTIDGVT
RSDQGLYTCAASSGLMTKKNSTFVRVHEKDKTHTCPPCPAPEAAGGPSVFLFPP

KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 22

> PR1119 (R1D2-R2D3-linker-R2D2-R2D3 (L277R)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
NCTARTELNVGIDFNWEYPSSKHQHKKLVNRDRKTQSGSEMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 23

> PR1120 (R1D2-R2D3-linker-R2D2-R2D3 (K278D)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
NCTARTELNVGIDFNWEYPSSKHQHKKLVNRDLDTQSGSEMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 24

> PR1121 R1D2-R2D3-linker-R2D2-R2D3 (K278S)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
NCTARTELNVGIDFNWEYPSSKHQHKKLVNRDLSTQSGSEMKKFLSTLTIDGVT

RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 25

> PR1122 (R1D2-R2D3-linker-R2D2-R2D3 (K278E)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
NCTARTELNVGIDFNWEYPSSKHQHKKLVNRDLETQSGSEMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 26

> PR1123 (R1D2-R2D3-linker-R2D2-R2D3 (Q280E)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
NCTARTELNVGIDFNWEYPSSKHQHKKLVNRDLKTESGSEMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 27

> PR1124 (R1D2-R2D3-linker-R2D2-R2D3 (S283T)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL

NCTARTELNVGIDFNWEYPSSKHQHKKLVNRDLKTQSGTEMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 28

> PR1125 R1D2-R2D3-linker-R2D2-R2D3 (E284H)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
NCTARTELNVGIDFNWEYPSSKHQHKKLVNRDLKTQSGSHMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEO ID NO: 29

> PR1126 (R1D2-R2D3-linker-R2D2-R2D3 (F288Y)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
NCTARTELNVGIDFNWEYPSSKHQHKKLVNRDLKTQSGSEMKKYLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 30

> PR1127 (R1D2-R2D3-linker-R2D2-R2D3 (L289Y)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS

YMISYAGMVFCEAKINDESYQSIMYIVVVVGYRIYDVVLSPSHGIELSVGEKLVL
NCTARTELNVGIDFNWEYPSSKHQHKKLVNRDLKTQSGSEMKKFYSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 31

> PR1128 (R1D2-R2D3-linker-R2D2-R2D3 (L313Y)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
NCTARTELNVGIDFNWEYPSSKHQHKKLVNRDLKTQSGSEMKKFLSTLTIDGVT
RSDQGLYTCAASSGYMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 32

> PR1129 (R1D2-R2D3-linker-R2D2-R2D3 (M314S)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
NCTARTELNVGIDFNWEYPSSKHQHKKLVNRDLKTQSGSEMKKFLSTLTIDGVT
RSDQGLYTCAASSGLSTKKNSTFVRVHEKDKTHTCPPCPAPEAAGGPSVFLFPPK
PKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK

SEQ ID NO: 33

> PR1130 (R1D2-R2D3-linker-R2D2-R2D3 (M314Q)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG

VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
NCTARTELNVGIDFNWEYPSSKHQHKKLVNRDLKTQSGSEMKKFLSTLTIDGVT
RSDQGLYTCAASSGLQTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 34

> PR1131 (R1D2-R2D3-linker-R2D2 (Y221N)-R2D3 (M314Q)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGNRIYDVVLSPSHGIELSVGEKLVL
NCTARTELNVGIDFNWEYPSSKHQHKKLVNRDLKTQSGSEMKKFLSTLTIDGVT
RSDQGLYTCAASSGLQTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 35

> PR1132 (R1D2-R2D3-linker-R2D2-R2D3 (L277R/L289Y)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
NCTARTELNVGIDFNWEYPSSKHQHKKLVNRDRKTQSGSEMKKFYSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 36

> PR1135 (R1D2-R2D3-linker-R2D2 (Y221S/R222K)-R2D3-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS

TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGSKIYDVVLSPSHGIELSVGEKLVL
NCTARTELNVGIDFNWEYPSSKHQHKKLVNRDLKTQSGSEMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 37

> PR1136 (R1D2-R2D3-linker-R2D2-R2D3 (L313R)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
NCTARTELNVGIDFNWEYPSSKHQHKKLVNRDLKTQSGSEMKKFLSTLTIDGVT
RSDQGLYTCAASSGRMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 38

> PR1139 (R1D2-R2D3-linker-R2D2-R2D3 (N245Q)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLCARYPEKRFVPDGNRISWDSKKGFTIPS
YMISYAGMVFCEAKINDESYQSIMYIVVVVGYRIYDVVLSPSHGIELSVGEKLVL
QCTARTELNVGIDFNWEYPSSKHQHKKLVNRDLKTQSGSEMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 39

> PR1141 (R1D2-R2D3-linker-R2D2 (C162V)-R2D3 (N274F)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS

VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLVARYPEKRFVPDGNRISWDSKKGFTIPSY
MISYAGMVFCEAKINDESYQSIMYIVVVVGYRIYDVVLSPSHGIELSVGEKLVLN
CTARTELNVGIDFNWEYPSSKHQHKKLVFRDLKTQSGSEMKKFLSTLTIDGVTR
SDQGLYTCAASSGLMTKKNSTFVRVHEKDKTHTCPPCPAPEAAGGPSVFLFPPK
PKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK

SEQ ID NO: 40

> PR1142 (R1D2-R2D3-linker-R2D2 (C162V)-R2D3 (N274I)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLVARYPEKRFVPDGNRISWDSKKGFTIPSY
MISYAGMVFCEAKINDESYQSIMYIVVVVGYRIYDVVLSPSHGIELSVGEKLVLN
CTARTELNVGIDFNWEYPSSKHQHKKLVIRDLKTQSGSEMKKFLSTLTIDGVTRS
DQGLYTCAASSGLMTKKNSTFVRVHEKDKTHTCPPCPAPEAAGGPSVFLFPPKP
KDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK

SEQ ID NO: 41

> PR1143 (R1D2-R2D3-linker-R2D2 (C162V)-R2D3 (R275L)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLVARYPEKRFVPDGNRISWDSKKGFTIPSY
MISYAGMVFCEAKINDESYQSIMYIVVVVGYRIYDVVLSPSHGIELSVGEKLVLN
CTARTELNVGIDFNWEYPSSKHQHKKLVNLDLKTQSGSEMKKFLSTLTIDGVTR
SDQGLYTCAASSGLMTKKNSTFVRVHEKDKTHTCPPCPAPEAAGGPSVFLFPPK
PKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK

SEQ ID NO: 42

> PR1144 (R1D2-R2D3-linker-R2D2 (C162V)-R2D3 (D276Q)-Fc)

SDTGRPFVEMYSEIPEIIHMTEGRELVIPCRVTSPNITVTLKKFPLDTLIPDGKRIIW

DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLVARYPEKRFVPDGNRISWDSKKGFTIPSY
MISYAGMVFCEAKINDESYQSIMYIVVVVGYRIYDVVLSPSHGIELSVGEKLVLN
CTARTELNVGIDFNWEYPSSKHQHKKLVNRQLKTQSGSEMKKFLSTLTIDGVTR
SDQGLYTCAASSGLMTKKNSTFVRVHEKDKTHTCPPCPAPEAAGGPSVFLFPPK
PKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK

SEQ ID NO: 43

> PR1145 (R1D2-R2D3-linker-R2D2 (C162V)-R2D3 (S283T)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLVARYPEKRFVPDGNRISWDSKKGFTIPSY
MISYAGMVFCEAKINDESYQSIMYIVVVVGYRIYDVVLSPSHGIELSVGEKLVLN
CTARTELNVGIDFNWEYPSSKHQHKKLVNRDLKTQSGTEMKKFLSTLTIDGVTR
SDQGLYTCAASSGLMTKKNSTFVRVHEKDKTHTCPPCPAPEAAGGPSVFLFPPK
PKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK

SEQ ID NO: 44

> PR1146 (R1D2-R2D3-linker-R2D2 (C162V)-R2D3 (L289Y)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLVARYPEKRFVPDGNRISWDSKKGFTIPSY
MISYAGMVFCEAKINDESYQSIMYIVVVVGYRIYDVVLSPSHGIELSVGEKLVLN
CTARTELNVGIDFNWEYPSSKHQHKKLVNRDLKTQSGSEMKKFYSTLTIDGVTR
SDQGLYTCAASSGLMTKKNSTFVRVHEKDKTHTCPPCPAPEAAGGPSVFLFPPK
PKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK

SEQ ID NO: 45

> PR1147 (R1D2-R2D3-linker-R2D2 (C162V)-R2D3 (L313Y)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLVARYPEKRFVPDGNRISWDSKKGFTIPSY
MISYAGMVFCEAKINDESYQSIMYIVVVVGYRIYDVVLSPSHGIELSVGEKLVLN
CTARTELNVGIDFNWEYPSSKHQHKKLVNRDLKTQSGSEMKKFLSTLTIDGVTR
SDQGLYTCAASSGYMTKKNSTFVRVHEKDKTHTCPPCPAPEAAGGPSVFLFPPK
PKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK

SEQ ID NO: 46

> PR1148 (R1D2-R2D3-linker-R2D2 (C162V)-R2D3 (L277R/L289Y)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLVARYPEKRFVPDGNRISWDSKKGFTIPSY
MISYAGMVFCEAKINDESYQSIMYIVVVVGYRIYDVVLSPSHGIELSVGEKLVLN
CTARTELNVGIDFNWEYPSSKHQHKKLVNRDRKTQSGSEMKKFYSTLTIDGVTR
SDQGLYTCAASSGLMTKKNSTFVRVHEKDKTHTCPPCPAPEAAGGPSVFLFPPK
PKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK

SEQ ID NO: 47

> PR1149 (R1D2-R2D3-linker-R2D2 (C162V/Y221S/R222K)-R2D3-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLVARYPEKRFVPDGNRISWDSKKGFTIPSY
MISYAGMVFCEAKINDESYQSIMYIVVVVGSKIYDVVLSPSHGIELSVGEKLVLN
CTARTELNVGIDFNWEYPSSKHQHKKLVNRDLKTQSGSEMKKFLSTLTIDGVTR
SDQGLYTCAASSGLMTKKNSTFVRVHEKDKTHTCPPCPAPEAAGGPSVFLFPPK
PKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK

SEQ ID NO: 48

> PR1150 (R1D2-R2D3-linker-R2D2 (C162V)-R2D3 (L313R)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLVARYPEKRFVPDGNRISWDSKKGFTIPSY
MISYAGMVFCEAKINDESYQSIMYIVVVVGYRIYDVVLSPSHGIELSVGEKLVLN
CTARTELNVGIDFNWEYPSSKHQHKKLVNRDLKTQSGSEMKKFLSTLTIDGVTR
SDQGLYTCAASSGRMTKKNSTFVRVHEKDKTHTCPPCPAPEAAGGPSVFLFPPK
PKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK

SEQ ID NO: 49

> PR1163 (heavy chain H: R2D2 (C162V)-R2D3-Fc-linker-VH-CH1; light chain L: VL-CL)

Heavy chain:

SPFIASVSDQHGVVYITENKNKTVVIPCLGSISNLNVSLVARYPEKRFVPDGNRIS
WDSKKGFTIPSYMISYAGMVFCEAKINDESYQSIMYIVVVVGYRIYDVVLSPSH
GIELSVGEKLVLNCTARTELNVGIDFNWEYPSSKHQHKKLVNRDLKTQSGSEMK
KFLSTLTIDGVTRSDQGLYTCAASSGLMTKKNSTFVRVHEKDKTHTCPPCPAPE
AAGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLSLSPGG
GGGSGGGGSGGGGSGGGGSEVQLVESGGGLVQPGGSLRLSCAASGYTFTNYG
MNWVRQAPGKGLEWVGWINTYTGEPTYAADFKRRFTFSLDTSKSTAYLQMNS
LRAEDTAVYYCAKYPHYYGSSHWYFDVWGQGTLVTVSSASTKGPSVFPLAPSS
KSTSGGTAALGCLVKDYFPEPVTVSWNSGALTSGVHTFPAVLQSSGLYSLSSVVT
VPSSSLGTQTYICNVNHKPSNTKVDKKVEPKSC

SEQ ID NO: 50

Light chain:

DIQMTQSPSSLSASVGDRVTITCSASQDISNYLNWYQQKPGKAPKVLIYFTSSLH
SGVPSRFSGSGSGTDFTLTISSLQPEDFATYYCQQYSTVPWTFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

SEQ ID NO: 51

> PR1165 (R1D2-R2D3-linker-R2D2 (C162I)-R2D3-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS

TLTIDGVTRSDQGLYTCAASSGLMTKKNSTFVRVHEKGGGGSSPFIASVSDQHG
VVYITENKNKTVVIPCLGSISNLNVSLIARYPEKRFVPDGNRISWDSKKGFTIPSY
MISYAGMVFCEAKINDESYQSIMYIVVVVGYRIYDVVLSPSHGIELSVGEKLVLN
CTARTELNVGIDFNWEYPSSKHQHKKLVNRDLKTQSGSEMKKFLSTLTIDGVTR
SDQGLYTCAASSGLMTKKNSTFVRVHEKDKTHTCPPCPAPEAAGGPSVFLFPPK
PKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK

SEQ ID NO: 52

> PR1166 (R1D2-R2D3-linker-R2D2 (C162L)-R2D3-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLLARYPEKRFVPDGNRISWDSKKGFTIPS
YMISYAGMVFCEAKINDESYQSIMYIVVVVGYRIYDVVLSPSHGIELSVGEKLVL
NCTARTELNVGIDFNWEYPSSKHQHKKLVNRDLKTQSGSEMKKFLSTLTIDGVT
RSDQGLYTCAASSGLMTKKNSTFVRVHEKDKTHTCPPCPAPEAAGGPSVFLFPP
KPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTL
PPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK

SEQ ID NO: 53

> PR1167 (R1D2-R2D3-R2D2 (C162V)-R2D3 (N274F/S283T)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKSPFIASVSDQHGVVYITE
NKNKTVVIPCLGSISNLNVSLVARYPEKRFVPDGNRISWDSKKGFTIPSYMISYA
GMVFCEAKINDESYQSIMYIVVVVGYRIYDVVLSPSHGIELSVGEKLVLNCTART
ELNVGIDFNWEYPSSKHQHKKLVFRDLKTQSGTEMKKFLSTLTIDGVTRSDQGL
YTCAASSGLMTKKNSTFVRVHEKDKTHTCPPCPAPEAAGGPSVFLFPPKPKDTL
MISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLT
VDKSRWQQGNVFSCSVMHEALHNHYTQKSLSLSPGK

SEQ ID NO: 54

> PR1168 (R1D2-R2D3-R2D2 (C162V/Y221S/R222K)-R2D3 (N274F)-Fc)


SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS

VGEKLVLNCTARTELNVGIDFNWEYPSSKHQHKKLVNRDLKTQSGSEMKKFLS
TLTIDGVTRSDQGLYTCAASSGLMTKKNSTFVRVHEKSPFIASVSDQHGVVYITE
NKNKTVVIPCLGSISNLNVSLVARYPEKRFVPDGNRISWDSKKGFTIPSYMISYA
GMVFCEAKINDESYQSIMYIVVVVGSKIYDVVLSPSHGIELSVGEKLVLNCTART
ELNVGIDFNWEYPSSKHQHKKLVFRDLKTQSGSEMKKFLSTLTIDGVTRSDQGL
YTCAASSGLMTKKNSTFVRVHEKDKTHTCPPCPAPEAAGGPSVFLFPPKPKDTL
MISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLT
VDKSRWQQGNVFSCSVMHEALHNHYTQKSLSLSPGK

SEQ ID NO: 55

> PR1169 (R1D2-R2D3-R2D2 (C162V/Y221S/R222K)-R2D3 (S283T)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKSPFIASVSDQHGVVYITE
NKNKTVVIPCLGSISNLNVSLVARYPEKRFVPDGNRISWDSKKGFTIPSYMISYA
GMVFCEAKINDESYQSIMYIVVVVGSKIYDVVLSPSHGIELSVGEKLVLNCTART
ELNVGIDFNWEYPSSKHQHKKLVNRDLKTQSGTEMKKFLSTLTIDGVTRSDQGL
YTCAASSGLMTKKNSTFVRVHEKDKTHTCPPCPAPEAAGGPSVFLFPPKPKDTL
MISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLT
VDKSRWQQGNVFSCSVMHEALHNHYTQKSLSLSPGK

SEQ ID NO: 56

> PR1170 (R1D2-R2D3-R2D2 (C162V/Y221S/R222K)-R2D3 (N274F/S283T)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKSPFIASVSDQHGVVYITE
NKNKTVVIPCLGSISNLNVSLVARYPEKRFVPDGNRISWDSKKGFTIPSYMISYA
GMVFCEAKINDESYQSIMYIVVVVGSKIYDVVLSPSHGIELSVGEKLVLNCTART
ELNVGIDFNWEYPSSKHQHKKLVFRDLKTQSGTEMKKFLSTLTIDGVTRSDQGL
YTCAASSGLMTKKNSTFVRVHEKDKTHTCPPCPAPEAAGGPSVFLFPPKPKDTL
MISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLT
VDKSRWQQGNVFSCSVMHEALHNHYTQKSLSLSPGK

SEQ ID NO: 57

> PR1171 (R1D2-R2D3-linker-R2D2 (C162V)-R2D3 (N274F/S283T)-Fc)

SDTGRPFVEMYSEIPEIIHMTEGRELVIPCRVTSPNITVTLKKFPLDTLIPDGKRIIW

DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLVARYPEKRFVPDGNRISWDSKKGFTIPSY
MISYAGMVFCEAKINDESYQSIMYIVVVVGYRIYDVVLSPSHGIELSVGEKLVLN
CTARTELNVGIDFNWEYPSSKHQHKKLVFRDLKTQSGTEMKKFLSTLTIDGVTR
SDQGLYTCAASSGLMTKKNSTFVRVHEKDKTHTCPPCPAPEAAGGPSVFLFPPK
PKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK

SEQ ID NO: 58

> PR1172 (R1D2-R2D3-linker-R2D2 (C162V/Y221S/R222K)-R2D3 (N274F)-Fc)


SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLVARYPEKRFVPDGNRISWDSKKGFTIPSY
MISYAGMVFCEAKINDESYQSIMYIVVVVGSKIYDVVLSPSHGIELSVGEKLVLN
CTARTELNVGIDFNWEYPSSKHQHKKLVFRDLKTQSGSEMKKFLSTLTIDGVTR
SDQGLYTCAASSGLMTKKNSTFVRVHEKDKTHTCPPCPAPEAAGGPSVFLFPPK
PKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK

SEQ ID NO: 59

> PR1173 (R1D2-R2D3-linker-R2D2 (C162V/Y221S/R222K)-R2D3 (S283T)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLVARYPEKRFVPDGNRISWDSKKGFTIPSY
MISYAGMVFCEAKINDESYQSIMYIVVVVGSKIYDVVLSPSHGIELSVGEKLVLN
CTARTELNVGIDFNWEYPSSKHQHKKLVNRDLKTQSGTEMKKFLSTLTIDGVTR
SDQGLYTCAASSGLMTKKNSTFVRVHEKDKTHTCPPCPAPEAAGGPSVFLFPPK
PKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK

SEQ ID NO: 60

> PR1174 (R1D2-R2D3-linker-R2D2 (C162V/Y221S/R222K)-R2D3 (N274F/S283T)-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKGGGGSSPFIASVSDQHG
VVYITENKNKTVVIPCLGSISNLNVSLVARYPEKRFVPDGNRISWDSKKGFTIPSY
MISYAGMVFCEAKINDESYQSIMYIVVVVGSKIYDVVLSPSHGIELSVGEKLVLN
CTARTELNVGIDFNWEYPSSKHQHKKLVFRDLKTQSGTEMKKFLSTLTIDGVTR
SDQGLYTCAASSGLMTKKNSTFVRVHEKDKTHTCPPCPAPEAAGGPSVFLFPPK
PKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYS
KLTVDKSRWQQGNVFSCSVMHEALHNHYTQKSLSLSPGK

SEQ ID NO: 61

> PR1175 (R2D2 (C162V)-R2D3 (N274F/S283T)-Fc)

SPFIASVSDQHGVVYITENKNKTVVIPCLGSISNLNVSLVARYPEKRFVPDGNRIS
WDSKKGFTIPSYMISYAGMVFCEAKINDESYQSIMYIVVVVGYRIYDVVLSPSH
GIELSVGEKLVLNCTARTELNVGIDFNWEYPSSKHQHKKLVFRDLKTQSGTEMK
KFLSTLTIDGVTRSDQGLYTCAASSGLMTKKNSTFVRVHEKDKTHTCPPCPAPE
AAGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLSLSPGK

SEQ ID NO: 62

> PR1176 (R2D2 (C162V/Y221S/R222K)-R2D3 (N274F)-Fc)

SPFIASVSDQHGVVYITENKNKTVVIPCLGSISNLNVSLVARYPEKRFVPDGNRIS
WDSKKGFTIPSYMISYAGMVFCEAKINDESYQSIMYIVVVVGSKIYDVVLSPSH
GIELSVGEKLVLNCTARTELNVGIDFNWEYPSSKHQHKKLVFRDLKTQSGSEMK
KFLSTLTIDGVTRSDQGLYTCAASSGLMTKKNSTFVRVHEKDKTHTCPPCPAPE
AAGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLSLSPGK

SEQ ID NO: 63

> PR1177 (R2D2 (C162V/Y221S/R222K)-R2D3 (S283T)-Fc)

SPFIASVSDQHGVVYITENKNKTVVIPCLGSISNLNVSLVARYPEKRFVPDGNRIS
WDSKKGFTIPSYMISYAGMVFCEAKINDESYQSIMYIVVVVGSKIYDVVLSPSH
GIELSVGEKLVLNCTARTELNVGIDFNWEYPSSKHQHKKLVNRDLKTQSGTEM
KKFLSTLTIDGVTRSDQGLYTCAASSGLMTKKNSTFVRVHEKDKTHTCPPCPAP
EAAGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISK

AKGQPREPQVYTLPPSREEMTKNQVSLTCLVKGFYPSDIAVEWESNGQPENNYK
TTPPVLDSDGSFFLYSKLTVDKSRWQQGNVFSCSVMHEALHNHYTQKSLSLSPG
K

SEQ ID NO: 64

> PR1178 (R2D2 (C162V/Y221S/R222K)-R2D3 (N274F/S283T)-Fc)

SPFIASVSDQHGVVYITENKNKTVVIPCLGSISNLNVSLVARYPEKRFVPDGNRIS
WDSKKGFTIPSYMISYAGMVFCEAKINDESYQSIMYIVVVVGSKIYDVVLSPSH
GIELSVGEKLVLNCTARTELNVGIDFNWEYPSSKHQHKKLVFRDLKTQSGTEMK
KFLSTLTIDGVTRSDQGLYTCAASSGLMTKKNSTFVRVHEKDKTHTCPPCPAPE
AAGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLSLSPGK

SEQ ID NO: 65

> PR1179 (R1D2-R2D3-R2D2 (C162V/Y221S/R222K)-R2D3-Fc)

SDTGRPFVEMYSEIPEIIHMTEGRELVIPCRVTSPNITVTLKKFPLDTLIPDGKRIIW
DSRKGFIISNATYKEIGLLTCEATVNGHLYKTNYLTHRQTNTIIDVVLSPSHGIELS
VGEKLVLNCTARTELNVGIDFNWEYPSSKHQHKKLVNRDLKTQSGSEMKKFLS
TLTIDGVTRSDQGLYTCAASSGLMTKKNSTFVRVHEKSPFIASVSDQHGVVYITE
NKNKTVVIPCLGSISNLNVSLVARYPEKRFVPDGNRISWDSKKGFTIPSYMISYA
GMVFCEAKINDESYQSIMYIVVVVGSKIYDVVLSPSHGIELSVGEKLVLNCTART
ELNVGIDFNWEYPSSKHQHKKLVNRDLKTQSGSEMKKFLSTLTIDGVTRSDQGL
YTCAASSGLMTKKNSTFVRVHEKDKTHTCPPCPAPEAAGGPSVFLFPPKPKDTL
MISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLT
VDKSRWQQGNVFSCSVMHEALHNHYTQKSLSLSPGK

SEQ ID NO: 66

> PR1186 (heavy chain H: R1D2-R2D3-R2D2 (C162V/Y221S/R222K)-R2D3 (N274F/S283T)-Fc-linker-VH-CH1; light chain L: VL-CL)

Heavy chain:

SPFIASVSDQHGVVYITENKNKTVVIPCLGSISNLNVSLVARYPEKRFVPDGNRIS
WDSKKGFTIPSYMISYAGMVFCEAKINDESYQSIMYIVVVVGSKIYDVVLSPSH
GIELSVGEKLVLNCTARTELNVGIDFNWEYPSSKHQHKKLVFRDLKTQSGTEMK
KFLSTLTIDGVTRSDQGLYTCAASSGLMTKKNSTFVRVHEKDKTHTCPPCPAPE
AAGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLSLSPGG
GGGSGGGGSGGGGSGGGGSEVQLVESGGGLVQPGGSLRLSCAASGYTFTNYG
MNWVRQAPGKGLEWVGWINTYTGEPTYAADFKRRFTFSLDTSKSTAYLQMNS

LRAEDTAVYYCAKYPHYYGSSHWYFDVWGQGTLVTVSSASTKGPSVFPLAPSS
KSTSGGTAALGCLVKDYFPEPVTVSWNSGALTSGVHTFPAVLQSSGLYSLSSVVT
VPSSSLGTQTYICNVNHKPSNTKVDKKVEPKSC

SEQ ID NO: 67

Light chain:

DIQMTQSPSSLSASVGDRVTITCSASQDISNYLNWYQQKPGKAPKVLIYFTSSLH
SGVPSRFSGSGSGTDFTLTISSLQPEDFATYYCQQYSTVPWTFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

SEQ ID NO: 68.

**Example 3: Assay for Binding Activity of VEGF-Binding Molecules to Ligands VEGF-A and VEGF-C**

The inhibition of KDR signaling activation by the VEGF-binding molecules was evaluated by measuring the VEGF-A-induced NFAT-RE Luc2P KDR cell reporter gene expression level. The experimental steps were as follows:

3A.1 Preparation of culture media: Complete culture medium: high-glucose DMEM (90%) + FBS (10%); complete culture medium: high-glucose DMEM (90%) + FBS (10%), 100 $\mu$g/mL hygromycin B, 250 $\mu$g/mL G-418. Analysis culture medium: high-glucose DMEM (99%) + FBS (1%).

3A.2 Dilution of VEGF-binding molecule samples: The test samples were serially diluted 3-fold, with the highest concentration being 300 nM. VEGFA dilution: the final concentration of VEGFA was 250 ng/mL.

3A.3 Preparation of reporter gene cell strain: Glo-ResponseTM NFAT-RE-luc2P/KDR HEK293 cells were digested, washed with 5 mL of PBS, and resuspended in the analysis culture medium, and the density was adjusted to $8 \times 10^5$ cells/mL. The cell suspension was added to a 96-well cell culture plate at 50 $\mu$L/well, i.e., $4 \times 10^4$ cells/well.

3A.4 Treatment with VEGF-binding molecules: The prepared different concentrations of VEGF-binding molecules were added to the cell wells at 50 $\mu$L/well, and the plate was incubated in a 37 °C, 5% $CO_2$ incubator for 6 h. Each concentration was tested in triplicate. Meanwhile, a VEGF-A-only control group and a VEGF-A-free control group were set.

3A.5 Color development: Bright-Glo color-developing solution was equilibrated to room temperature. After the incubation, 50 $\mu$L of Bright-Glo color-developing solution was added to the 96-well plate, thoroughly mixed by shaking at room temperature for 10 min, and left to stand for 2 min. The culture plate was placed on a multi-mode microplate reader, and chemiluminescence values were measured.

3A.6 Data processing:

$$\text{Top value} = \text{fluorescence value of VEGF-A} + 0 \text{ nM VEGF-binding molecule group}$$

$$\text{Bottom value} = \text{fluorescence value of VEGF-A-free group}$$

$$\text{Inhibition rate} = (\text{fluorescence value - bottom value})/(\text{top value - bottom value}) \times 100\%$$

Graphpad plotting: The results were analyzed using a four-parameter fit to obtain dose-response curves. The four-parameter fit formula was:

$$Y = (A - D)/(1 + (X/C)^{\wedge}B) + D$$

A: the estimated value of the lower asymptote; B: the slope of the curve; C: $IC_{50}$; D: the estimated value of the upper asymptote.

For the evaluation of the binding activity of the VEGF-binding molecules to VEGF-C, a BAF3-VEGFR2/R3 cell proliferation experiment was used in this example. BAF3-VEGFR2/R3 is a BA-F3 cell line overexpressing human VEGFR2/R3, and the growth of the cells depends on the exogenous addition of the growth factor VEGF-C. The basic experimental steps were as follows:

3B.1 Cell culture: The cell line described above was cultured in RPMI1640 with 10% FBS (supplemented with 10 ng/mL mouse IL3, 1 $\mu$g/mL puromycin, and 250 $\mu$g/mL G418) at $1 \times 10^5$ to $1.5 \times 10^5$ cells/mL. The culture was plated in a 37 °C, 5% $CO_2$ incubator, with the cell density not exceeding $1 \times 10^6$ cells/mL.

3B.2 Cell proliferation experiment: Cells in the logarithmic growth phase were washed three times with PBS and centrifuged at 800 r/min for 3 min. After the cell density was adjusted with RPMI1640 (FBS: 2%, 143 ng/mL VEFG-C), the cells were added to a 96-well plate at $2 \times 10^4$ cells/well/50 $\mu$L. Then, 50 $\mu$L of serially diluted VEGF-binding molecule (highest concentration 300 nM, 3-fold dilution) was added. Meanwhile, a VEGF-C-free control group and a VEGF-A-only control group were set. After 3 days of culture, 30 $\mu$L of cell titer-glo was added and thoroughly mixed, and cell counting was performed.

3B.3 Data processing:

Top value = fluorescence value of VEGF-C + VEGF-binding molecule concentration 0 group

Bottom value = fluorescence value of VEGF-C-free group

Inhibition rate = (fluorescence value - bottom value)/(top value - bottom value) × 100%

Graphpad plotting: The results were analyzed using a four-parameter fit to obtain dose-response curves. The four-parameter fit formula was:

$$Y = (A - D)/(1 + (X/C)^{\wedge}B) + D$$

where A: the estimated value of the lower asymptote; B: the slope of the curve; C: $IC_{50}$; D: the estimated value of the upper asymptote.

In this example, the binding activity of VEGF-A and VEGF-C to the VEGF-binding molecules was evaluated through the two *in vitro* experiments described above, respectively. The specific data are shown in Table 4:

Table 4: Data on the activity of molecules based on molecular format 1 with single point mutations

| Structure | Protein name | VEGF-A $IC_{50}$(nM) | VEGF-C $IC_{50}$(nM) |
|---|---|---|---|
| R1D2-R2D3-Fc(SEQ ID NO: 70) | Aflibercept | 0.76 | - |
| SEQ ID NO: 71 | OPT-302 | - | 0.97 |
| R1D2-R2D3 -linker-R2D2-R2D3 -Fc | PR1010 | 2.71 | 9.45 |
| R1D2-R2D3-linker-R2D2(C162V)-R2D3-Fc | PR1094 | 0.34 | 2.57 |
| R1D2-R2D3-linker-R2D2(C162I)-R2D3-Fc | PR1165 | 0.92 | 2.89 |
| R1D2-R2D3-linker-R2D2(C162L)-R2D3-Fc | PR1166 | 1.12 | 2.69 |
| R1D2-R2D3-linker-R2D2(M197Y)-R2D3-Fc | PR1101 | 0.54 | 2.00 |
| R1D2-R2D3-linker-R2D2(V219I)-R2D3-Fc | PR1109 | 0.18 | 2.70 |
| R1D2-R2D3-linker-R2D2-R2D3(N274F)-Fc | PR1113 | 0.49 | 1.12 |
| R1D2-R2D3-linker-R2D2-R2D3(N274I)-Fc | PR1114 | 0.36 | 1.06 |
| R1D2-R2D3-linker-R2D2-R2D3(R275L)-Fc | PR1115 | 0.28 | 0.90 |
| R1D2-R2D3-linker-R2D2-R2D3(R275E)-Fc | PR1116 | 0.54 | 1.23 |
| R1D2-R2D3-linker-R2D2-R2D3(D276Q)-Fc | PR1118 | 0.22 | 0.92 |
| R1D2-R2D3-linker-R2D2-R2D3(L277R)-Fc | PR1119 | 0.14 | 1.84 |
| R1D2-R2D3-linker-R2D2-R2D3(L278D)-Fc | PR1120 | 0.38 | 2.20 |
| R1D2-R2D3-linker-R2D2-R2D3(K278E)-Fc | PR1122 | 0.36 | 2.26 |
| R1D2-R2D3-linker-R2D2-R2D3(Q280E)-Fc | PR1123 | 0.21 | 1.53 |
| R1D2-R2D3-linker-R2D2-R2D3(S283T)-Fc | PR1124 | 0.29 | 1.04 |
| R1D2-R2D3-linker-R2D2-R2D3(E284H)-Fc | PR1125 | 0.23 | 1.62 |
| R1D2-R2D3-linker-R2D2-R2D3(F288Y)-Fc | PR1126 | 0.70 | 2.36 |
| R1D2-R2D3-linker-R2D2-R2D3(L289Y)-Fc | PR1127 | 0.52 | 1.16 |
| R1D2-R2D3-linker-R2D2-R2D3(L313Y)-Fc | PR1128 | 0.42 | 1.06 |
| R1D2-R2D3-linker-R2D2-R2D3(M314S)-Fc | PR1129 | 0.67 | 2.62 |
| R1D2-R2D3-linker-R2D2-R2D3(M314Q)-Fc | PR1130 | 0.45 | 1.99 |
| R1D2-R2D3-linker-R2D2-R2D3(L277R/L289Y)-Fc | PR1132 | 0.42 | 1.06 |
| R1D2-R2D3-linker-R2D2(Y221S/R222K)-R2D3-Fc | PR1135 | 0.50 | 1.90 |
| R1D2-R2D3-linker-R2D2-R2D3(L313R)-Fc | PR1136 | <u>0.14</u> | <u>0.98</u> |

The *in vitro* activity of the VEGF-binding molecules of the present disclosure to VEGF-A and VEGF-C was comparable to that of the positive controls aflibercept and OPT-302.

Table 5: Data on the activity of proteins produced by the CHO-K1 system, containing combinations of some of the single point mutations and C162V

| Protein name | Structure | VEGF-A IC$_{50}$ (nM) | VEGF-C IC$_{50}$ (nM) |
|---|---|---|---|
| PR1160 | R2D2(C162V)-R2D3-Fc | 28.36 | 0.80 |
| PR1141 | R1D2-R2D3-linker-R2D2(C162V)-R2D3(N274F)-Fc | 1.14 | 1.78 |
| PR1142 | R1D2-R2D3-linker-R2D2(C162V)-R2D3(N274I)-Fc | 0.99 | 1.92 |
| PR1143 | R1D2-R2D3-linker-R2D2(C162V)-R2D3(R275L)-Fc | 0.84 | 1.63 |
| PR1144 | R1D2-R2D3-linker-R2D2(C162V)-R2D3(D276Q)-Fc | 0.51 | 0.97 |
| PR1145 | R1D2-R2D3-linker-R2D2(C162V)-R2D3(S283T)-Fc | 0.92 | 1.05 |
| PR1146 | R1D2-R2D3-linker-R2D2(C162V)-R2D3(L289Y)-Fc | 0.98 | 2.01 |
| PR1147 | R1D2-R2D3-linker-R2D2(C162V)-R2D3(L313Y)-Fc | 1.16 | 1.26 |
| PR1148 | R1D2-R2D3-linker-R2D2(C162V)-R2D3(L277R/L289Y)-Fc | 1.49 | 2.53 |
| PR1149 | R1D2-R2D3-linker-R2D2(C162V/Y221S/R222K)-R2D3-Fc | 0.82 | 1.66 |
| PR1150 | R1D2-R2D3-linker-R2D2(C162V)-R2D3(L313R)-Fc | 0.81 | 1.07 |
| Aflibercept | SEQ ID NO: 70 | 0.50 | - |
| OPT-302 | SEQ ID NO: 71 | - | 0.42 |
| "-" indicates that the activity was unmeasurable. | | | |

Table 6: Data on the activity of proteins produced by the CHO-K1 system, containing combination mutations

| Protein name | Structure | VEGF-A IC$_{50}$ (nM) | VEGF-C IC$_{50}$ (nM) |
|---|---|---|---|
| PR1167 | R1D2-R2D3-R2D2(C162V)-R2D3(N274F/S283T)-Fc | 1.10 | 2.91 |
| PR1168 | R1D2-R2D3-R2D2(C162V/Y221S/R222K)-R2D3(N274F)-Fc | 1.15 | 5.43 |
| PR1169 | R1D2-R2D3-R2D2(C162V/Y221S/R222K)-R2D3(S283T)-Fc | 0.90 | 3.49 |
| PR1170 | R1D2-R2D3-R2D2(C162V/Y221S/R222K)-R2D3(N274F/S283T)-Fc | 0.89 | 5.95 |
| PR1171 | R1D2-R2D3-linker-R2D2(C162V)-R2D3(N274F/S283T)-Fc | 1.09 | 3.37 |
| PR1172 | R1D2-R2D3-linker-R2D2(C162V/Y221S/R222K)-R2D3(N274F)-Fc | 0.92 | 7.57 |
| PR1173 | R1D2-R2D3-linker-R2D2(C162V/Y221S/R222K)-R2D3(S283T)-Fc | 1.08 | 4.30 |
| PR1174 | R1D2-R2D3-linker-R2D2(C162V/Y221S/R222K)-R2D3(N274F/S283T)-Fc | 1.02 | 5.56 |
| PR1179 | R1D2-R2D3-R2D2(C162V/Y221S/R222K)-R2D3-Fc | 0.97 | 3.63 |
| Aflibercept | SEQ ID NO: 70 | 0.72 | - |
| OPT-302 | SEQ ID NO: 71 | - | 1.03 |

As shown in Table 5 and Table 6, although PR1160, a VEGFR2 D2D3 (C162V)-Fc fusion protein, exhibited some *in vitro* inhibitory activity against VEGF-A, its activity was weaker than the positive control aflibercept, while the inhibitory activity of the other fusion proteins against VEGF-A and VEGF-C was comparable to that of the two positive control molecules. As shown in Table 7, the inhibitory activity of the VEGF-binding molecules PR1145, PR1174, PR1163, and PR1186 against VEGF-A and VEGF-C was comparable to that of the two positive control molecules and stronger than the inhibitory activity of PR1010 and bevacizumab against VEGF-A.

Table 7: A summary of data on the activity of some fusion proteins

| Protein name | Structure | VEGF-A IC$_{50}$ (nM) | VEGF-C IC$_{50}$ (nM) |
|---|---|---|---|
| PR1010 | R1D2-R2D3-linker-R2D2-R2D3 -Fc | 0.45 | 6.63 |
| PR1145 | R1D2-R2D3-linker-R2D2(C162V)-R2D3(S283T)-Fc | 0.11 | 0.75 |
| PR1174 | R1D2-R2D3-linker-R2D2(C162V/Y221S/R222K)-R2D3(N274F/S283T)-Fc | 0.12 | 2.59 |
| PR1163 | Heavy chain H: R2D2 (C162V)-R2D3-Fc-linker-VH-CH1; Light chain L: VL-CL | 0.072 | 0.75 |
| PR1186 | Heavy chain H: R1D2-R2D3-R2D2 (C162V/Y221S/R222K)-R2D3 (N274F/S283T)-Fc-linker-VH-CH1; Light chain L: VL-CL | 0.092 | 1.55 |
| Aflibercept | SEQ ID NO: 70 | 0.099 | - |
| OPT-302 | SEQ ID NO: 71 | - | 0.66 |
| Bevazicumab | Heavy chain H: SEQ ID NO: 72; Light chain L: SEQ ID NO: 73 | 0.34 | - |

## Example 4: Analysis of Thermal Stability of Different VEGF-Binding Molecules

The thermal stability of fusion proteins is an important criterion for evaluating the quality of molecules. Developing a fusion protein molecule with relatively good thermal stability has positive significance for process production, drug transportation, etc. In this example, accelerated thermal stability evaluation was performed on candidate molecules.

The specific experimental steps were as follows: The candidate molecules were diluted to 1 mg/mL and aliquoted into three 1.5 mL EP tubes, with 100 μL in each tube. One of the tubes was taken as the time-0 sample and stored frozen. The remaining samples were placed in a constant-temperature, constant-humidity incubator at 40 °C with 75% humidity. One tube of sample was taken at two weeks, and one tube of sample was taken at two four weeks. The samples were stored frozen. After samples at three time points (0, 2, and 4 weeks) were taken, they were thawed at 4 °C and centrifuged at 12,000 rpm for 10 min to remove precipitates, and the supernatant was taken for HPLC analysis. In this example, the HPLC used was the ACQUITY Arc model produced by Waters, the column was XBridge BEH 200Å SEC 3.5 μm, the flow rate was 0.5 mL/min, and 30 μL of sample was injected. The final HPLC chromatograms of the samples are shown in the table below. Although the parent molecule PR1010 of molecular format 1 had an initial purity of up to 90%, the purity dropped below 30% after storage at 40°C for two weeks, suggesting that the stability of this molecule needs to be improved. The purity of the PR1094 molecule obtained by introducing the VEGFR2 C162V mutation into PR1010 increased to over 95% in HEK 293F, and the SEC purity was still as high as about 90% after storage at 40 °C for four weeks.

According to the HPLC chromatogram results, the thermal stability of the molecules was significantly improved after the C162V mutation, both on the VEGFR2 D2D3-Fc fusion protein (PR1160 vs. PR1088) and on molecular format 1 (PR1094 vs. RP1010). Additionally, point mutations such as N274F (PR1113), N274I (PR1114), D276Q (PR1118), Q280E (PR1123), E284H (PR1125), Y221S/R222K (PR1135), and L313R (PR1136) can also significantly improve the stability of fusion proteins by stabilizing the VEGFR2 D2D3 domain.

To further study the effects of C162V/I-based combination mutations on protein expression and purification, CHO-K1 was subsequently used to express combination-mutation molecules: in most cases, combination mutations can further improve the stability of molecules (e.g., PR1145/PR1149 vs. PR1094).

The results are shown in Table 8-1 and Table 8-2, where PR1088 to PR1136 (Table 8-1) were obtained from HEK 293F expression, and PR1094 to RP1179 (Table 8-2) were obtained from CHO-K1 expression.

Table 8-1: The results of the analysis of the thermal stability of different VEGF-binding molecules

| Protein name | Structure | SEC(%)/ 0 weeks | SEC(%)/ 2 weeks | SEC(%)/ 4 weeks |
|---|---|---|---|---|
| PR1088 | R2D2-R2D3-Fc | 70.38 | - | - |
| PR1010 | R1D2-R2D3-linker-R2D2-R2D3-Fc | 90.02 | <30 | - |
| PR1011 | R2D2-R2D3-linker-R1D2-R2D3-Fc | 81.92 | <30 | - |

(continued)

| Protein name | Structure | SEC(%)/ 0 weeks | SEC(%)/ 2 weeks | SEC(%)/ 4 weeks |
|---|---|---|---|---|
| PR1160 | R2D2(C162V)-R2D3-Fc | 93.24 | 82.20 | 86.16 |
| PR1094 | R1D2-R2D3-linker-R2D2(C162V)-R2D3-Fc | 95.23 | 91.75 | 89.45 |
| PR1101 | R1D2-R2D3-linker-R2D2(MI97Y)-R2D3-Fc | 82.77 | 20.33 | - |
| PR1109 | R1D2-R2D3-linker-R2D2(V219I)-R2D3-Fc | 83.34 | 22.73 | - |
| PR1113 | R 1D2-R2D3-linker-R2D2-R2D3 (N274F)-Fc | 83.18 | 51.64 | 16.36 |
| PR1114 | R1D2-R2D3-linker-R2D2-R2D3 (N2741)-Fc | 85.40 | 56.41 | 26.69 |
| PR1115 | R 1D2-R2D3-linker-R2D2-R2D3 (R275L)-Fc | 80.85 | 27.12 | - |
| PR1116 | R1D2-R2D3-linker-R2D2-R2D3 (R275E)-Fc | 83.99 | 19.72 | - |
| PR1118 | R1D2-R2D3-linker-R2D2-R2D3 (D276Q)-Fc | 83.93 | 54.95 | 49.24 |
| PR1119 | R1D2-R2D3-linker-R2D2-R2D3 (L277R)-Fc | 78.78 | 35.87 | - |
| PR1120 | R1D2-R2D3-linker-R2D2-R2D3 (K278D)-Fc | 86.00 | 27.33 | - |
| PR1122 | R1D2-R2D3-linker-R2D2-R2D3 (K278E)-Fc | 87.19 | 29.04 | - |
| PR1123 | R1D2-R2D3-linker-R2D2-R2D3 (Q280E)-Fc | 87.73 | 58.97 | 52.72 |
| PR1124 | R1D2-R2D3-linker-R2D2-R2D3 (S283T)-Fc | 88.46 | 23.73 | - |
| PR1125 | R1D2-R2D3-linker-R2D2-R2D3 (E284H)-Fc | 87.25 | 53.93 | 47.11 |
| PR1126 | R1D2-R2D3-linker-R2D2-R2D3 (F288Y)-Fc | 87.12 | 55.38 | - |
| PR1127 | R1D2-R2D3-linker-R2D2-R2D3 (L289Y)-Fc | 86.60 | 18.59 | - |
| PR1128 | R1D2-R2D3-linker-R2D2-R2D3(L313Y)-Fc | 88.84 | 31.48 | - |
| PR1129 | R1D2-R2D3-linker-R2D2-R2D3(M314S)-Fc | 81.61 | 8.80 | - |
| PR1130 | R1D2-R2D3-linker-R2D2-R2D3(M314Q)-Fc | 84.85 | 25.17 | - |
| PR1132 | R1D2-R2D3-linker-R2D2-R2D3 (L277R/L289Y)-Fc | 83.03 | 47.18 | - |
| PR1135 | R1D2-R2D3-linker-R2D2(Y221S/R222K)-R2D3-Fc | 91.76 | 72.49 | 65.05 |
| PR1136 | R1D2-R2D3-linker-R2D2-R2D3(L313R)-Fc | 92.13 | 67.75 | 60.39 |

Table 8-2: The results of the analysis of the thermal stability of different VEGF-binding molecules

| Protein name | Structure | SEC(%)/ 0 weeks | SEC(%)/ 2 weeks | SEC(%)/ 4 weeks |
|---|---|---|---|---|
| PR1094 | R1D2-R2D3-linker-R2D2(C162V)-R2D3-Fc | 85.56 | 81.43 | 77.17 |
| PR1141 | R1D2-R2D3-linker-R2D2(C162V)-R2D3(N274F)-Fc | 88.95 | 84.63 | 76.10 |
| PR1142 | R1D2-R2D3-linker-R2D2(C162V)-R2D3(R275L)-Fc | 87.09 | 81.39 | 77.13 |
| PR1143 | R1D2-R2D3-linker-R2D2(C162V)-R2D3(R275L)-Fc | 87.42 | 81.73 | 76.92 |
| PR1144 | R1D2-R2D3-linker-R2D2(C162V)-R2D3(D276Q)-Fc | 87.00 | 79.21 | 74.21 |
| PR1145 | R1D2-R2D3-linker-R2D2(C162V)-R2D3(S283T)-Fc | 90.10 | 83.81 | 78.61 |
| PR1146 | R1D2-R2D3-linker-R2D2(C162V)-R2D3(L289Y)-Fc | 87.04 | 72.54 | 65.91 |

(continued)

| Protein name | Structure | SEC(%)/ 0 weeks | SEC(%)/ 2 weeks | SEC(%)/ 4 weeks |
|---|---|---|---|---|
| PR1147 | R1D2-R2D3-linker-R2D2(C162V)-R2D3(L313Y)-Fc | 88.25 | 81.73 | 75.98 |
| PR1148 | R1D2-R2D3-linker-R2D2(C162V)-R2D3(L277R/L289Y)-Fc | 81.40 | 56.77 | 50.52 |
| PR1149 | R1D2-R2D3-linker-R2D2(C162V/Y221S/R222K)-R2D3-Fc | 90.21 | 83.81 | 80.36 |
| PR1150 | R1D2-R2D3-linker-R2D2(C162V)-R2D3(L313R)-Fc | 87.08 | 71.75 | 66.13 |
| PR1163 | Heavy chain H: R2D2 (C162V)-R2D3-Fc-linker-VH-CH1; Light chain L: VL-CL | 98.92 | 92.31 | 83.80 |
| PR1167 | R1D2-R2D3-R2D2(C162V)-R2D3(N274F/S283T)-Fc | 84.38 | 72.05 | 67.58 |
| PR1168 | R1D2-R2D3-R2D2(C162V/Y221S/R222K)-R2D3(N274-F)-Fc | 95.19 | 89.21 | 84.45 |
| PR1169 | R1D2-R2D3-R2D2(C162V/Y221S/R222K)-R2D3(S283-T)-Fc | 88.96 | 80.78 | 75.48 |
| PR1170 | R1D2-R2D3-R2D2(C162V/Y221S/R222K)-R2D3(N274-F/S283T)-Fc | 89.10 | 79.57 | 77.22 |
| PR1171 | R1D2-R2D3-linker-R2D2(C162V)-R2D3(N274F/S283T)-Fc | 82.88 | 70.43 | 67.74 |
| PR1172 | R1D2-R2D3-linker-R2D2(C162V/Y221S/R222K)-R2D3(N274-F)-Fc | 90.34 | 81.77 | 76.05 |
| PR1173 | R1D2-R2D3-linker-R2D2(C162V/Y221S/R222K)-R2D3(S283-T)-Fc | 91.35 | 81.96 | 77.37 |
| PR1174 | R1D2-R2D3-linker-R2D2(C162V/Y221S/R222K)-R2D3(N274-F/S283T)-Fc | 96.86 | 91.58 | 88.10 |
| PR1179 | R1D2-R2D3-R2D2(C162V/Y221S/R222K)-R2D3-Fc | 90.08 | 80.65 | 76.93 |

**Example 5: SPR Assay for Affinity Between VEGF-Binding Molecules and Different Ligands**

Surface plasmon resonance (SPR) was used to measure the affinity between different VEGF-binding molecules and VEGF-A/B/C proteins (A Protein A chip was used, and the VEGF-binding molecules were captured onto the chip. The coupling level was set to 100 RU, and measurement was performed using a Biacore instrument (T200, GE Healthcare, BIAC-B20-03). The running buffer was HBS-EP + (10 mM HEPES, 150 mM NaCl, 3 mM EDTA, 0.05% surfactant P20). Diluted antibodies (6.25, 12.5, 25, 50, 100 nM) were flowed through the experimental and reference channels (100 nM VEGF-A/B/C) at a flow rate of 30 $\mu$L/min for 3 min, followed by 5 min of dissociation. Then the regeneration buffer, 10 mM glycine pH 1.5 (GE Healthcare, BR-1003-54), was then run at a flow rate of 30 $\mu$L/min for 30 s. Data were analyzed using Biacore 8K evaluation software.

The affinity constants of the VEGF-binding molecules for the human VEGF-A, VEGF-B, and VEGF-C proteins were calculated using the MuLti-Cycle method. The kinetic parameters ka, kd, and KD values obtained from the calculations are shown in the table below.

Table 9: The affinity of VEGF-binding molecules for different ligands

| Protein name | Analyte | ka (1/Ms) | kd (1/s) | $K_D$ (M) |
|---|---|---|---|---|
| PR1145 | Human VEGF-A protein | 2.19E+06 | 9.87E-05 | 4.50E-11 |
| PR1174 | | 2.68E+06 | 1.15E-04 | 4.29E-11 |
| PR1163 | | 2.45E+06 | 1.26E-04 | 5.14E-11 |
| PR1186 | | 1.33E+06 | 4.05E-05 | 3.04E-11 |
| Aflibercept | | 1.03E+07 | 8.46E-05 | 8.18E-12 |
| PR1145 | Human VEGF-B protein | 2.05E+07 | 2.71E-04 | 1.32E-11 |
| PR1174 | | 5.01E+05 | 1.61E-04 | 3.21E-10 |
| Aflibercept | | 1.51E+06 | 1.14E-04 | 7.54E-11 |
| PR1145 | Human VEGF-C protein | 2.81E+06 | 7.98E-05 | 2.84E-11 |
| PR1174 | | 2.21E+06 | 5.17E-05 | 2.34E-11 |
| PR1163 | | 6.56E+06 | 8.79E-05 | 1.34E-11 |
| PR1186 | | 3.36E+06 | 4.13E-05 | 1.23E-11 |
| OPT-302 | | 3.21E+06 | 4.85E-05 | 1.51E-11 |

**Example 6: Evaluation *of In Vivo* Stability of VEGF-Binding Molecules**

To determine the *in vivo* stability of VEGF-binding molecules, changes in the concentration of these molecules in eye tissues were measured after intravitreal injection of these molecules. The specific experiment was as follows: Fourteen New Zealand rabbits, with an equal number of males and females, weighing 1.5-2.5 kg, were purchased. The experimental animals were divided into four groups: a PR1145 high-dose group, a PR1145 low-dose group, a PR1163 high-dose group, and a blank control group. All procedures were conducted on both eyes. Vitreous and aqueous humor samples were collected from 12 animals in the administration groups 3 h, 3 d, 7 d, 14 d, and 28 d after bilateral intravitreal administration. After the samples were taken, the content of the target protein in the samples was measured using an enzyme-linked immunosorbent assay. The experiment was completed by Chengdu Bio-HT Company Limited. The dosages are shown in Table 10.

Table 10: The administration regimen for the evaluation of the *in vivo* stability of VEGF-binding molecules

| Protein name | Number of animals | Protein dosage | Injection volume |
|---|---|---|---|
| PR1145 (high) | 4 | 400 μg/eye | 50 μL |
| PR1145 (low) | 4 | 200 μg/eye | 50 μL |
| PR1163 (high) | 4 | 542 μg/eye | 50 μL |

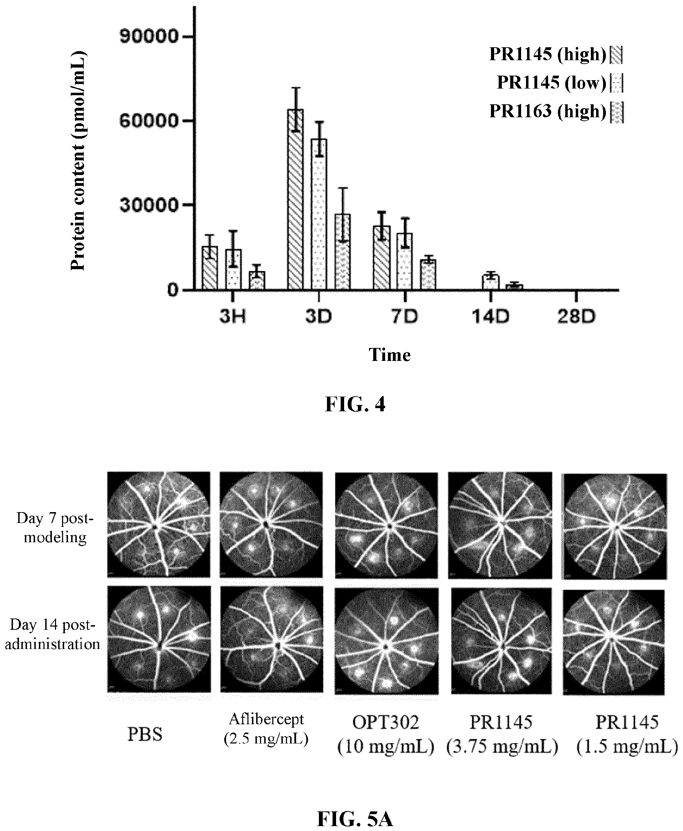

FIG. 4 shows the VEGF-binding molecule content in rabbits measured 3 h, 3 d, 7 d, 14 d, and 28 d after bilateral intravitreal administration. The results show that in the PR1145 high-dose group, the PR1145 low-dose group, and the PR1163 high-dose group, the drug concentration in the animals peaked 3 days after injection administration. PR1145 and PR1163 were detectable in the animals for 14 consecutive days, indicating that the VEGF-binding molecules have relatively good *in vivo* stability.

**Example 7: Evaluation *of In Vivo* Efficacy of VEGF-Binding Molecules**

To evaluate the inhibitory effects of VEGF-binding molecules on fundus choroidal neovascularization, a laser-induced fundus choroidal neovascularization (CNV) model was used for efficacy validation. This model is currently the classic model commonly used internationally for validating anti-choroidal neovascularization drugs. The specific experiment was as follows:
Forty-two rats, with an equal number of males and females, 8-10 weeks old, were purchased, and the experimental animals were divided into seven groups: a PR1145 high-dose group, a PR1145 low-dose group, a control group 1 (aflibercept), a control group 2 (OPT302), and a negative control group (PBS). The administration regimen is shown in Table 11. Each group consisted of 6 animals, and both eyes underwent laser CNV modeling. One week later, 4 μL of protein molecule was injected into both eyes. Two weeks post-administration, fundus angiography was performed to measure efficacy. PR1145 (3.75 mg/mL) and aflibercept (2.5 mg/mL) were administered at equimolar doses, and the molar ratio of aflibercept (2.5 mg/mL) to OPT302 (10 mg/mL) was 1:4.

Table 11: The administration regimen for the evaluation of the *in vivo* efficacy of VEGF-binding molecules

| Protein name | Group | Number of animals | Protein concentration | Injection volume |
|---|---|---|---|---|
| PR1145 (high) | Experimental group | 6 | 3.75 mg/mL | 4 μL |
| PR1145 (low) | Experimental group | 6 | 1.5 mg/mL | 4 μL |
| Aflibercept | Control group 1 | 6 | 2.5 mg/mL | 4 μL |
| OPT302 | Control group 2 | 6 | 10 mg/mL | 4 μL |
| PBS | Negative control | 6 | - | 4 μL |

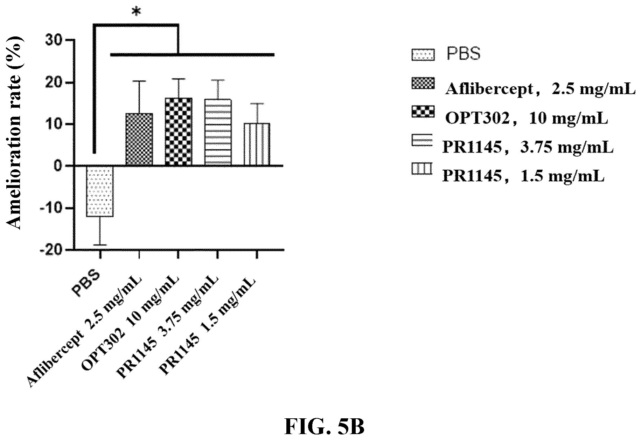

Fundus photography was performed on the rats before modeling, and no abnormalities were observed in the fundi of the rats. As shown in FIG. 5A, significant laser spot fluorescein sodium leakage was observed in the PBS group 7 days post-modeling, indicating successful model establishment. All the rats were dosed 7 days after CNV modeling, and fluorescein fundus angiography (FFA) analysis was performed 14 days post-administration. In the PBS group, increased fluorescent spot fluorescein sodium leakage was observed, suggesting that within 21 days post-modeling, the degree of laser-induced choroidal neovascularization continued to progress and had a tendency to worsen. The results of FFA analysis of the inhibition of choroidal neovascularization by the VEGF-binding molecules 14 days post-administration are shown in FIG. 5B. Compared to the PBS group, the PR1145 high-dose group, the PR1145 low-dose group, aflibercept, and OPT302 all significantly ameliorated laser-induced choroidal neovascularization fluorescein sodium leakage. The amelioration rate of PR1145 (3.75 mg/mL) was 15.8%, higher than the amelioration rate of aflibercept (2.5 mg/mL) (12.5%). PR1145 (3.75 mg/mL) and OPT302 (10 mg/mL) exhibited comparable ameliorating effects, but the dosage of PR1145 was significantly lower.

**Example 8: Structure of AAV Delivered Gene Expression Cassette**

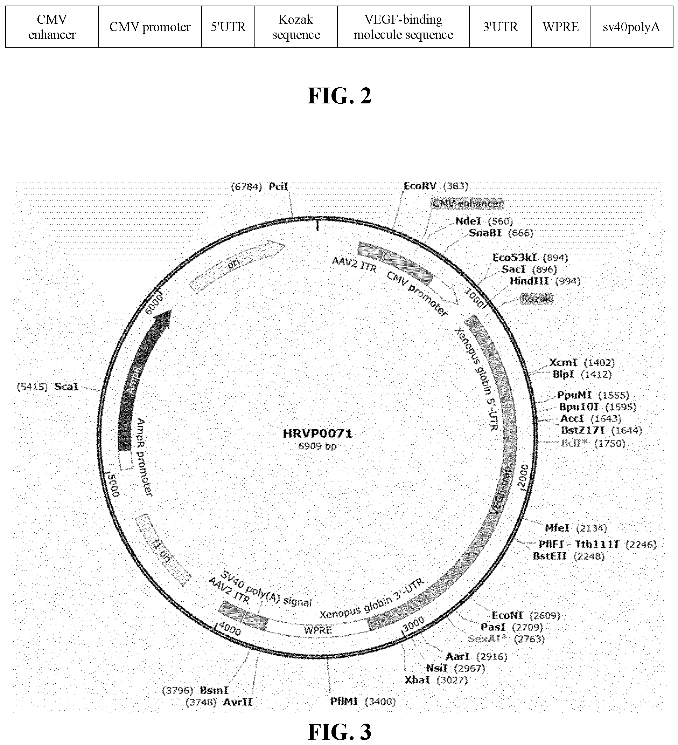

The expression vector provided in this example comprises a nucleic acid molecule that expresses a VEGF-binding molecule. Its structure is shown in FIG. 2. From the 5' end to the 3' end, it comprises: a CMV enhancer, a promoter, a 5' UTR, a Kozak sequence (gccacc), a VEGFR-binding molecule coding sequence, a 3' UTR, a WPRE, and an SV40polyA. The nucleic acid sequence of the CMV enhancer is set forth in SEQ ID NO: 74, the nucleic acid sequence of the CMV promoter is set forth in SEQ ID NO: 75, the 5' UTR is set forth in SEQ ID NO: 76, the Kozak sequence (gccacc)) and the VEGF-binding molecule coding sequence are set forth in any one of SEQ ID NOs: 78-86 (SEQ ID NOs: 78-80 correspond to codon-optimized sequences 1, 2, and 3 of PR1145; SEQ ID NOs: 81-83 correspond to codon-optimized sequences 1, 2, and 3 for PR1174; SEQ ID NOs: 84-86 correspond to codon-optimized sequences 1, 2, and 3 for PR1163), the 3' UTR sequence is set forth in SEQ ID NO: 87, the WPRE sequence is set forth in SEQ ID NO: 88, and the SV40polyA sequence is set forth in SEQ ID NO: 89.

The CMV enhancer sequence:

CGTTACATAACTTACGGTAAATGGCCCGCCTGGCTGACCGCCCAACGACCCC
CGCCCATTGACGTCAATAATGACGTATGTTCCCATAGTAACGCCAATAGGGAC
TTTCCATTGACGTCAATGGGTGGAGTATTTACGGTAAACTGCCCACTTGGCAG
TACATCAAGTGTATCATATGCCAAGTACGCCCCCTATTGACGTCAATGACGGT
AAATGGCCCGCCTGGCATTATGCCCAGTACATGACCTTATGGGACTTTCCTAC
TTGGCAGTACATCTACGTATTAGTCATCGCTATTACCATG

SEQ ID NO: 74

The CMV promoter sequence:

TGCTGATGCGGTTTTGGCAGTACACCAATGGGCGTGGATAGCGGTTTGACTC
ACGGGGATTTCCAAGTCTCCACCCCATTGACGTCAATGGGAGTTTGTTTTGG
CACCAAAATCAACGGGACTTTCCAAAATGTCGTAATAACCCCGCCCCGTTGA

CGCAAATGGGCGGTAGGCGTGTACGGTGGGAGGTCTATATAAGCAGAGCTCG
TTTAGTGAACCGTCAGATCGCCTGGAGAGGCCATCCACGCTGTTTTGACCTC
CATAGTGGACACCGGGACCGATCCAGCTCCGCGTCTCAGGGGAAGCTT

SEQ ID NO: 75

The 5' UTR sequence CTTGTTCTTTTTGCAGAAGCTCAGAATAAACGCTCAACTTTGG SEQ ID NO: 76

The Kozak sequence: gccacc;

codon-optimized sequence 1 of PR1145

ATGCCTCTGCTGCTGCTGTTGCCTTTGTTGTGGGCTGGCGCCCTGGCTTCTGA
TACCGGCAGACCTTTCGTGGAAATGTACAGCGAGATCCCCGAGATCATCCAC
ATGACCGAGGGCAGAGAGCTGGTCATCCCCTGCAGAGTGACCTCTCCTAACA
TCACCGTGACTCTGAAGAAGTTCCCTCTGGACACACTGATCCCCGACGGCAA
GAGAATCATCTGGGACTCCCGGAAGGGCTTCATCATCTCCAACGCCACCTAC
AAAGAGATCGGCCTGCTGACCTGCGAGGCCACCGTTAATGGCCACCTGTACA
AGACCAACTATCTGACCCACAGACAGACCAACACCATCATCGACGTGGTGCT
GAGCCCCTCTCATGGCATCGAGCTGTCCGTGGGAGAAAAGCTGGTGCTGAA
CTGCACCGCCAGAACCGAGCTGAACGTGGGCATCGACTTCAACTGGGAGTA
CCCCTCCAGCAAGCACCAGCACAAGAAGCTGGTCAACCGGGACCTGAAAAC
CCAGTCCGGCTCCGAGATGAAGAAATTCCTGAGCACCCTGACCATCGACGGC
GTGACCAGATCTGACCAGGGCCTGTATACCTGCGCCGCTTCTTCTGGCCTGAT
GACCAAGAAAAACTCCACCTTCGTGCGGGTGCACGAGAAAGGTGGCGGAG
GCAGCTCTCCTTTTATCGCCTCTGTGTCTGATCAGCACGGCGTGGTGTACATC
ACCGAGAACAAGAACAAGACCGTCGTGATCCCCTGCCTGGGCTCCATCTCTA
ACCTGAACGTGTCCCTGGTGGCTAGATACCCCGAGAAGAGATTCGTGCCTGA
CGGCAACCGGATCTCCTGGGACAGCAAGAAGGGCTTTACAATCCCCTCCTAC
ATGATCTCCTACGCCGGCATGGTGTTCTGCGAGGCTAAGATCAACGACGAGT
CCTACCAGTCCATCATGTACATCGTGGTGGTCGTGGGCTACAGAATCTACGAT
GTGGTGCTGTCCCCTAGCCACGGAATCGAACTGAGCGTGGGCGAGAAACTG
GTCCTCAACTGTACCGCTCGGACAGAACTGAATGTGGGGATTGATTTCAATT
GGGAATACCCTAGCTCCAAACATCAGCATAAGAAACTCGTGAACCGCGATCT
CAAGACCCAGAGCGGAACAGAAATGAAGAAGTTTCTGTCCACACTCACCAT
TGACGGGGTCACCAGAAGCGATCAGGGACTCTATACCTGTGCTGCCTCCAGC
GGACTGATGACAAAAAAGAACAGCACATTTGTCCGCGTCCACGAGAAGGAC
AAAACTCACACATGCCCACCGTGCCCGGCGCCTGAAGCCGCTGGGGGACCG
TCAGTCTTCCTCTTCCCCCCAAAACCCAAGGACACCCTCATGATCTCCCGGA
CCCCTGAGGTCACATGCGTGGTGGTGGACGTGAGCCACGAAGACCCTGAGG
TCAAGTTCAACTGGTACGTGGACGGCGTGGAGGTGCATAATGCCAAGACAA
AGCCGCGGGAGGAGCAGTACAACAGCACGTACCGGGTGGTCAGCGTCCTCA
CCGTCCTGCACCAGGACTGGCTGAATGGCAAGGAGTACAAGTGCAAGGTCT
CCAACAAAGCCCTCCCAGCCCCCATCGAGAAAACCATCTCCAAAGCCAAAG

GGCAGCCCAGGGAACCACAGGTGTACACCCTGCCCCCATCCCGGGAGGAGA
TGACCAAGAACCAGGTCAGCCTGACCTGCCTGGTCAAAGGCTTCTATCCCAG
CGACATCGCCGTGGAGTGGGAGAGCAATGGGCAGCCGGAGAACAACTACAA
GACCACGCCTCCCGTGCTGGACTCCGACGGCTCCTTCTTCCTCTACAGCAAG
CTCACCGTGGACAAGAGCAGGTGGCAGCAGGGGAACGTCTTCTCATGCTCC
GTGATGCATGAGGCTCTGCACAACCACTACACGCAGAAGAGCCTCTCCCTGT
CTCCGGGTAAATAA

SEQ ID NO: 78

codon-optimized sequence 2 of PR1145

ATGCCTCTGCTGCTGCTGCTGCCTCTGCTGTGGGCCGGCGCCCTGGCCTCCG
ACACCGGCCGGCCTTTTGTTGAAATGTACTCCGAGATCCCCGAAATCATCCAC
ATGACAGAAGGCAGAGAGCTGGTGATCCCTTGTAGAGTGACCAGCCCAAAC
ATCACCGTGACTCTGAAGAAGTTCCCCCTGGATACACTGATCCCTGATGGAA
AGAGAATCATTTGGGATAGCAGAAGGGCTTTATCATCAGCAACGCCACATA
CAAAGAGATCGGCCTGCTCACCTGCGAGGCCACCGTGAACGGCCACCTGTAT
AAGACAAACTACCTGACACATAGACAGACCAACACCATCATTGACGTGGTGC
TGTCCCCAAGCCACGGAATCGAGCTGTCTGTGGGCGAGAAGCTGGTGCTGA
ACTGCACCGCCAGAACAGAGCTGAACGTGGGCATTGACTTCAATTGGGAGT
ACCCTAGCTCCAAACACCAACACAAGAAGCTGGTGAATAGAGATCTGAAAA
CCCAGTCTGGCAGCGAAATGAAGAAATTTCTGAGCACACTGACCATTGACGG
CGTGACCCGCAGCGACCAGGGCCTGTACACCTGTGCCGCCTCTTCTGGCCTT
ATGACCAAAAAAATTCTACCTTCGTGCGGGTGCACGAGAAGGGCGGAGGC
GGCTCTAGCCCCTTCATCGCCAGCGTCAGCGATCAGCACGGCGTGGTCTACA
TCACCGAAAACAAGAACAAGACCGTGGTCATTCCTTGCCTGGGCAGCATCA
GTAATCTGAACGTGAGCCTGGTGGCCAGATACCCTGAGAAGCGGTTCGTGCC
CGACGGCAACAGGATCAGCTGGGACAGCAAGAAGGGCTTTACGATCCCCTC
TTATATGATCTCCTACGCCGGAATGGTGTTCTGCGAGGCTAAGATCAACGACG
AGAGCTACCAGAGCATCATGTACATCGTGGTCGTGGTCGGCTACCGGATCTAC
GACGTGGTGCTGAGCCCTTCTCACGGCATCGAGCTGTCCGTCGGAGAGAAG
CTGGTGTTGAATTGCACCGCCAGAACCGAGCTGAACGTGGGCATCGACTTTA
ACTGGGAATACCCCAGCTCTAAACACCAGCACAAGAAGCTGGTGAACCGGG
ACCTGAAGACCCAATCTGGTACAGAGATGAAGAAGTTCCTAAGCACACTGA
CCATCGACGGCGTCACAAGAAGCGATCAGGGACTGTACACCTGCGCCGCTA
GCAGCGGACTGATGACCAAGAAAAACAGCACCTTCGTGCGGGTGCACGAGA
AAGACAAGACACACACCTGCCCTCCATGTCCTGCCCCTGAAGCTGCTGGCG
GACCTTCTGTGTTCCTCTTCCCACCTAAGCCTAAGGACACCCTGATGATCAGC
CGGACACCTGAAGTGACATGCGTGGTGGTGGATGTGTCTCACGAAGATCCCG
AGGTGAAATTCAACTGGTACGTGGACGGCGTTGAAGTGCATAATGCCAAGAC
CAAGCCCAGAGAGGAACAGTACAACAGCACCTACAGAGTGGTGTCCGTACT
GACCGTGCTGCACCAGGACTGGCTGAACGGCAAGGAATACAAGTGCAAGGT
GTCCAACAAAGCCCTGCCTGCCCCTATCGAGAAGACCATCTCTAAGGCCAAG

GGCCAGCCTAGAGAACCCCAAGTGTACACACTGCCTCCTTCCAGAGAGGAA
ATGACCAAGAACCAGGTGTCATTGACATGTCTGGTGAAGGGGTTCTACCCAA
GCGACATCGCCGTGGAATGGGAGAGCAACGGACAGCCCGAGAACAACTACA
AGACCACACCTCCAGTGCTCGATAGCGACGGCAGCTTCTTCCTGTATAGCAA
GCTGACCGTGGACAAGTCCAGATGGCAGCAGGGCAATGTGTTCAGCTGCAG
CGTGATGCACGAGGCTCTGCACAACCACTACACCCAGAAGAGCCTGTCCCTG
AGCCCCGGCAAGTGA

SEQ ID NO: 79

codon-optimized sequence 3 of PR1145

ATGCCCCTGCTCCTGCTCCTGCCCCTGCTGTGGGCCGGCGCCCTGGCTAGCG
ACACCGGCAGACCCTTCGTGGAGATGTACAGCGAGATCCCCGAGATCATCCA
CATGACCGAGGGCAGAGAGCTCGTGATCCCTTGCAGAGTGACAAGCCCCAA
CATCACCGTGACCCTGAAAAAGTTCCCCCTGGACACCCTGATCCCCGACGGC
AAGAGAATCATCTGGGACAGCAGAAAGGGCTTCATCATCAGCAACGCCACC
TACAAGGAGATCGGCCTGCTGACCTGCGAGGCCACCGTGAACGGCCACCTG
TACAAGACCAACTACCTGACCCACAGACAGACCAACACCATCATCGACGTCG
TGCTGAGCCCTAGCCACGGCATCGAGCTGAGCGTGGGCGAAAAACTGGTCC
TCAACTGTACCGCTCGGACCGAACTGAACGTGGGGATCGATTTCAACTGGGA
GTATCCTAGCTCCAAGCATCAGCATAAGAAGCTGGTCAACAGAGATCTCAAG
ACACAGAGCGGCTCCGAAATGAAAAAATTCCTCTCCACCCTGACAATTGACG
GCGTCACACGGAGCGACCAAGGCCTGTATACCTGCGCTGCTAGCAGCGGGCT
GATGACCAAGAAGAACTCCACCTTTGTCAGAGTGCACGAAAAGGGCGGGGG
CGGCAGCAGCCCCTTCATCGCTAGCGTGAGCGATCAGCACGGCGTGGTGTAC
ATCACCGAGAACAAGAACAAGACCGTGGTGATCCCCTGCCTGGGCAGCATC
AGCAACCTGAACGTGAGCCTGGTGGCTAGATACCCCGAGAAGAGATTCGTG
CCCGACGGCAACAGAATCAGCTGGGACAGCAAGAAGGGCTTCACCATCCCT
AGCTACATGATCAGCTACGCCGGCATGGTGTTCTGCGAGGCCAAGATCAACG
ACGAGAGCTATCAGAGCATCATGTACATCGTGGTCGTGGTGGGCTACAGAAT
CTACGACGTGGTGCTGTCCCCTAGCCACGGCATTGAACTGTCCGTGGGGGGAG
AAGCTGGTGCTGAACTGTACCGCCCGGACAGAGCTGAACGTGGGCATCGAC
TTTAACTGGGAGTACCCTAGCAGCAAACATCAGCACAAGAAGCTGGTGAAC
CGGGACCTGAAGACACAGAGCGGCACAGAAATGAAGAAATTCCTGAGCACA
CTGACAATTGATGGCGTGACAAGAAGCGACCAAGGCCTGTACACCTGCGCC
GCTAGCAGCGGCCTCATGACAAAGAAGAACAGCACATTTGTGCGGGTGCAC
GAGAAAGACAAAACACATACCTGTCCTCCCTGTCCCGCCCCTGAAGCCGCTG
GGGGGCCTAGCGTGTTCCTGTTCCCCCCCAAGCCCAAGGACACACTGATGAT
CAGCAGAACCCCCGAGGTGACCTGCGTGGTCGTGGACGTGAGCCACGAGGA
CCCCGAGGTGAAGTTCAACTGGTACGTGGACGGCGTGGAGGTGCACAACGC
CAAGACCAAGCCTAGAGAGGAGCAGTACAACAGCACCTACAGAGTGGTGAG
CGTGCTGACCGTGCTGCACCAAGACTGGCTGAACGGCAAGGAGTACAAGTG
CAAGGTGAGCAACAAGGCCCTGCCCGCCCCCATCGAGAAGACCATCAGCAA

GGCCAAGGGGCAGCCTAGAGAGCCCCAAGTGTACACCCTGCCCCCTAGCAG
AGAGGAGATGACCAAAAACCAAGTGAGCCTGACCTGCCTGGTGAAGGGCTT
CTACCCTAGCGACATCGCCGTGGAGTGGGAGAGCAACGGGCAGCCCGAGAA
CAACTACAAGACCACCCCCCCGTGCTGGACAGCGACGGCAGCTTCTTCCTG
TACAGCAAGCTGACCGTGGACAAGAGCAGATGGCAGCAAGGCAACGTGTTC
AGCTGCAGCGTGATGCACGAGGCCCTGCACAACCACTACACACAGAAGAGC
CTGAGCCTGAGCCCCGGCAAGTGA

SEQ ID NO: 80

codon-optimized sequence 1 of PR1174

ATGCCTCTGCTGCTGCTGTTGCCTTTGTTGTGGGCTGGCGCCCTGGCTTCTGA
TACCGGCAGACCTTTCGTGGAAATGTACAGCGAGATCCCCGAGATCATCCAC
ATGACCGAGGGCAGAGAGCTGGTCATCCCCTGCAGAGTGACCTCTCCTAACA
TCACCGTGACTCTGAAGAAGTTCCCTCTGGACACACTGATCCCCGACGGCAA
GAGAATCATCTGGGACTCCCGGAAGGGCTTCATCATCTCCAACGCCACCTAC
AAAGAGATCGGCCTGCTGACCTGCGAGGCCACCGTTAATGGCCACCTGTACA
AGACCAACTATCTGACCCACAGACAGACCAACACCATCATCGACGTGGTGCT
GAGCCCCTCTCATGGCATCGAGCTGTCCGTGGGAGAAAAGCTGGTGCTGAA
CTGCACCGCCAGAACCGAGCTGAACGTGGGCATCGACTTCAACTGGGAGTA
CCCCTCCAGCAAGCACCAGCACAAGAAGCTGGTCAACCGGGACCTGAAAAC
CCAGTCCGGCTCCGAGATGAAGAAATTCCTGAGCACCCTGACCATCGACGGC
GTGACCAGATCTGACCAGGGCCTGTATACCTGCGCCGCTTCTTCTGGCCTGAT
GACCAAGAAAAACTCCACCTTCGTGCGGGTGCACGAGAAAGGTGGCGGAG
GCAGCTCTCCTTTTATCGCCTCTGTGTCTGATCAGCACGGCGTGGTGTACATC
ACCGAGAACAAGAACAAGACCGTCGTGATCCCCTGCCTGGGCTCCATCTCTA
ACCTGAACGTGTCCCTGGTGGCTAGATACCCCGAGAAGAGATTCGTGCCTGA
CGGCAACCGGATCTCCTGGGACAGCAAGAAGGGCTTTACAATCCCCTCCTAC
ATGATCTCCTACGCCGGCATGGTGTTCTGCGAGGCTAAGATCAACGACGAGT
CCTACCAGTCCATCATGTACATCGTGGTGGTCGTGGGCTCCAAGATCTACGAT
GTGGTGCTGTCCCCTAGCCACGGAATCGAACTGAGCGTGGGCGAGAAACTG
GTCCTCAACTGTACCGCTCGGACAGAACTGAATGTGGGGATTGATTTCAATT
GGGAATACCCTAGCTCCAAACATCAGCATAAGAAACTCGTGTTCCGCGATCT
CAAGACCCAGAGCGGAACAGAAATGAAGAAGTTTCTGTCCACACTCACCAT
TGACGGGGTCACCAGAAGCGATCAGGGACTCTATACCTGTGCTGCCTCCAGC
GGACTGATGACAAAAAAGAACAGCACATTTGTCCGCGTCCACGAGAAGGAC
AAAACTCACACATGCCCACCGTGCCCGGCGCCTGAAGCCGCTGGGGGACCG
TCAGTCTTCCTCTTCCCCCCAAAACCCAAGGACACCCTCATGATCTCCCGGA
CCCCTGAGGTCACATGCGTGGTGGTGGACGTGAGCCACGAAGACCCTGAGG
TCAAGTTCAACTGGTACGTGGACGGCGTGGAGGTGCATAATGCCAAGACAA
AGCCGCGGGAGGAGCAGTACAACAGCACGTACCGGGTGGTCAGCGTCCTCA
CCGTCCTGCACCAGGACTGGCTGAATGGCAAGGAGTACAAGTGCAAGGTCT
CCAACAAAGCCCTCCCAGCCCCCATCGAGAAAACCATCTCCAAAGCCAAAG

GGCAGCCCAGGGAACCACAGGTGTACACCCTGCCCCCATCCCGGGAGGAGA
TGACCAAGAACCAGGTCAGCCTGACCTGCCTGGTCAAAGGCTTCTATCCCAG
CGACATCGCCGTGGAGTGGGAGAGCAATGGGCAGCCGGAGAACAACTACAA
GACCACGCCTCCCGTGCTGGACTCCGACGGCTCCTTCTTCCTCTACAGCAAG
CTCACCGTGGACAAGAGCAGGTGGCAGCAGGGGAACGTCTTCTCATGCTCC
GTGATGCATGAGGCTCTGCACAACCACTACACGCAGAAGAGCCTCTCCCTGT
CTCCGGGTAAATAA

SEQ ID NO: 81

codon-optimized sequence 2 of PR1174

ATGCCTCTGCTGCTGCTCCTGCCTCTGCTGTGGGCCGGCGCCCTGGCCAGCG
ATACCGGCAGACCATTCGTGGAAATGTACAGCGAAATCCCCGAGATCATCCA
CATGACCGAGGGCAGAGAACTGGTGATCCCCTGCAGAGTGACCAGCCCAAA
CATCACCGTGACCCTCAAGAAGTTTCCCCTGGACACCCTGATCCCTGATGGA
AAAAGAATCATCTGGGACAGCAGAAAGGGCTTTATCATTTCTAATGCCACATA
CAAAGAGATAGGACTGTTGACCTGTGAAGCTACAGTTAACGGCCACCTGTAC
AAGACCAACTACCTGACCCACCGGCAGACAAACACCATCATCGACGTGGTG
CTGAGCCCTAGCCACGGAATCGAGCTGTCCGTGGGAGAAAAGCTGGTGCTG
AATTGCACCGCCAGAACCGAGCTGAACGTAGGCATCGACTTCAACTGGGAG
TATCCTTCAAGCAAACATCAGCACAAAAAACTGGTGAACAGAGACCTGAAG
ACCCAGAGTGGCAGCGAGATGAAAAAGTTCCTGAGCACACTGACAATCGAC
GGAGTGACTCGGAGCGATCAGGGCCTGTATACATGTGCTGCTTCTAGCGGCC
TGATGACAAAGAAGAACAGCACATTCGTGCGGGTGCACGAGAAGGGGGGGC
GGCGGATCTTCCCCATTCATCGCCAGCGTGTCCGACCAGCATGGCGTGGTTTA
CATCACAGAAAACAAGAACAAGACCGTGGTGATCCCCTGCCTGGGCTCCATC
AGCAACCTGAATGTGTCGCTGGTGGCCAGATACCCCGAAAAGCGGTTCGTCC
CCGACGGCAACAGAATCAGCTGGGACAGCAAAAAGGGCTTCACCATCCCAA
GCTATATGATCAGCTACGCCGGAATGGTGTTCTGCGAGGCCAAAATCAACGA
CGAGTCCTACCAGAGCATCATGTACATCGTGGTGGTTGTCGGCAGCAAGATC
TACGACGTCGTGCTGAGCCCATCTCACGGCATTGAGCTGTCTGTGGGCGAGA
AGCTGGTGCTGAATTGCACCGCCCGGACAGAACTCAATGTGGGCATCGATTT
CAACTGGGAGTACCCTAGCTCTAAGCACCAGCACAAGAAGCTGGTCTTTAGA
GATCTGAAAACCCAATCTGGCACCGAGATGAAAAAGTTCCTGAGCACCCTG
ACCATCGACGGCGTGACCAGAAGCGACCAGGGCCTGTACACCTGCGCCGCC
TCTAGCGGCCTGATGACCAAGAAAAACAGCACCTTCGTGCGCGTTCACGAG
AAAGATAAGACACACACCTGTCCTCCTTGTCCTGCCCCTGAGGCCGCTGGCG
GACCTTCTGTGTTCCTGTTCCCTCCAAAGCCCAAGGACACCCTGATGATCAG
CAGAACACCCGAAGTGACCTGCGTGGTGGTGGACGTCTCTCACGAGGACCC
CGAGGTGAAATTTAACTGGTACGTGGACGGCGTCGAGGTGCATAATGCCAAG
ACAAAGCCTCGGGAAGAGCAGTACAACAGCACATACAGAGTGGTCAGCGTG
CTGACAGTGCTGCACCAGGATTGGCTGAACGGCAAGGAATACAAGTGCAAG
GTGAGCAACAAGGCCCTGCCTGCTCCTATCGAGAAGACCATTAGCAAGGCCA

AGGGCCAGCCTAGGGAGCCTCAGGTGTACACCTTACCACCTAGCAGAGAGG
AAATGACGAAGAACCAGGTGTCCCTGACATGCCTGGTGAAGGGTTTCTACCC
TTCTGATATCGCCGTGGAATGGGAATCCAACGGCCAACCTGAGAACAACTAC
AAGACCACCCCTCCCGTGCTCGATAGCGACGGCTCCTTCTTCCTGTACAGCA
AGCTGACCGTGGATAAGAGCCGGTGGCAGCAGGGCAATGTGTTTAGCTGCTC
TGTGATGCACGAAGCCCTGCACAACCACTACACCCAAAAGAGCCTGAGCCT
GAGCCCCGGCAAGTGA

SEQ ID NO: 82

codon-optimized sequence 3 of PR1174

ATGCCCCTGCTCCTGCTCCTGCCCCTGCTGTGGGCCGGCGCCCTGGCTAGCG
ACACCGGCAGACCCTTCGTGGAGATGTACAGCGAGATCCCCGAGATCATCCA
CATGACCGAGGGCAGAGAGCTCGTGATCCCTTGCAGAGTGACAAGCCCCAA
CATCACCGTGACCCTGAAAAAGTTCCCCCTGGACACCCTGATCCCCGACGGC
AAGAGAATCATCTGGGACAGCAGAAAGGGCTTCATCATCAGCAACGCCACC
TACAAGGAGATCGGCCTGCTGACCTGCGAGGCCACCGTGAACGGCCACCTG
TACAAGACCAACTACCTGACCCACAGACAGACCAACACCATCATCGACGTCG
TGCTGAGCCCTAGCCACGGCATCGAGCTGTCCGTGGGCGAAAAGCTCGTCCT
GAACTGCACCGCTAGAACCGAACTCAACGTGGGGATTGATTTCAACTGGGA
GTATCCTAGCTCCAAGCACCAACACAAGAAGCTGGTGAACCGGGATCTGAA
GACACAGAGCGGGAGCGAGATGAAGAAGTTCCTGAGCACCCTGACAATCGA
CGGGGTGACAAGATCCGACCAAGGCCTGTACACCTGCGCTGCTAGCAGCGG
CCTGATGACAAAGAAAAATAGCACATTTGTGAGAGTGCACGAGAAGGGCGG
GGGCGGCAGCAGCCCCTTCATCGCTAGCGTGAGCGATCAGCACGGCGTGGT
GTACATCACCGAGAACAAGAACAAGACCGTGGTGATCCCCTGCCTGGGCAG
CATCAGCAACCTGAACGTGAGCCTGGTGGCTAGATACCCCGAGAAGAGATTC
GTGCCCGACGGCAACAGAATCAGCTGGGACAGCAAGAAGGGCTTCACCATC
CCTAGCTACATGATCAGCTACGCCGGCATGGTGTTCTGCGAGGCCAAGATCA
ACGACGAGAGCTATCAGAGCATCATGTACATCGTGGTCGTGGTGGGCAGCAA
GATCTACGACGTGGTGCTGAGCCCCTCCCACGGCATTGAGCTCAGCGTGGGC
GAGAAGCTGGTCCTCAACTGCACAGCCCGGACCGAGCTGAACGTGGGCATC
GATTTTAACTGGGAGTACCCTAGCAGCAAGCATCAGCATAAAAAGCTGGTGT
TCCGGGACCTGAAGACCCAAAGCGGCACCGAAATGAAGAAGTTTCTCTCCA
CCCTGACCATCGACGGCGTGACAAGAAGCGACCAAGGCCTGTACACATGCG
CCGCTAGCTCCGGCCTCATGACAAAGAAGAATTCCACCTTTGTCAGAGTCCA
TGAAAAAGATAAGACACACACCTGTCCCCCTTGTCCCGCCCCCGAAGCCGCT
GGGGGCCCTAGCGTGTTCCTGTTCCCCCCCAAGCCCAAGGATACCCTGATGA
TCAGCAGAACCCCCGAGGTGACCTGCGTGGTCGTGGACGTGAGCCACGAGG
ACCCCGAGGTGAAGTTCAACTGGTACGTGGACGGCGTGGAGGTGCACAACG
CCAAGACCAAGCCTAGAGAGGAGCAGTACAACAGCACCTACAGAGTGGTGA
GCGTGCTGACCGTGCTGCACCAAGACTGGCTGAACGGCAAGGAGTACAAGT
GCAAGGTGAGCAACAAGGCCCTGCCCGCCCCCATCGAGAAGACCATCAGCA

AGGCCAAGGGGCAGCCTAGAGAGCCCCAAGTGTACACCCTGCCCCCTAGCA
GAGAGGAGATGACCAAGAACCAAGTGAGCCTGACCTGCCTGGTGAAGGGCT
TCTACCCTAGCGACATCGCCGTGGAGTGGGAGAGCAACGGGCAGCCCGAGA
ACAACTACAAGACCACCCCCCCCGTGCTGGACAGCGACGGCAGCTTCTTCCT
GTACAGCAAGCTGACCGTGGACAAGAGCAGATGGCAGCAAGGCAACGTGTT
CAGCTGCAGCGTGATGCACGAGGCCCTGCACAACCACTACACACAGAAGAG
CCTGAGCCTGAGCCCCGGCAAGTGA

SEQ ID NO: 83

codon-optimized sequence 1 of PR1163

ATGCCTCTGCTGCTGCTGTTGCCTTTGTTGTGGGCTGGCGCTCTGGCCTCTCC
TTTTATCGCCTCTGTGTCTGATCAGCACGGCGTGGTGTACATCACCGAGAACA
AGAACAAGACCGTCGTGATCCCCTGCCTGGGCTCCATCTCTAACCTGAACGT
GTCCCTGGTGGCTAGATACCCCGAGAAGAGATTCGTGCCTGACGGCAACCGG
ATCTCCTGGGACAGCAAGAAGGGCTTTACAATCCCCTCCTACATGATCTCCTA
CGCCGGCATGGTGTTCTGCGAGGCTAAGATCAACGACGAGTCCTACCAGTCC
ATCATGTACATCGTGGTGGTCGTGGGCTACAGAATCTACGATGTGGTGCTGTC
CCCTAGCCACGGAATCGAACTGAGCGTGGGCGAGAAACTGGTCCTCAACTG
TACCGCTCGGACAGAACTGAATGTGGGGATTGATTTCAATTGGGAATACCCTA
GCTCCAAACATCAGCATAAGAAACTCGTGAACCGCGATCTCAAGACCCAGA
GCGGAAGCGAAATGAAGAAGTTTCTGTCCACACTCACCATTGACGGGGTCA
CCAGAAGCGATCAGGGACTCTATACCTGTGCTGCCTCCAGCGGACTGATGAC
AAAAAAGAACAGCACATTTGTCCGCGTCCACGAGAAGGACAAAACTCACAC
ATGCCCACCGTGCCCGGCGCCTGAAGCCGCTGGGGGACCGTCAGTCTTCCTC
TTCCCCCCAAAACCCAAGGACACCCTCATGATCTCCCGGACCCCTGAGGTCA
CATGCGTGGTGGTGGACGTGAGCCACGAAGACCCTGAGGTCAAGTTCAACT
GGTACGTGGACGGCGTGGAGGTGCATAATGCCAAGACAAAGCCGCGGGAGG
AGCAGTACAACAGCACGTACCGGGTGGTCAGCGTCCTCACCGTCCTGCACC
AGGACTGGCTGAATGGCAAGGAGTACAAGTGCAAGGTCTCCAACAAAGCCC
TCCCAGCCCCCATCGAGAAAACCATCTCCAAAGCCAAAGGGCAGCCCAGGG
AACCACAGGTGTACACCCTGCCCCCATCCCGGGAGGAGATGACCAAGAACC
AGGTCAGCCTGACCTGCCTGGTCAAAGGCTTCTATCCCAGCGACATCGCCGT
GGAGTGGGAGAGCAATGGGCAGCCGGAGAACAACTACAAGACCACGCCTCC
CGTGCTGGACTCCGACGGCTCCTTCTTCCTCTACAGCAAGCTCACCGTGGAC
AAGAGCAGGTGGCAGCAGGGGAACGTCTTCTCATGCTCCGTGATGCATGAG
GCTCTGCACAACCACTACACGCAGAAGAGCCTCTCCCTGTCTCCGGGTGGCG
GAGGCGGCTCCGGTGGAGGCGGTTCAGGCGGAGGTGGCTCTGGCGGTGGCG
GATCGGAGGTGCAGCTGGTGGAGAGCGGGGGGGGACTGGTGCAGCCAGGA
GGAAGCCTGAGACTGAGCTGTGCCGCCAGCGGGTACACATTTACCAACTACG
GAATGAACTGGGTGAGGCAGGCTCCTGGCAAGGGGCTGGAGTGGGTGGGGT
GGATTAACACCTACACCGGAGAGCCCACATACGCTGCTGATTTTAAGCGGAG
ATTCACCTTCAGCCTGGACACCAGCAAGAGCACCGCTTACCTGCAGATGAAC

AGCCTGAGAGCCGAGGACACAGCCGTGTACTACTGCGCCAAATACCCCCATT
ACTACGGCAGCAGCCACTGGTACTTCGACGTGTGGGGCCAGGGAACACTGG
TGACCGTGAGCAGCGCTAGCACCAAGGGCCCATCGGTCTTCCCCCTGGCACC
CTCCTCCAAGAGCACCTCTGGGGGCACAGCGGCCCTGGGCTGCCTGGTCAA
GGACTACTTCCCCGAACCGGTGACGGTGTCGTGGAACTCAGGCGCCCTGAC
CAGCGGCGTGCACACCTTCCCGGCTGTCCTACAGTCCTCAGGACTCTACTCC
CTCAGCAGCGTGGTGACCGTGCCCTCCAGCAGCTTGGGCACCCAGACCTAC
ATCTGCAACGTGAATCACAAGCCCAGCAACACCAAGGTGGACAAGAAAGTT
GAGCCCAAATCTTGTGAGGGCAGAGGAAGTCTTCTAACATGCGGTGACGTG
GAGGAGAATCCCGGCCCTATGCCTCTGCTGCTGCTGTTGCCTTTGTTGTGGGC
TGGCGCTCTGGCCGACATTCAGATGACCCAGAGCCCCAGCAGCCTGAGCGC
CAGCGTGGGAGACAGAGTGACCATCACCTGCAGCGCCAGCCAGGACATCAG
CAACTACCTGAACTGGTATCAGCAGAAACCCGGCAAAGCCCCCAAAGTGCT
GATCTACTTCACAAGTTCACTGCACAGCGGAGTGCCCAGCAGATTTTCAGGA
AGCGGCAGCGGCACCGACTTCACCCTGACCATCAGCAGCCTGCAGCCCGAG
GATTTCGCCACCTACTACTGCCAGCAGTACTCTACCGTGCCATGGACCTTCGG
CCAGGGGACCAAGGTGGAAATCAAGCGTACGGTGGCTGCACCATCTGTCTTC
ATCTTCCCGCCATCTGATGAGCAGTTGAAATCTGGAACTGCCTCTGTTGTGTG
CCTGCTGAATAACTTCTATCCCAGAGAGGCCAAAGTACAGTGGAAGGTGGAT
AACGCCCTCCAATCGGGTAACTCCCAGGAGAGTGTCACAGAGCAGGACAGC
AAGGACAGCACCTACAGCCTCAGCAGCACCCTGACGCTGAGCAAAGCAGAC
TACGAGAAACACAAAGTCTACGCCTGCGAAGTCACCCATCAGGGCCTGAGT
TCGCCCGTCACAAAGAGCTTCAACAGGGGAGAGTGTTAA

SEQ ID NO: 84

codon-optimized sequence 2 of PR1163

ATGCCTCTGCTTCTGCTGCTGCCTCTGCTTTGGGCTGGCGCCCTGGCCAGCCC
CTTCATCGCCTCTGTTAGTGACCAGCATGGCGTGGTGTATATCACGGAGAACA
AGAATAAGACAGTAGTGATTCCTTGCCTGGGCAGCATCTCTAATCTGAACGTG
TCGCTGGTTGCCAGATACCCTGAGAAGAGATTCGTGCCCGACGGCAACCGGA
TCAGCTGGGACAGCAAGAAGGGCTTTACCATCCCCTCTTACATGATCAGCTAT
GCCGGAATGGTGTTCTGCGAAGCCAAGATCAACGACGAGTCCTATCAGTCTA
TCATGTACATCGTGGTCGTAGTGGGCTACAGAATCTACGACGTGGTGCTGAG
CCCATCTCACGGCATCGAGCTGAGCGTGGGAGAGAAGCTGGTGCTGAACTG
CACAGCTAGAACCGAGCTGAACGTGGGCATAGACTTCAACTGGGAGTACCC
CTCCAGCAAGCACCAGCACAAGAAACTGGTCAACAGAGATCTGAAGACCCA
GAGCGGCTCTGAGATGAAGAAGTTCCTGTCCACCCTGACAATCGACGGCGT
GACCAGAAGCGATCAGGGACTGTACACTTGTGCTGCGAGCTCTGGCCTGATG
ACCAAGAAAAACAGCACCTTCGTGCGGGTGCACGAGAAGGACAAGACCCA
CACATGCCCTCCTTGTCCTGCCCCTGAGGCCGCTGGCGGCCCTAGCGTGTTC
CTGTTCCCCCCTAAACCCAAGGACACCCTCATGATCTCCCGGACACCTGAAG
TGACTTGCGTGGTCGTGGACGTCAGCCACGAAGATCCAGAGGTGAAGTTCA

ACTGGTATGTGGATGGCGTGGAAGTGCATAATGCGAAGACAAAGCCTCGGGA
AGAGCAGTACAACTCCACGTACAGAGTGGTGAGCGTGCTGACCGTGCTGCA
TCAAGACTGGCTGAACGGCAAGGAATACAAGTGCAAGGTGTCCAACAAGGC
CCTGCCCGCTCCTATCGAAAAAACCATCTCTAAGGCCAAGGGCCAGCCTAGA
GAGCCTCAGGTGTATACCCTGCCTCCTAGCCGGGAAGAAATGACAAAGAACC
AGGTGTCTCTGACCTGCCTGGTGAAGGGCTTCTACCCCAGCGACATCGCCGT
GGAGTGGGAGTCCAACGGCCAACCTGAAAACAATTACAAGACCACCCCTCC
CGTGCTGGACAGCGACGGCAGCTTCTTCCTGTATTCCAAGCTGACCGTCGAC
AAGAGCAGATGGCAGCAGGGCAACGTGTTTAGCTGTAGCGTGATGCACGAG
GCCCTGCACAACCACTACACACAAAGTCTCTGTCCCTGAGCCCTGGAGGC
GGCGGCGGAAGCGGAGGGGGCGGCTCCGGCGGCGGAGGCAGCGGCGGCGG
CGGTTCCGAAGTGCAGCTGGTGGAAAGCGGAGGCGGTCTGGTTCAGCCGGG
CGGCAGCCTGAGACTGTCTTGCGCCGCCAGCGGCTACACATTCACCAACTAC
GGCATGAACTGGGTCAGACAGGCCCCTGGCAAGGGCCTGGAATGGGTGGGA
TGGATCAACACCTACACCGGCGAACCAACATACGCCGCTGATTTTAAGCGGA
GGTTCACCTTTTCTCTGGATACCTCCAAAAGCACCGCCTACCTGCAAATGAA
CAGCCTGCGGGCCGAGGACACCGCCGTTTACTACTGCGCCAAATATCCTCAC
TACTACGGCAGCTCTCACTGGTACTTCGACGTTTGGGGCCAGGGCACACTGG
TGACCGTGTCCTCGGCCAGCACAAAGGGCCCTAGCGTGTTCCCACTGGCACC
ATCTAGCAAGAGCACCAGTGGAGGCACCGCCGCCCTGGGATGCCTGGTGAA
AGATTACTTCCCCGAGCCTGTCACCGTGTCTTGGAACTCCGGTGCTCTGACG
AGCGGAGTGCACACCTTCCCAGCCGTGCTTCAGTCCAGCGGTCTGTACAGCC
TGTCTTCTGTTGTGACCGTGCCTTCCAGCTCCCTGGGCACGCAGACCTACATC
TGCAACGTGAACCACAAGCCTAGCAATACCAAGGTGGATAAGAAGGTGGAA
CCCAAAAGCTGCGAGGGAAGAGGCTCCCTCCTGACATGCGGCGACGTGGAA
GAGAACCCCGGCCCTATGCCCCTGCTGCTGCTGCTGCCTCTGCTGTGGGCCG
GCGCCCTGGCCGATATCCAGATGACCCAGTCTCCTTCCTCCTTATCCGCCTCT
GTGGGCGACAGAGTGACCATCACCTGTTCTGCCAGCCAGGATATCAGCAACT
ACCTGAACTGGTACCAGCAGAAGCCCGGCAAAGCCCCAAAGGTGCTGATTT
ACTTCACAAGCAGCCTGCACAGCGGAGTGCCTAGCCGCTTCAGCGGCTCTG
GCAGCGGGACCGACTTTACACTGACCATTAGCAGCCTGCAGCCTGAGGATTT
CGCCACATACTACTGTCAGCAATACAGCACAGTCCCTTGGACCTTTGGACAA
GGAACAAAGGTGGAAATCAAGCGGACCGTGGCCGCTCCCAGCGTGTTCATC
TTCCCTCCTAGCGACGAGCAACTGAAATCTGGCACCGCCAGCGTCGTGTGCC
TGCTGAACAACTTCTACCCGAGAGAGGCTAAGGTGCAGTGGAAGGTGGACA
ACGCCCTGCAGAGCGGGAATAGCCAGGAGAGCGTGACCGAGCAGGACTCCA
AAGACAGCACATACTCTCTGAGCAGCACCCTTACACTGAGCAAGGCCGACTA
CGAGAAACACAAAGTGTACGCTTGTGAGGTGACCCACCAGGGCCTGAGCTC
TCCCGTGACCAAAAGCTTTAATAGAGGCGAGTGCTGA

SEQ ID NO: 85

codon-optimized sequence 3 of PR1163

ATGCCTCTGCTGCTGCTTCTGCCTCTTCTTTGGGCTGGCGCTCTGGCTAGCCC
TTTTATCGCCTCCGTGTCCGATCAGCACGGCGTGGTGTACATCACCGAGAAC
AAGAACAAGACCGTGGTCATCCCCTGCCTGGGCAGCATCAGCAACCTGAAT
GTGTCCCTGGTGGCCAGATATCCCGAGAAGAGATTCGTGCCCGACGGCAACA
GAATCAGCTGGGACAGCAAGAAGGGCTTCACAATCCCCAGCTACATGATCAG
CTACGCCGGCATGGTGTTCTGCGAGGCCAAGATCAACGACGAGAGCTACCA
GAGCATCATGTACATCGTGGTGGTCGTGGGCTACAGAATCTACGACGTGGTG
CTGAGCCCTAGCCACGGCATTGAACTGTCTGTGGGCGAGAAGCTGGTGCTGA
ACTGTACCGCCAGAACCGAGCTGAACGTGGGCATCGACTTCAACTGGGAGT
ACCCCAGCAGCAAGCACCAGCACAAGAAACTGGTCAACCGGGACCTGAAA
ACCCAGAGCGGCAGCGAGATGAAGAAGTTCCTGAGCACCCTGACCATCGAC
GGCGTGACCAGATCTGACCAGGGCCTGTACACATGTGCCGCCAGCTCTGGCC
TGATGACCAAGAAAAACAGCACCTTCGTGCGGGTGCACGAGAAGGACAAG
ACCCACACCTGTCCTCCATGTCCTGCTCCAGAAGCTGCTGGCGGCCCTTCCG
TGTTTCTGTTCCCTCCAAAGCCTAAGGACACCCTGATGATCAGCAGAACCCC
TGAAGTGACCTGCGTGGTGGTGGATGTGTCCCACGAGGATCCCGAAGTGAA
GTTCAATTGGTACGTGGACGGCGTGGAAGTGCACAACGCCAAGACCAAGCC
TAGAGAGGAACAGTACAACTCCACCTACAGAGTGGTGTCCGTGCTGACCGT
GCTGCACCAGGATTGGCTGAACGGCAAAGAGTACAAGTGCAAGGTGTCCAA
CAAGGCCCTGCCTGCTCCTATCGAGAAAACCATCAGCAAGGCCAAGGGCCA
GCCTAGGGAACCCCAGGTTTACACACTGCCTCCAAGCCGGGAAGAGATGAC
AAAGAACCAGGTGTCCCTGACCTGCCTGGTCAAGGGCTTCTACCCTTCCGAT
ATCGCCGTGGAATGGGAGAGCAATGGCCAGCCTGAGAACAACTACAAGACA
ACCCCTCCTGTGCTGGACAGCGACGGCTCATTCTTCCTGTACAGCAAGCTGA
CAGTGGACAAGAGCAGATGGCAGCAGGGCAACGTGTTCAGCTGCAGCGTGA
TGCACGAGGCCCTGCACAACCACTACACCCAGAAGTCCCTGTCTCTGTCTCC
TGGCGGAGGCGGAGGATCTGGTGGCGGAGGAAGCGGTGGCGGCGGATCAG
GCGGTGGTGGTTCTGAAGTTCAGCTGGTGGAAAGCGGCGGTGGCCTTGTTC
AACCTGGCGGATCTCTGAGACTGAGCTGTGCCGCCTCTGGCTACACCTTCAC
CAACTACGGCATGAATTGGGTCCGACAGGCCCCTGGCAAAGGCCTGGAATG
GGTCGGATGGATCAACACCTACACCGGCGAGCCAACATACGCCGCCGACTTC
AAGCGGAGATTCACCTTCAGCCTGGACACCAGCAAGAGCACCGCCTACCTG
CAGATGAACTCCCTGAGAGCCGAGGACACCGCCGTGTACTACTGCGCCAAG
TATCCCCACTACTACGGCAGCAGCCACTGGTACTTTGACGTGTGGGGACAGG
GCACCCTGGTCACAGTTAGCAGCGCCTCTACAAAGGGCCCCAGCGTTTTCCC
ACTGGCTCCTAGCAGCAAGTCTACCTCCGGTGGAACAGCTGCCCTGGGCTGT
CTCGTGAAGGACTACTTTCCTGAGCCTGTGACCGTGTCCTGGAACTCTGGCG
CACTGACATCTGGCGTGCACACCTTTCCAGCTGTGCTGCAGTCTAGCGGCCT
GTACTCTCTGAGCAGCGTCGTGACAGTGCCTAGCAGCTCTCTGGGCACCCAG
ACCTACATCTGCAATGTGAACCACAAGCCTAGCAACACCAAGGTCGACAAG
AAGGTGGAACCCAAGAGCTGCGAAGGCAGAGGCAGCCTGCTTACATGTGGC

GACGTGGAAGAGAACCCCGGACCTATGCCACTTCTCCTGCTGTTGCCATTGC
TGTGGGCAGGCGCACTGGCCGACATTCAGATGACACAGAGCCCAAGCAGCC
TGAGCGCCTCTGTGGGAGATAGAGTGACCATCACCTGTTCCGCCAGCCAGGA
CATCTCCAACTACCTGAACTGGTATCAGCAAAAGCCCGGCAAGGCCCCTAAG
GTGCTGATCTACTTTACCAGCAGCCTGCACAGCGGCGTGCCCAGCAGATTTT
CTGGCTCTGGCAGCGGCACCGACTTCACCCTGACAATATCTAGCCTGCAGCC
AGAGGACTTCGCCACCTACTACTGCCAGCAGTACAGCACCGTGCCTTGGACA
TTTGGCCAGGGCACCAAGGTGGAAATCAAGCGGACAGTCGCCGCTCCTAGC
GTGTTCATCTTTCCACCTAGCGACGAGCAGCTGAAGTCTGGCACAGCCTCTG
TCGTGTGCCTGCTGAACAACTTCTACCCCAGAGAAGCCAAGGTGCAGTGGA
AGGTGGACAATGCCCTGCAGTCCGGCAACAGCCAAGAGAGCGTGACAGAGC
AGGACTCCAAGGACAGCACCTACAGCCTGTCTAGCACACTGACCCTGAGCA
AGGCCGACTATGAGAAGCACAAAGTGTACGCCTGCGAAGTGACCCACCAGG
GGCTTTCTAGCCCAGTGACCAAGAGCTTCAACCGGGGCGAGTGTTGA

SEQ ID NO: 86

The 3'-UTR sequence:

GTCTAGAAACCAGCCTCAAGAACACCCGAATGGAGTCTCTAAGCTACATAAT
ACCAACTTACACTTTACAAAATGTTGTCCCCCAAAATGTAGCCATTCGTATCT
GCTCCTAATAAAAGAAAGTTTCTTCAC

SEQ ID NO: 87

The WPRE sequence:

AATCAACCTCTGGATTACAAAATTTGTGAAAGATTGACTGGTATTCTTAACTA
TGTTGCTCCTTTTACGCTATGTGGATACGCTGCTTTAATGCCTTTGTATCATGCT
ATTGCTTCCCGTATGGCTTTCATTTTCTCCTCCTTGTATAAATCCTGGTTGCTGT
CTCTTTATGAGGAGTTGTGGCCCGTTGTCAGGCAACGTGGCGTGGTGTGCAC
TGTGTTTGCTGACGCAACCCCCACTGGTTGGGGCATTGCCACCACCTGTCAG
CTCCTTTCCGGGACTTTCGCTTTCCCCCTCCCTATTGCCACGGCGGAACTCAT
CGCCGCCTGCCTTGCCCGCTGCTGGACAGGGGCTCGGCTGTTGGGCACTGAC
AATTCCGTGGTGTTGTCGGGGAAATCATCGTCCTTTCCTTGGCTGCTCGCCTG
TGTTGCCACCTGGATTCTGCGCGGGACGTCCTTCTGCTACGTCCCTTCGGCCC
TCAATCCAGCGGACCTTCCTTCCCGCGGCCTGCTGCCGGCTCTGCGGCCTCT
TCCGCGTCTTCGCCTTCGCCCTCAGACGAGTCGGATCTCCCTTTGGGCCGCCT
CCCCGC

SEQ ID NO: 88

The SV40polyA sequence:

CCTAGGTAAGATACATTGATGAGTTTGGACAAACCACAACTAGAATGCAGTG
AAAAAAATGCTTTATTTGTGAAATTTGTGATGCTATTGCTTTATTTGTAACCAT
TATAAGCTGCAATAAACAAGTTATCGATAGATCT

SEQ ID NO: 89

The capsid protein sequence:

MAADGYLPDWLEDTLSEGIRQWWKLKPGPPPPKPAERHKDDSRGLVLPGYKY

LGPFNGLDKGEPVNEADAAALEHDKAYDRQLDSGDNPYLKYNHADAEFQERL
KEDTSFGGNLGRAVFQAKKRVLEPLGLVEEPVKTAPGKKRPVEHSPVEPDSSSG
TGKAGQQPARKRLNFGQTGDADSVPDPQPLGQPPAAPSGLGTNTMATGSGAPM
ADNNEGADGVGNSSGNWHCDSTWMGDRVITTSTRTWALPTYNNHLYKQISSQ
SGASNDNHYFGYSTPWGYFDFNRFHCHFSPRDWQRLINNNWGFRPKRLNFKLF
NIQVKEVTQNDGTTTIANNLTSTVQVFTDSEYQLPYVLGSAHQGCLPPFPADVF
MVPQYGYLTLNNGSQAVGRSSFYCLEYFPSQMLRTGNNFTFSYTFEDVPFHSSY
AHSQSLDRLMNPLIDQYLYYLSRTNTPSGTTTQSRLQFSQAGASDIRDQSRNWL
PGPCYRQQRVSKTSADNNNSEYSWTGATKYHLNGRDSLVNPGPAMASHKDDE
EKFFPQSGVLIFGKQGSEKTNVDIEKVMITDEEEIRTTNPVATEQYGSVSTNLQR
GNLALGDTTRPARQAATADVNTQGVLPGMVWQDRDVYLQGPIWAKIPHTDGH
FHPSPLMGGFGLKHPPPQILIKNTPVPANPSTTFSAAKFASFITQYSTGQVSVEIE
WELQKENSKRWNPEIQYTSNYNKSINVDFTVDTNGVYSEPRPIGTRYLTRNL

SEQ ID NO: 77.

**Example 9: Construction of Expression Vector**

An expression cassette was constructed. An AAV packaging plasmid expressing a VEGF-binding molecule protein gene (see FIG. 3) was constructed by conventional molecular biology procedures such as enzyme digestion, ligation, transformation, and clone screening identification, and it sequentially comprises: a CMV enhancer, a promoter, a 5' UTR, a Kozak sequence (gccacc), a VEGFR-binding molecule, a 3' UTR, a WPRE, and an SV40polyA. The expression cassette was flanked by inverted terminal repeats (ITRs). EcoRV and BSMI are enzymatic digestion sites.

**Example 10: AAV Virus Preparation and Purification**

The amino acid sequence of the AAV capsid used for delivering the gene expression cassette in this example is set forth in SEQ ID NO: 77. Referring to the method for packaging and purifying recombinant AAV viruses reported by Martin Lock et al., PEI was used to co-transfect HEK293 cells with plasmids expressing AAV Rep and Cap proteins, a helper plasmid, and the expression vector of Example 9 to package and prepare a recombinant adeno-associated virus. After 48 h of transfection, cells and the culture supernatant were harvested, and the AAV virus was purified using iodixanol gradient ultracentrifugation (see Martin Lock, et al. Rapid, Simple, and Versatile Manufacturing of Recombinant Adeno-Associated Viral Vectors at Scale. HUMAN GENE THERAPY, 2010. 21:1259-1271) to obtain a recombinant adeno-associated viral vector. The viral titer was measured using real-time fluorescence quantitative PCR.

**Example 11: Intraocular Expression Experiment**

To determine the expression levels of the efficacy proteins in vitreous humor, aqueous humor, retina, and choroid tissues in the rabbit eye and changes in the expression levels after AAV-1174 (expressing the PR1174 (the nucleic acid sequence is SEQ ID NO: 81) efficacy protein) and ADVM-022 (see rAAV2.7m8-aflibercept in WO2017218974) were injected into the rabbit eye, the following experiment was conducted. Thirty-two New Zealand rabbits, 2 to 5 months old, were purchased, divided into 4 groups of 8, and bilaterally intravitreally injected with the doses shown in Table 12 (each sample was injected at a high dose and at a low dose). At day 58 post-injection, vitreous humor, aqueous humor, retina, and choroid tissues were collected from both eyes. At each time point, the sampling number corresponding to each administration group was 2 rabbits, i.e., 4 eyes. The samples were sent back to our laboratory, where the efficacy protein content in the samples was measured using an enzyme-linked immunosorbent assay.

Table 12: Dose grouping for the intraocular expression experiment

| Sample | Number (rabbit/eye) | Viral titer | Injection volume | VG/eye |
|---|---|---|---|---|
| AAV-1174 (low dose) | 2/4 | 2E12 vg/mL | 100 μL | 2E11 vg/eye |
| AAV-1174 (high dose) | 2/4 | 6E12 vg/mL | 100 μL | 6E11 vg/eye |
| ADVM-022 ((low dose) | 2/4 | 2E12 vg/mL | 100 μL | 2E11 vg/eye |
| ADVM-022 (high dose) | 2/4 | 6E12 vg/mL | 100 μL | 6E11 vg/eye |

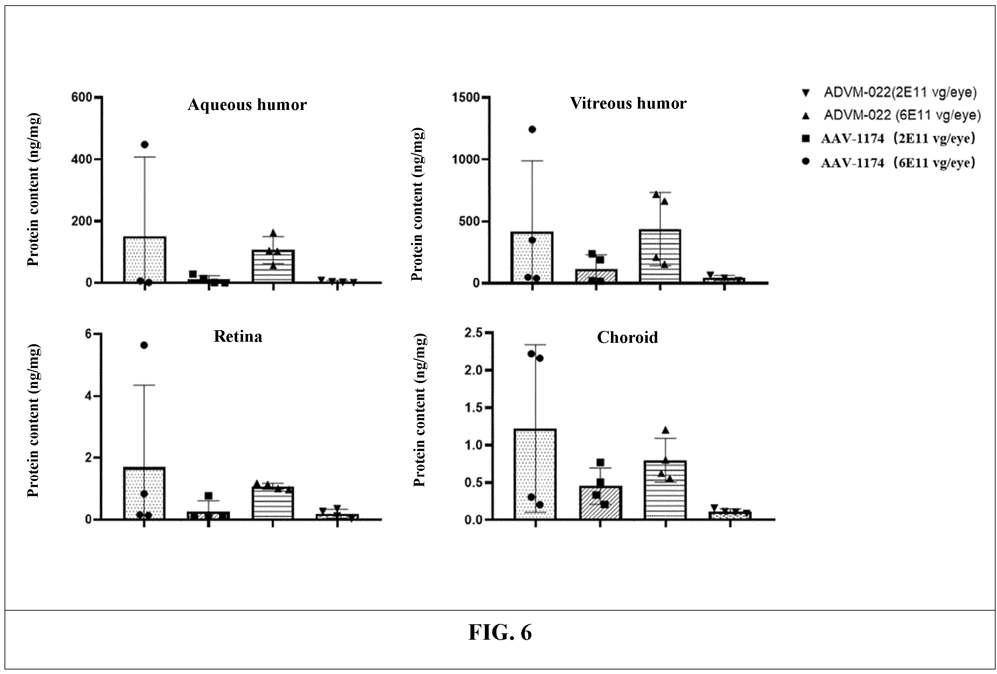

FIG. 6 shows the intraocular expression results. At D58, both the AAV-1174 high-dose group and the AAV-1174 low-dose group exhibited stable expression of PR1174 protein in the aqueous humor, vitreous humor, retina, and choroid of the rabbit eye, and the protein expression levels were higher than those of ADVM-022.

### Example 12: *In Vivo* Efficacy Experiment

The laser-induced fundus choroidal neovascularization model is currently the classic model commonly used internationally for validating anti-choroidal neovascularization drugs. To validate the inhibitory effect of AAV-1174 on fundus choroidal neovascularization, this model was used for efficacy validation.

Thirty-six rats, with an equal number of males and females, 8-10 weeks old, were purchased, divided into 6 groups of 6, and bilaterally intravitreally injected with the doses shown in Table 13. Three weeks after AAV-1174 (expressing the PR1174 (the nucleic acid sequence is SEQ ID NO: 81) efficacy protein) was administered, both eyes underwent laser modeling. One week post-modeling, fundus angiography was performed to measure efficacy.

Table 13: Dose grouping for the *In vivo* efficacy experiment

| Sample | Number | Viral dosage | Injection volume |
|---|---|---|---|
| AAV-1174 (high dose) | 6 | 2E10 vg/mL | 1 μL |
| AAV-1174 (low dose) | 6 | 6E9 vg/mL | 1 μL |
| ADVM-022 (high dose) | 6 | 2E10 vg/mL | 1 μL |
| ADVM-022 (low dose) | 6 | 6E9 vg/mL | 1 μL |
| PBS | 6 | NA | 1 μL |

Table 14: The inhibition of fundus choroidal neovascularization

| Group | D29: grade 3 leakage spot ratio (%) (mean ± SD) | D29 spot leakage average score (mean ± SD) |
|---|---|---|
| 1-PBS | 56.7±27.5 | 2.33±0.50 |
| AAV-1174(6E+9 vg) | 63.3±24.6 | 2.33±0.42 |
| AAV-1174(2E+10 vg) | 44.4±21.7 | 2.05±0.40 |
| ADVM-022 (6E+9 vg) | 63.9±17.2 | 2.42±0.35 |
| ADVM-022 (2E+10 vg) | 40.7±14.7 | 1.81±0.38 |

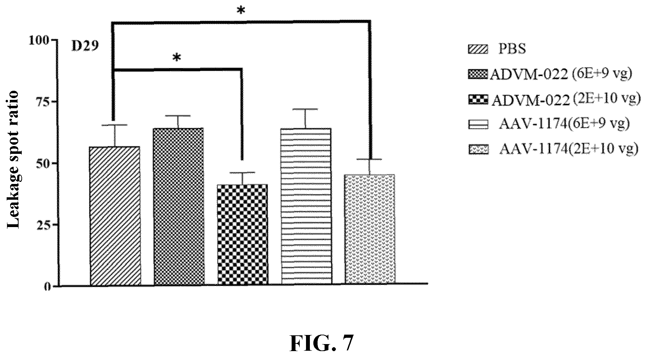

Fundus photography was performed on the rats before modeling, and no abnormalities were observed in the fundi of the rats. All the rats underwent CNV modeling 21 days post-administration, and FFA analysis was performed 28 days post-administration. Significant laser spot fluorescein sodium leakage was observed in the PBS group 7 days post-modeling, indicating successful model establishment; the fluorescent spot grading score was determined by FFA 28 days post-administration, and the grade 3 leakage spot ratio was determined. The results are shown in Table 14 and FIG. 7. The differences between the AAV-1174 (high dose) group and the PBS group were statistically significant. AAV-1174 exhibited a significant inhibitory effect on laser-induced choroidal neovascularization fluorescein sodium leakage.

**Claims**

1. A VEGF-binding molecule, comprising:

   a first VEGF-binding domain, and
   a second VEGF-binding domain;
   wherein,
   the first VEGF-binding domain comprises immunoglobulin-like domain 2 of VEGFR2 (R2D2) and immunoglobulin-like domain 3 of VEGFR2 (R2D3);
   the second VEGF-binding domain comprises a VEGF Trap or comprises an anti-VEGF antibody or an antigen-binding fragment thereof.

2. The VEGF-binding molecule according to claim 1, wherein:
   the VEGF Trap comprises immunoglobulin-like domain 2 of VEGFR1 (R1D2) and/or immunoglobulin-like domain 3 of VEGFR2 (R2D3).

3. The VEGF-binding molecule according to claim 1 or 2, wherein:

   the first VEGF-binding domain comprises an amino acid mutation at any one or more positions selected from the group consisting of the following: position 162, position 221, position 222, position 274, position 276, position 277, position 280, position 284, position 288, position 289, and position 313; the position of the amino acid mutation is relative to a natural sequence position of the amino acid sequence set forth in SEQ ID NO: 1;
   preferably, the first VEGF-binding domain comprises an amino acid mutation at any one or more sets of positions selected from the group consisting of the following:

   - position 162;
   - position 162 and position 221;
   - position 162 and position 222;
   - position 162 and position 274;
   - position 162 and position 276;
   - position 162 and position 277;
   - position 162 and position 289;
   - position 162 and position 313;
   - position 221 and position 222;
   - position 277 and position 289;
   - position 162, position 221, and position 222;
   - position 162, position 277, and position 289; and
   - position 162, position 221, position 222, and position 274;

   more preferably, the first VEGF-binding domain comprises an amino acid mutation at any one set of positions selected from the group consisting of the following:

   - position 162; and
   - position 162, position 221, position 222, and position 274.

4. The VEGF-binding molecule according to any one of claims 1-3, wherein:

   the first VEGF-binding domain comprises any one or more sets of amino acid mutations selected from the group consisting of the following:

   - 162A, 162V, 162S, 162I, or 162L;
   - 221S or 221N;
   - 222K;
   - 274F or 274I;

- 276Q;
- 277R;
- 280E;
- 284H;
- 288Y;
- 289Y; and
- 313R;

preferably, the first VEGF-binding domain comprises any one or more sets of amino acid mutations selected from the group consisting of the following:

- 162V or 162I;
- 162V/274F;162V/274I;162I/274F; or 162I/274I;
- 221S/222K;
- 277R/289Y;
- 162V/221S/222K;
- 162V/276Q;
- 162V/289Y;
- 162V/277/289Y;
- 162V/313R; and
- 162V/221S/222K/274F;

more preferably, the first VEGF-binding domain comprises any one or more sets of amino acid mutations selected from the group consisting of the following:

- 162V; and
- 162V/221S/222K/274F.

5. The VEGF-binding molecule according to any one of claims 1-3, wherein:

the first VEGF-binding domain comprises an amino acid mutation at any one or more positions selected from the group consisting of the following: position 135, position 197, position 199, position 213, position 215, position 219, position 257, position 275, position 277, position 278, position 283, position 289, position 313, and position 314; the position of the amino acid mutation is relative to a natural sequence position of the amino acid sequence set forth in SEQ ID NO: 1; preferably, the first VEGF-binding domain comprises an amino acid mutation at position 283.

6. The VEGF-binding molecule according to claim 5, wherein:

the first VEGF-binding domain comprises any one or more sets of amino acid mutations selected from the group consisting of the following:

- 135Y;
- 197Y;
- 199Q or 199T;
- 213N;
- 215F or 215H;
- 219I;
- 257Q or 257T;
- 275L or 275E;
- 277R;
- 278D, 278S, or 278E;
- 283T;
- 289Y;
- 313Y; and
- 314S or 314Q;

preferably, the first VEGF-binding domain comprises an amino acid mutation 283T.

7. The VEGF-binding molecule according to any one of claims 1-6, wherein:

the first VEGF-binding domain comprises an amino acid mutation at any one or more positions selected from the group consisting of the following: position 162, position 221, position 222, position 274, position 276, position 277, position 280, position 284, position 288, position 289, and position 313; and
comprises an amino acid mutation at any one or more positions selected from the group consisting of the following: position 135, position 197, position 199, position 213, position 215, position 219, position 257, position 275, position 277, position 278, position 283, position 289, position 313, and position 314;
preferably, the first VEGF-binding domain comprises an amino acid mutation at any one set of positions selected from the group consisting of the following:

- position 162 and position 275;
- position 162 and position 283;
- position 162 and position 313;
- position 162, position 274, and position 283;
- position 162, position 221, position 222, and position 283; and
- position 162, position 221, position 222, position 274, and position 283;

more preferably, the first VEGF-binding domain comprises an amino acid mutation at any one set of positions selected from the group consisting of the following:

- position 162 and position 283; and
- position 162, position 221, position 222, position 274, and position 283.

8. The VEGF-binding molecule according to any one of claim 7, wherein:

the first VEGF-binding domain comprises:

(i) any one or more sets of amino acid mutations selected from the group consisting of the following:

- 162A, 162V, 162S, 162I, or 162L;
- 221S;
- 222K;
- 274F or 274I;
- 276Q;
- 277R;
- 280E;
- 284H;
- 288Y;
- 289Y; and
- 313R; and

(ii) any one or more sets of amino acid mutations selected from the group consisting of the following:

- 135Y;
- 197Y;
- 199Q or 199T;
- 213N;
- 215F or 215H;
- 219I;
- 257Q or 257T;
- 275L or 275E;
- 277R;
- 278D, 278S, or 278E;
- 283T;
- 289Y;
- 313Y; and
- 314S or 314Q;

preferably, the first VEGF-binding domain comprises any one or more sets of amino acid mutations selected from the group consisting of the following:

- 162V/275L;
- 162 V/283 T;
- 162V/313Y;
- 162V/274F/283T;
- 162V/221S/222K/283T; and
- 162V/2215/222K/274F/283T;

more preferably, the first VEGF-binding domain comprises any one or more sets of amino acid mutations selected from the group consisting of the following:

- 162V/283T; and
- 162V/221S/222K/274F/283T.

9. A VEGF-binding molecule, comprising:

at least one VEGF-binding domain comprising immunoglobulin-like domain 2 of VEGFR2 (R2D2) and immunoglobulin-like domain 3 of VEGFR2 (R2D3),
wherein the VEGF-binding domain comprises an amino acid mutation at one or more positions selected from the group consisting of the following: position 162, position 221, position 222, position 274, position 276, position 277, position 280, position 284, position 288, position 289, and position 313; the position of the amino acid mutation is relative to a natural sequence position of the amino acid sequence set forth in SEQ ID NO: 1.

10. The VEGF-binding molecule according to claim 9, wherein the VEGF-binding domain comprises an amino acid mutation at any one or more sets of positions selected from the group consisting of the following:

- position 162;
- position 162 and position 221;
- position 162 and position 222;
- position 162 and position 274;
- position 162 and position 276;
- position 162 and position 277;
- position 162 and position 289;
- position 162 and position 313;
- position 221 and position 222;
- position 277 and position 289;
- position 162, position 221, and position 222;
- position 162, position 277, and position 289; and
- position 162, position 221, position 222, and position 274;

preferably, the VEGF-binding domain comprises an amino acid mutation at any one set of positions selected from the group consisting of the following:

- position 162; and
- position 162, position 221, position 222, and position 274.

11. The VEGF-binding molecule according to claim 9 or 10, wherein the VEGF-binding domain comprises any one or more sets of amino acid mutations selected from the group consisting of the following:

- 162A, 162V, 162S, 162I, or 162L;
- 221S or 221N;
- 222K;
- 274F or 274I;
- 276Q;
- 277R;
- 280E;

- 284H;
- 288Y;
- 289Y; and
- 313R;

preferably, the VEGF-binding domain comprises any one or more sets of amino acid mutations selected from the group consisting of the following:

- 162V or 162I;
- 162V/274F;162V/274I;162I/274F; or 162I/274I;
- 221S/222K;
- 277R/289Y;
- 162V/221S/222K;
- 162V/276Q;
- 162V/289Y;
- 162V/277/289Y;
- 162V/313R; and
- 162V/221S/222K/274F;

more preferably, the VEGF-binding domain comprises any one or more sets of amino acid mutations selected from the group consisting of the following:

- 162V; and
- 162V/221S/222K/274F.

12. The VEGF-binding molecule according to claim 9 or 10, wherein the VEGF-binding domain comprises an amino acid mutation at any one or more positions selected from the group consisting of the following: position 135, position 197, position 199, position 213, position 215, position 219, position 257, position 275, position 277, position 278, position 283, position 289, position 313, and position 314; the position of the amino acid mutation is relative to a natural sequence position of the amino acid sequence set forth in SEQ ID NO: 1; preferably, the VEGF-binding domain comprises an amino acid mutation at position 283.

13. The VEGF-binding molecule according to claim 12, wherein the VEGF-binding domain comprises any one or more sets of amino acid mutations selected from the group consisting of the following:

- 135Y;
- 197Y;
- 199Q or 199T;
- 213N;
- 215F or 215H;
- 219I;
- 257Q or 257T;
- 275L or 275E;
- 277R;
- 278D, 278S, or 278E;
- 283T;
- 289Y;
- 313Y; and
- 314S or 314Q;

preferably, the VEGF-binding domain comprises an amino acid mutation 283T.

14. The VEGF-binding molecule according to any one of claims 9-13, wherein the VEGF-binding domain comprises an amino acid mutation at any one or more positions selected from the group consisting of the following: position 162, position 221, position 222, position 274, position 276, position 277, position 280, position 284, position 288, position 289, and position 313; and

comprises an amino acid mutation at any one or more positions selected from the group consisting of the following: position 135, position 197, position 199, position 213, position 215, position 219, position 257, position 275, position 277, position 278, position 283, position 289, position 313, and position 314; preferably, the VEGF-binding domain comprises an amino acid mutation at any one or more sets of positions selected from the group consisting of the following:

- position 162 and position 275;
- position 162 and position 283;
- position 162 and position 313;
- position 162, position 274, and position 283;
- position 162, position 221, position 222, and position 283; and
- position 162, position 221, position 222, position 274, and position 283;

more preferably, the VEGF-binding domain comprises an amino acid mutation at any one or more sets of positions selected from the group consisting of the following:

- position 162 and position 283; and
- position 162, position 221, position 222, position 274, and position 283.

15. The VEGF-binding molecule according to claim 14, wherein the VEGF-binding domain comprises:

(i) any one or more sets of amino acid mutations selected from the group consisting of the following:

- 162A, 162V, 162S, 162I, or 162L;
- 221S;
- 222K;
- 274F or 274I;
- 276Q;
- 277R;
- 280E;
- 284H;
- 288Y;
- 289Y; and
- 313R; and

(ii) any one or more sets of amino acid mutations selected from the group consisting of the following:

- 135Y;
- 197Y;
- 199Q or 199T;
- 213N;
- 215F or 215H;
- 219I;
- 257Q or 257T;
- 275L or 275E;
- 277R;
- 278D, 278S, or 278E;
- 283T;
- 289Y;
- 313Y; and
- 314S or 314Q;

preferably, the VEGF-binding domain comprises any one or more sets of amino acid mutations selected from the group consisting of the following:

- 162V/275L;
- 162 V/283 T;
- 162V/313Y;
- 162V/274F/283T;
- 162V/221S/222K/283T; and

- 162V/221S/222K/274F/283T;

more preferably, the VEGF-binding domain comprises any one or more sets of amino acid mutations selected from the group consisting of the following:

- 162V/283T; and
- 162V/221S/222K/274F/283T.

16. The VEGF-binding molecule according to any one of claims 1-15, also comprising an Fc, wherein preferably, the Fc is an Fc of human IgG1, human IgG2, human IgG3, or human IgG4.

17. The VEGF-binding molecule according to any one of claims 1-8, wherein the VEGF Trap is aflibercept or conbercept; the anti-VEGF antibody or the antigen-binding fragment thereof is an anti-VEGF-A antibody or an antigen-binding fragment thereof, bevacizumab or an antigen-binding fragment thereof, or ranibizumab or an antigen-binding fragment thereof;
preferably, the antigen-binding fragment is an scFv, Fv, Fab, or Fab' fragment.

18. The VEGF-binding molecule according to any one of claims 1-17, wherein:
the VEGF-binding molecule comprises, from N-terminus to C-terminus, any one selected from the group consisting of the following:

R2D2-R2D3-Fc;
R1D2-R2D3-R2D2-R2D3-Fc;
R 1 D2-R2D3 -linker-R2D2-R2D3 -F c;
R2D2-R2D3-R1D2-R2D3-Fc; and
R2D2-R2D3-linker-R1D2-R2D3-Fc;
or, the VEGF-binding molecule comprises a heavy chain and a light chain, wherein the heavy chain comprises, from N-terminus to C-terminus: R2D2-R2D3-Fc-linker-VH-CH1, and the light chain comprises, from N-terminus to C-terminus: VL-CL;
preferably, the VH and VL are the VH and VL of bevacizumab or the VH and VL of ranibizumab;
preferably, the linker is a peptide linker;
more preferably, the linker is selected from the group consisting of: (GS)a(GGS)b(GGGS)c(GGGGS)d(GGGGG)e, wherein a, b, c, d, and e are independently integers greater than or equal to 0; or
the linker is selected from the group consisting of: $(EAAAK)_3$, $(EAAAR)_3$, $(EGGGK)_3$, $(EGGGR)_3$, $(DAAAR)_3$, $(DAAAK)_3$, $(DGGGR)_3$, and $(DGGGK)_3$; or
the linker is $(G_xS)_y$, wherein x is selected from the group consisting of integers of 1-5, and y is selected from the group consisting of integers of 1-6.

19. The VEGF-binding molecule according to any one of claims 9-18, wherein:

the VEGF-binding molecule comprises the amino acid sequence set forth in any one of SEQ ID NOs: 5-6 and SEQ ID NOs: 62-65 or an amino acid sequence having at least 90% sequence identity thereto; or
the VEGF-binding molecule consists of the amino acid sequence set forth in any one of SEQ ID NOs: 5-6 and SEQ ID NOs: 62-65 or an amino acid sequence having at least 90% sequence identity thereto.

20. The VEGF-binding molecule according to any one of claims 1-18, wherein:

1) the VEGF-binding molecule comprises the amino acid sequence set forth in any one of SEQ ID NOs: 3-4, SEQ ID NOs: 7-49, SEQ ID NOs: 52-61, and SEQ ID NO: 66, or an amino acid sequence having at least 90% sequence identity thereto;
2) the heavy chain of the VEGF-binding molecule comprises the amino acid sequence set forth in SEQ ID NO: 50, or an amino acid sequence having at least 90% sequence identity thereto, and the light chain of the VEGF-binding molecule comprises the amino acid sequence set forth in SEQ ID NO: 51, or an amino acid sequence having at least 90% sequence identity thereto; or
3) the heavy chain of the VEGF-binding molecule comprises the amino acid sequence set forth in SEQ ID NO: 67, or an amino acid sequence having at least 90% sequence identity thereto, and the light chain of the VEGF-binding molecule comprises the amino acid sequence set forth in SEQ ID NO: 68, or an amino acid sequence having at least 90% sequence identity thereto.

21. An isolated nucleic acid, comprising a polynucleotide encoding the VEGF-binding molecule according to any one of claims 1-20.

22. The isolated nucleic acid according to claim 21, wherein the polynucleotide comprises a nucleic acid sequence having at least 70% identity to the nucleic acid sequence set forth in any one of SEQ ID NOs: 78-86.

23. The isolated nucleic acid according to any one of claims 21-22, further comprising a promoter and/or an enhancer that are/is operatively linked to the polynucleotide, wherein preferably, the promoter is a CMV promoter;
preferably, the enhancer is a CMV enhancer.

24. The isolated nucleic acid according to any one of claims 21-23, further comprising any one or any combination of the following: a Kozak sequence, a 5'-UTR and a 3'-UTR, a WPRE sequence, a polyA sequence, and an intron, wherein

preferably, the Kozak sequence is located between the intron and the polynucleotide encoding the VEGF-binding molecule;
preferably, the polyA sequence is an sv40 polyA sequence.

25. The isolated nucleic acid according to any one of claims 21-24, being a DNA or RNA, preferably a cDNA or mRNA.

26. A vector, wherein the vector comprises the isolated nucleic acid according to any one of claims 21-25; preferably, the vector is selected from the group consisting of any one of the following or a combination thereof: a plasmid, a lentiviral vector, an adenovirus, an adeno-associated viral (AAV) vector, a recombinant adeno-associated viral (rAAV) vector, and a retroviral vector;
more preferably, the vector is a recombinant adeno-associated viral vector.

27. A recombinant adeno-associated viral vector, comprising:

(i) the polynucleotide encoding the VEGF-binding molecule defined in any one of claims 21-25;
(ii) an enhancer, preferably a CMV enhancer;
(iii) a promoter, preferably a CMV promoter;
(iv) a 5'-UTR and a 3'-UTR;
(v) a Kozak sequence;
(vi) a WPRE sequence; and
(vii) an sv40 polyA sequence; wherein

any one or any combination of (ii)-(vii) is operatively linked to the (i).

28. A recombinant adeno-associated viral particle, comprising:

the isolated nucleic acid according to any one of claims 21-25, or
the recombinant adeno-associated viral vector according to claim 27, and a capsid protein; wherein
preferably, the capsid protein is selected from the group consisting of any one of the following serotypes: AAV1, AAV2, AAV3, AAV4, AAV5, AAV6, AAV7, AAV8, AAV9, AAV9.47, AAV9 (hu14), AAV10, AAV11, AAV12, AAVrh8, AAVrh10, AAV-DJ, and AAV-DJ8 or variants thereof;
more preferably, the capsid protein comprises the amino acid sequence set forth in SEQ ID NO: 77, or an amino acid sequence having at least 70% sequence identity to SEQ ID NO: 77.

29. A host cell, comprising the isolated nucleic acid according to any one of claims 21-25, or the vector according to any one of claims 26-27.

30. A pharmaceutical composition, comprising the VEGF-binding molecule according to any one of claims 1-20, the isolated nucleic acid according to any one of claims 21-25, the vector according to any one of claims 26-27, or the recombinant adeno-associated viral particle according to claim 28, wherein
preferably, the pharmaceutical composition further comprises at least one pharmaceutically acceptable carrier, diluent, or excipient.

31. Use of the VEGF-binding molecule according to any one of claims 1-20, the isolated nucleic acid according to any one of claims 21-25, or the vector according to any one of claims 26-27, or the recombinant adeno-associated viral particle according to claim 28 in the preparation of a medicament for treating a disease associated with abnormal angiogenesis, wherein

preferably, the disease associated with abnormal angiogenesis is an angiogenic ocular disease or cancer;
more preferably, the angiogenic ocular disease is selected from the group consisting of macular degeneration (e.g., age-related macular degeneration), macular edema (e.g., macular edema following retinal vein occlusion, or diabetic macular edema), retinal vein occlusion (e.g., central retinal vein occlusion, branch retinal vein occlusion), choroidal neovascularization, iris neovascularization, neovascular glaucoma, post-operative fibrosis in glaucoma, proliferative vitreoretinopathy, optic disc neovascularization, corneal neovascularization, retinal neovascularization, vitreous neovascularization, pannus, pterygium, choroidal retinopathy, retinopathy of prematurity, vascular retinopathy, diabetic retinopathy, non-proliferative diabetic retinopathy, and proliferative diabetic retinopathy;
the cancer is selected from the group consisting of small cell lung cancer, kidney cancer, uterine cancer, prostate cancer, bladder cancer, ovarian cancer, colon cancer, breast cancer, leukemia, lymphoma, myeloma, sarcoma (e.g., fibrosarcoma, myxosarcoma, liposarcoma, lymphatic endotheliosarcoma, angiosarcoma, endotheliosarcoma, chondrosarcoma, osteosarcoma, chordoma, lymphangiosarcoma, synovioma, mesothelioma, leiomyosarcoma, or rhabdomyosarcoma), neuroglioma, pancreatic cancer, squamous cell carcinoma, basal cell carcinoma, adenocarcinoma, sweat gland cancer, sebaceous carcinoma, papillary carcinoma, papillary adenocarcinoma, cystadenocarcinoma, medullary carcinoma, bronchopulmonary carcinoma, choriocarcinoma, renal cell carcinoma, liver cancer, bile duct cancer, seminoma, embryonal carcinoma, cervical cancer, testicular tumor, lung cancer, small cell lung cancer, bladder cancer, epithelial cancer, astrocytoma, ependymoma, pinealoma, hemangioblastoma, acoustic neuroma, medulloblastoma, craniopharyngioma, oligodendroglioma, meningioma, melanoma, neutrophilic tumor, and retinoblastoma.

**32.** An rAAV production system, for producing the recombinant adeno-associated viral particle according to claim 28, wherein the production system comprises:

(a) a nucleic acid sequence encoding the amino acid sequence of an AAV capsid protein;
(b) the isolated nucleic acid according to any one of claims 21-25 or the vector according to any one of claims 26-27; and
(c) a helper packaging element having sufficient AAV rep function and helper function that allow packaging of the isolated nucleic acid according to any one of claims 21-25 or the vector according to any one of claims 26-27 into an AAV capsid;

wherein the AAV capsid is selected from the group consisting of any one of the following: AAV1, AAV2, AAV3, AAV4, AAV5, AAV6, AAV7, AAV8, AAV9, AAV9.47, AAV9 (hu14), AAV10, AAV11, AAV12, AAVrh8, AAVrh10, AAV-DJ, and AAV-DJ8 or variants thereof, preferably AAV2.

**33.** The rAAV production system according to claim 32, wherein:

the helper packaging element is provided by a packaging cell;
preferably, the packaging cell comprises a plasmid selected from the group consisting of any one of the following or a combination thereof: pHelper, pRC9, and pGOI.

**34.** A method for producing the recombinant adeno-associated viral particle according to claim 28, comprising:

a step of packaging the isolated nucleic acid according to any one of claims 21-25 or the vector according to any one of claims 26-27 into an AAV capsid using a packaging cell, wherein
the AAV capsid is selected from the group consisting of any one of the following: AAV1, AAV2, AAV3, AAV4, AAV5, AAV6, AAV7, AAV8, AAV9, AAV9.47, AAV9 (hu14), AAV10, AAV11, AAV12, AAVrh8, AAVrh10, AAV-DJ, and AAV-DJ8 or variants thereof, preferably AAV2;
the packaging cell comprises a plasmid selected from the group consisting of any one of the following or a combination thereof: pHelper, pRC9, and pGOI.

# DRAWINGS OF THE SPECIFICATION

FIG. 1

| CMV enhancer | CMV promoter | 5'UTR | Kozak sequence | VEGF-binding molecule sequence | 3'UTR | WPRE | sv40polyA |
|---|---|---|---|---|---|---|---|

## FIG. 2

**FIG. 3**

**FIG. 4**

| PBS | Aflibercept (2.5 mg/mL) | OPT302 (10 mg/mL) | PR1145 (3.75 mg/mL) | PR1145 (1.5 mg/mL) |

**FIG. 5A**

**FIG. 5B**

**FIG. 6**

**FIG. 7**

## INTERNATIONAL SEARCH REPORT

| International application No. |
| --- |
| **PCT/CN2023/077365** |

**A.    CLASSIFICATION OF SUBJECT MATTER**

C07K19/00(2006.01)i;  C07K16/22(2006.01)i;  C07K14/005(2006.01)i;  C12N15/86(2006.01)i;  A61K38/17(2006.01)i; A61P27/02(2006.01)i

According to International Patent Classification (IPC) or to both national classification and IPC

**B.    FIELDS SEARCHED**

Minimum documentation searched (classification system followed by classification symbols)

IPC:C07K C12N A61K A61P

Documentation searched other than minimum documentation to the extent that such documents are included in the fields searched

Electronic data base consulted during the international search (name of data base and, where practicable, search terms used)

CNTXT; VCN; VEN; ENTXT; ENTXTC; OETXT; CNKI; 万方, WANFANG; 读秀学术, DUXIU SCHOLAR; 中国专利生物序列检索系统, Chinese Patent Biological Sequence Retrieval System; Genbank; EMBL; STNEXT; ISI Web of Science; Springer: 上海瑞宏迪, VEGF, 血管内皮生长因子, 结构域, R1D2, R2D2, R2D3, 突变, Trap, Fc, scFV, Fab, 阿柏西普, 康柏西普, 抗体, 抗原, IgG, 连接子, AAV, 基于说明书核苷酸和氨基酸序列的检索, search on the basis of the nucleotide and amino acid sequences of the description, REGENELEAD, vascular endothelial growth factor, domain, mutation, aflibercept, conbercept, antibody, antigen, linker

**C.    DOCUMENTS CONSIDERED TO BE RELEVANT**

| Category* | Citation of document, with indication, where appropriate, of the relevant passages | Relevant to claim No. |
| --- | --- | --- |
| X | CN 101838329 A (GENOR BIOPHARMA CO., LTD.) 22 September 2010 (2010-09-22) claims 1-10, descriptions [0017], [0018], [0023], [0041], [0042], and [0045], and embodiments 1-3 | 1, 2, 16-18, 21-34 |
| A | CN 101838329 A (GENOR BIOPHARMA CO., LTD.) 22 September 2010 (2010-09-22) claims 1-10, descriptions [0017], [0018], [0023], [0041], [0042], and [0045], and embodiments 1-3 | 3-34 |
| A | CN 110423281 A (CHENGDU GENE VECTOR BIO-TECH CO., LTD.) 08 November 2019 (2019-11-08) claims 1-10 | 1-34 |
| A | CN 101397343 A (CHENGDU KANGHONG BIOTECHNOLOGY CO., LTD.) 01 April 2009 (2009-04-01) claims 1-9 | 1-34 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **11 May 2023** | **02 June 2023** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/077365**

| C. | DOCUMENTS CONSIDERED TO BE RELEVANT | |
|---|---|---|
| Category* | Citation of document, with indication, where appropriate, of the relevant passages | Relevant to claim No. |
| A | US 2014213769 A1 (HONG HYO JEONG et al.) 31 July 2014 (2014-07-31)<br>abstract, and description, paragraphs [0010]-[0051] | 1-34 |

Form PCT/ISA/210 (second sheet) (July 2022)

**INTERNATIONAL SEARCH REPORT**

| International application No. |
| --- |
| **PCT/CN2023/077365** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

            ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

INTERNATIONAL SEARCH REPORT
Information on patent family members

International application No.

**PCT/CN2023/077365**

| Patent document cited in search report | | | Publication date (day/month/year) | Patent family member(s) | | | Publication date (day/month/year) |
|---|---|---|---|---|---|---|---|
| CN | 101838329 | A | 22 September 2010 | US | 2010331250 | A1 | 30 December 2010 |
| | | | | US | 8926972 | B2 | 06 January 2015 |
| | | | | JP | 2012520661 | A | 10 September 2012 |
| | | | | BRPI | 1006368 | A2 | 25 October 2016 |
| | | | | WO | 2010105573 | A1 | 23 September 2010 |
| | | | | RU | 2011141522 | A | 27 April 2013 |
| | | | | ZA | 201106638 | A | 30 May 2012 |
| CN | 110423281 | A | 08 November 2019 | CN | 110423281 | B | 30 April 2021 |
| CN | 101397343 | A | 01 April 2009 | CN | 100567325 | C | 09 December 2009 |
| US | 2014213769 | A1 | 31 July 2014 | US | 9605043 | B2 | 28 March 2017 |
| | | | | KR | 20120137270 | A | 20 December 2012 |
| | | | | KR | 101397088 | B1 | 19 May 2014 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210157037X
- CN 202210156042
- WO 2018232055 A
- WO 2005033321 A
- WO 2015168666 A
- WO 2015121501 A
- WO 2015038958 A
- WO 2016065001 A
- WO 2016130589 A
- WO 2016049230 A
- WO 2016134375 A
- WO 2017100671 A
- WO 2017083722 A
- WO 2017015102 A
- WO 2017058892 A
- WO 2017066764 A
- US 9546112 B
- US 7198951 B
- US 9233131 B
- US 6156303 A
- US 9624274 B
- US 9475845 B
- US 8734809 B
- US 20130224836 A
- US 20140359799 A
- US 20150315612 A
- US 20150376240 A
- US 20150159173 A
- US 20150376607 A
- US 20150238550 A
- US 20160369298 A
- US 20160361439 A
- US 20170145405 A
- US 20160017005 A
- WO 2016005324 A
- WO 2016005004 A
- WO 2016091391 A
- WO 2019036513 A
- WO 2020074642 A
- WO 200028004 A
- WO 200123001 A
- WO 2004112727 A
- WO 2005005610 A
- WO 2005072364 A
- WO 2013123503 A
- WO 2015191508 A
- US 20130195801 A
- US 7271002 B
- US 297958
- US 20050053922 A
- US 20090202490 A
- WO 2017218974 A

**Non-patent literature cited in the description**

- **NPULICHERLA et al.** *MolecularTherapy*, 2011, vol. 19 (6), 1070-1078
- **MATHER et al.** *Annals N.YAcad Sci*, 1982, vol. 383, 44-68
- Computational Molecular Biology. Oxford University Press, 1988
- Biocomputing: Informatics and Genome Projects. Academic Press, 1993
- **VON HEINJE, G.** Sequence Analysis in Molecular Biology. Academic Press, 1987
- Computer Analysis of Sequence Data. Humana Press, 1994
- Sequence Analysis Primer. M Stockton Press, 1991
- **MEYERS** ; **MILLER**. *CABIOS*, 1989, vol. 4, 11-17
- **CARILLO, H.** ; **LIPMAN, D.** *SIAM J Applied Math.*, vol. 48, 1073
- *J. biol. chem.*, 1968, vol. 243, 3558
- Antibodies: A Laboratory Manual and Molecular Cloning: A Laboratory Manual. Cold Spring Harbor Laboratory
- **MARTIN LOCK et al.** Rapid, Simple, and Versatile Manufacturing of Recombinant Adeno-Associated Viral Vectors at Scale. *HUMAN GENE THERAPY*, 2010, vol. 21, 1259-1271